# EUROPEAN PATENT SPECIFICATION

(11) **EP 2 215 082 B1**
(45) Date of publication and mention of the grant of the patent: **21.09.2011**
(21) Application number: 08805231.1
(22) Date of filing: 10.10.2008
(51) Int. Cl.: C07D 403/06, C07D 471/04, A61K 31/502, A61K 31/5025, A61P 29/00, A61P 37/08

(54) **PHTHALAZINE AND PYRIDO[3,4-D]PYRIDAZINE COMPOUNDS AS H1 RECEPTOR ANTAGONISTS**
PHTHALAZIN- UND PYRIDO[3,4-D]PYRIDAZINVERBINDUNGEN ALS H1-REZEPTOR-ANTAGONISTEN
PHTHALAZINE ET COMPOSÉS PYRIDO[3,4-D]PYRIDAZINE COMME ANTAGONISTES DU RÉCEPTEUR H1

(30) Priority: 11.10.2007 US 979105 P
(43) Date of publication of application: 11.08.2010
(73) Proprietor: Glaxo Group Limited, Greenford Middlesex UB6 0NN (GB)
(72) Inventor: GORE, Paul, Martin, Hertfordshire SG1 2NY (GB); LOOKER, Brian, Edgar, Hertfordshire SG1 2NY (GB); PROCOPIOU, Panayiotis, Alexandrou, Hertfordshire SG1 2NY (GB); VILE, Sadie, Hertfordshire SG1 2NY (GB)
(74) Representative: Le Mière, Jennifer Louise
(86) International application number: PCT/EP2008/063642
(87) International publication number: WO 2009/047336

(56) References cited:
- DE-A1- 3 634 942

## Description

The present invention relates to a class of compounds which are 4-benzyl-1(2*H*)-phthalazinone derivatives, processes for their preparation, pharmaceutical compositions containing them and to their use in the treatment of various inflammatory and/or allergic diseases, in particular inflammatory and/or allergic diseases of the respiratory tract.

Allergic rhinitis (seasonal and perennial), pulmonary inflammation and congestion are medical conditions that are often associated with other conditions such as asthma and chronic obstructive pulmonary disease (COPD). In general, these conditions are mediated, at least in part, by inflammation associated with the release of histamine from various cells, in particular mast cells.

Allergic rhinitis, which includes 'hay fever' affects a large proportion of the population worldwide. There are two types of allergic rhinitis, seasonal and perennial. The clinical symptoms of seasonal allergic rhinitis typically include nasal itching and irritation, sneezing and watery rhinorrhea, which is often accompanied by nasal congestion. The clinical symptoms of perennial allergic rhinitis are similar, except that nasal blockage may be more pronounced. Either type of allergic rhinitis may also cause other symptoms, such as itching of the throat and/or eyes, epiphora and oedema around the eyes. The symptoms of allergic rhinitis may vary in intensity from the nuisance level to debilitating.

Allergic rhinitis and other allergic conditions are associated with the release of histamine from various cell types, but particularly mast cells. The physiological effects of histamine are classically mediated by three receptor subtypes, termed H1, H2 and H3. H1 receptors are widely distributed throughout the CNS and periphery, and are involved in wakefulness and acute inflammation. H2 receptors mediate gastric acid secretion in response to histamine. H3 receptors are present on the nerve endings in both the CNS and periphery and mediate inhibition of neurotransmitter release [Hill et al., Pharmacol. Rev., 49:253-278, (1997)]. Recently a fourth member of the histamine receptor family has been identified, termed the H4 receptor [Hough, Mol. Pharmacol., 59:415-419, (2001)]. Whilst the distribution of the H4 receptor appears to be restricted to cells of the immune and inflammatory systems, a physiological role for this receptor remains to be identified.

The activation of H1 receptors in blood vessels and nerve endings are responsible for many of the symptoms of allergic rhinitis, which include itching, sneezing, and the production of watery rhinorrhea. Oral antihistamine compounds which are selective H1 receptor antagonists, such as chlorphenyramine, cetirizine, desloratidine and fexofenadine are effective in treating the itching, sneezing and rhinorrhea associated with allergic rhinitis. Intranasal antihistamines which are selective H1 receptor antagonists, such azelastine and levocabastine, are thought to have similar therapeutic effects to their oral counterparts. However, such compounds generally require twice daily administration and may still cause sedatation despite their local application.

A class of compounds have been identified as H1 receptor antagonists.

Thus, the present invention provides a compound of formula (I) 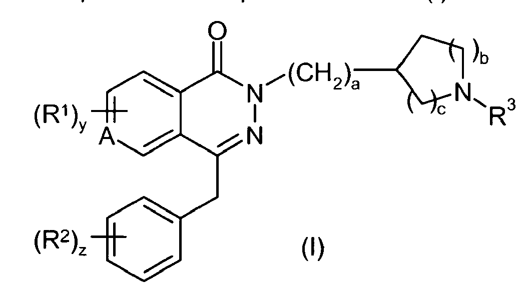 wherein
A represents CH or N;
R¹ and R² each independently represent halogen, C₁₋₆alkyl, C₁-₆alkoxy, hydroxyl or trifluoromethyl;
y and z each independently represent 0, 1 or 2;
a represents 0 or 1;
b represents 0, 1 or 2 and c represents 0, 1, 2 or 3, such that b and c cannot both be 0;
R³ represents -C₁₋₆alkylene-R⁴-R⁵, in which the alkylene is straight chain and is optionally substituted by one C₁₋₃alkyl, or R³ represents a saturated 5 to 7 membered ring containing one SO₂;
R⁴ represents -SO₂-, -N(R⁶)SO₂-, -SO₂N(R⁷)- or -N(R⁸)C(O)N(R⁹)-;
R⁵ represents -C₁₋₆alkyl (optionally substituted by one, two or three halogen or by one or two C₁₋₆alkoxy, in which the C₁₋₆alkoxy may be optionally substituted by one, two or three halogen), -C₅₋₇cycloalkyl (optionally substituted by one or two C₁₋₃alkyl), -C₁₋₃alkyleneC₅₋₇cycloalkyl (in which the C₅₋₇cycloalkyl is optionally substituted by one or two C₁₋₃alkyl), -aryl (optionally substituted by one or two substituents independently selected from halogen, C₁₋₃alkyl, trifluoromethyl, or cyano), or -C₁₋₃alkylenearyl (optionally substituted on aryl by one or two substituents independently selected from halogen, C₁₋₃alkyl, trifluoromethyl, or cyano); R⁶, R⁷, R⁸ and R⁹ each independently represent hydrogen or C₁₋₆alkyl;
or together R⁷ and R⁵ together with the N atom to which they are attached represent a 5 to 7 membered saturated heterocyclic ring optionally containing one further heteroatom independently selected from O and S;
or a salt thereof.

The compounds of the invention may be expected to be useful in the treatment of various diseases in particular inflammatory and/or allergic diseases, such as inflammatory and/or allergic diseases of the respiratory tract (for example allergic rhinitis) that are associated with the release of histamine from cells such as mast cells. Further, the compounds may show an improved profile in that they may possess one or more of the following properties:
(i) greater selectivity over the H3 receptor;
(ii) lower CNS penetration;
(iii) prolonged duration of action.

Compounds having such a profile may be particularly suitable for intranasal delivery, and/or capable of once daily administration and/or further may have an improved side effect profile compared with other existing therapies.

By 'selectivity' it is meant that the compounds may be more potent at the H1 receptor than at the H3 receptor and/or the hERG receptor. The activity at the H1 receptor may be at least about 10 fold greater (e.g. about 100 fold greater) than activity at the H3 receptor.

In one embodiment, y represents 0;
z represents 1;
R² represents halogen, C₁₋₆alkyl, C₁₋₆alkoxy, hydroxyl or trifluoromethyl;
a represents 0 or 1;
b represents 0, 1 or 2 and c represents 0, 1, 2 or 3, such that b and c cannot both be 0;
R³ represents -C₁₋₆alkylene-R⁴-R⁵, in which the alkylene is straight chain and is optionally substituted by one C₁₋₃alkyl;
R⁴ represents -SO₂-, -N(R⁶)SO₂⁻, -SO₂N(R⁷)- or -N(R⁸)C(O)N(R⁹)-;
R⁵ represents -C₁₋₆alkyl (optionally substituted by one or two C₁₋₆alkoxy), -C₅₋₇cycloalkyl (optionally substituted by one or two C₁₋₃alkyl), -C₁₋₃alkyleneC₅₋₇cycloalkyl (in which the C₅₋₇cycloalkyl is optionally substituted by one or two C₁₋₃alkyl);
R⁶, R⁷, R⁸ and R⁹ each independently represent hydrogen or C₁₋₆alkyl;
or together R⁷ and R⁵ together with the N atom to which they are attached represent a 5 to 7 membered saturated heterocyclic ring optionally containing one further heteroatom independently selected from O and S;
or a salt thereof.

In another embodiment, y represents 0;
z represents 1;
R² represents halogen (e.g. chlorine or fluorine), C₁₋₆alkyl (e.g. methyl), C₁₋₆alkoxy (e.g. methoxy), hydroxyl or trifluoromethyl;
a represents 0 or 1;
b represents 0, 1 or 2 and c represents 0, 1, 2 or 3, such that b and c cannot both be 0;
R³ represents -C₂₋₅alkylene-R⁴-R⁵, in which the alkylene is straight chain and is optionally substituted by one C₁₋₃alkyl (e.g. methyl);
R⁴ represents -SO₂-, -N(R⁶)SO₂⁻, -SO₂N(R⁷)- or -N(R⁸)C(O)N(R⁹)-;
R⁵ represents -C₁₋₄alkyl (optionally substituted by one or two (e.g. one) C₁₋₆alkoxy (e.g. -O-methyl)),
R⁶, R⁷, R⁸ and R⁹ each independently represent hydrogen or C₁₋₃alkyl;
or together R⁷ and R⁵ together with the N atom to which they are attached represent a 5 to 7 membered saturated heterocyclic ring (e.g. a 6 membered ring);
or a salt thereof.

In another embodiment, A represents CH.

In another embodiment, R² represents halogen (e.g. chlorine or fluorine), C₁₋₃alkyl (e.g. methyl), C₁₋₃alkoxy (e.g. methoxy), hydroxyl or trifluoromethyl.

In another embodiment, R² represents halogen; in a further embodiment, R² represents chloro; in yet a further embodiment R² represents chloro substituted in the para position.

In another embodiment y is 0.

In another embodiment, Z is 1.

In another embodiment when z is 1, R² is in the para position.

In another embodiment a represents 0, b represents 2 and c represents 1.

In another embodiment, a represents 1, b represents 0 and c represents 2.

In another embodiment, R³ represents -C₁₋₆alkylene-R⁴-R⁵, in which the alkylene is straight chain and is optionally substituted by one C₁₋₃alkyl. In a further embodiment, R³ represents - C₂₋₅alkylene-R⁴-R⁵, in which the alkylene is straight chain and is optionally substituted by one C₁-₃alkyl (e.g. methyl). In another embodiment, R³ represents straight chain C₁₋₆alkylene-R⁴-R⁵ e.g. straight chain C₂₋₅alkylene-R⁴-R⁵.

In another embodiment, R⁵ represents -C₁₋₆alkyl (optionally substituted by one or two C₁₋₆₋alkoxy), -C₅₋₇cycloalkyl (optionally substituted by one or two C₁₋₃alkyl), -C₁₋₃alkyleneC₅₋₇cycloalkyl (in which the C₅₋₇cycloalkyl is optionally substituted by one or two C₁₋₃alkyl).

In another embodiment, R⁵ represents -C₁₋₄alkyl (optionally substituted by one or two (e.g. one) C₁₋₆alkoxy (e.g. -O-methyl)).

In another embodiment, R⁶, R⁷, R⁸ and R⁹ each independently represent hydrogen or C₁₋₃alkyl (e.g. methyl) or together R⁷ and R⁵ together with the N atom to which they are attached represent a 5 to 7 membered saturated heterocyclic ring (e.g. a 6 membered ring).

Representative compounds of formula (I) include the compounds of Examples 1 to 14, including individual isomers thereof and isomeric mixtures (e.g. a racemate or a racemic mixture), in the form of a free base, or as salts thereof (e.g. pharmaceutically acceptable salts thereof).

It is to be understood that the invention includes all possible combinations of embodiments and substituents described herein.

C₁₋₆alkyl, whether alone or as part of another group, may be straight chain or branched and C₁₋₆alkoxy shall be interpreted similarly. Representative examples include, but are not limited to methyl, ethyl, *n-*propyl, *iso*-propyl, *n-*butyl, *sec*-butyl, *iso*-butyl, *t*-butyl, *n-*pentyl, *neo*-pentyl and *n-*hexyl. Particular alkyl and alkoxy groups are C₁₋₃alkyl and C₁₋₃alkoxy.

Representative examples of C₁₋₆alkylene include methlyene [-(CH₂)-], ethylene [-(CH₂)₂-], propylene, [-(CH₂)₃-], butylene [-(CH₂)₄-], pentylene [-(CH₂)₅-] and hexylene [-(CH₂)₆-].

As defined herein, the term "aryl" includes single and fused aromatic rings. Representative examples of aryl groups include, but are not limited to phenyl and naphthyl. Aryl is intended to denote all positional isomers thereof. A representative aryl ring is phenyl.

As defined herein, the term "C₅₋₇cycloalkyl" refers to a non-aromatic hydrocarbon ring having from five to seven carbon atoms. Representative examples of such rings include cyclopentyl, cyclohexyl and cycloheptyl.

The term "halogen" is used herein to describe, unless otherwise stated, a group selected from fluorine, chlorine, bromine or iodine, particularly chlorine or fluorine.

It is to be understood that the present invention covers compounds of formula (I) as the free base and as salts thereof, for example as a pharmaceutically acceptable salt.

It is to be further understood that references hereinafter to compounds of the invention or to compounds of formula (I) mean a compound of formula (I) as the free base, or as a salt, unless otherwise stated.

The compounds of formula (I) may be in the form of and/or may be administered as a pharmaceutically acceptable salt. For a review on suitable salts see Berge et al., J. Pharm. Sci., 1977, 66, 1-19. Suitable pharmaceutically acceptable salts include acid addition salts. As used herein, the term "pharmaceutically acceptable salt", means any pharmaceutically acceptable salt or solvate of a compound of formula (I), which upon administration to the recipient is capable of providing (directly or indirectly) a compound of formula (I), or an active metabolite or residue thereof.

Typically, a pharmaceutically acceptable salt may be readily prepared by using a desired acid as appropriate. The salt may precipitate from solution and be collected by filtration or may be recovered by evaporation of the solvent.

A pharmaceutically acceptable acid addition salt can be formed by reaction of a compound of formula (I) with a suitable inorganic or organic acid (such as hydrobromic, hydrochloric, sulphuric, nitric, phosphoric, succinic, maleic, formic, acetic, propionic, fumaric, citric, tartaric, lactic, benzoic, salicylic, glutamic, aspartic, p-toluenesulfonic, benzenesulfonic, methanesulfonic, ethanesulfonic, naphthalenesulfonic (e.g. 2-naphthalenesulfonic), naphthalene disulfonic or hexanoic acid), optionally in a suitable solvent such as an organic solvent, to give the salt which is usually isolated for example by crystallisation and filtration. A pharmaceutically acceptable acid addition salt of a compound of formula (I) can comprise or be for example a hydrobromide, hydrochloride, sulfate, nitrate, phosphate, succinate, maleate, formate, acetate, propionate, fumarate, citrate, tartrate, lactate, benzoate, salicylate, glutamate, aspartate, p-toluenesulfonate, benzenesulfonate, methanesulfonate, ethanesulfonate, naphthalenesulfonate (e.g. 2-naphthalenesulfonate), naphthalene disulfonate or hexanoate salt.

Compounds of formula (I) in which R³ represents -NR⁵SO₂- or -SO₂NR⁶- may form base addition salts. Suitable pharmaceutically acceptable base salts include ammonium salts, alkali metal salts such as those of sodium and potassium, alkaline earth metal salts such as those of calcium and magnesium, and salts with organic bases whose pKₐ is >13.

Other non-pharmaceutically acceptable salts, e.g. oxalates or trifluoroacetates, may be used, for example in the isolation of the compounds of formula (I), and are included within the scope of this invention.

The invention includes within its scope all possible stoichiometric and non-stoichiometric forms of the salts of the compounds of formula (I).

It will be appreciated that many organic compounds can form complexes with solvents in which they are reacted or from which they are precipitated or crystallized. These complexes are known as "solvates". For example, a complex with water is known as a "hydrate". Solvents with high boiling points and/or capable of forming hydrogen bonds such as water, xylene, *N*-methyl pyrrolidinone, methanol and ethanol may be used to form solvates. Methods for identification of solvates include, but are not limited to, NMR and microanalysis. Solvates of the compounds of formula (I) are within the scope of the invention.

Compounds of formula (I) may exist in different physical forms. Such forms are within the scope of the present invention. Thus, the compounds of formula (I) may be in a crystalline or amorphous state. Furthermore, if crystalline, the compounds of formula (I) may exist in one or more polymorphic forms, which are included in the scope of the present invention. The most thermodynamically stable polymorphic form, at room temperature, of compounds of formula (I) is of particular interest.

Polymorphic forms of compounds of formula (I) may be characterized and differentiated using a number of conventional analytical techniques, including, but not limited to, X-ray powder diffraction (XRPD) patterns, infrared (IR) spectra, Raman spectra, differential scanning calorimetry (DSC), thermogravimetric analysis (TGA) and solid state nuclear magnetic resonance (ssNMR).

It will be appreciated that the compounds of formula (I) may possess one or more asymmetric carbon atoms so that optical isomers e.g. enantiomers or diastereoisomers may be formed. The present invention encompasses optical isomers of the compounds of formula (I) whether as individual isomers isolated such as to be substantially free of the other isomer (i.e. pure) or as mixtures thereof (e.g. racemates and racemic mixtures). An individual isomer isolated such as to be substantially free of the other isomer (i.e. pure) may be isolated such that less than about 10%, particularly less than about 1%, for example less than about 0.1 % of the other isomer is present.

Further, it will be appreciated that the R and S enantiomers may be isolated from the racemate by conventional resolution methods such as preparative HPLC involving a chiral stationary phase, by resolution using fractional crystallisation of a salt of the free base with a chiral acid, by chemical conversion to a diastereoisomer using a chiral auxiliary followed by chromatographic separation of the isomers and then removal of the chiral auxiliary and regeneration of the pure enantiomer, or by total asymmetric synthesis.

Certain compounds of formula (I) may exist in one of several tautomeric forms. It will be understood that the present invention encompasses tautomers of the compounds of formula (I) whether as individual tautomers or as mixtures thereof.

It will be appreciated from the foregoing that included within the scope of the invention are solvates (e.g. hydrates), complexes, tautomers, optical isomers and polymorphic forms of the compounds of formula (I) and salts thereof.

There is also provided processes for the preparation of compounds of formula (I) or salts thereof.

For the avoidance of doubt, throughout the process section, unless otherwise stated, (CH₂)ₙ corresponds to the C₁₋₆alkylene chain defined in R³ in the compound of formula (I), and thus may be optionally substituted by one C₁₋₃alkyl group.

According to a first process, A, a compound of formula (I) in which R⁴ represents -SO₂- may be prepared by reacting a compound of formula (II) 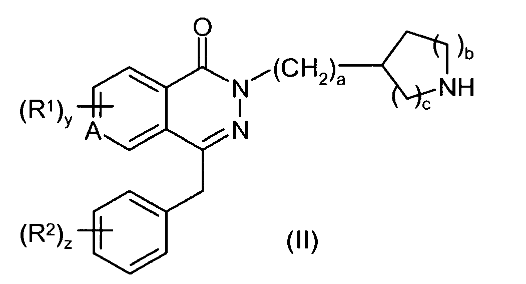 with a compound of formula (III) 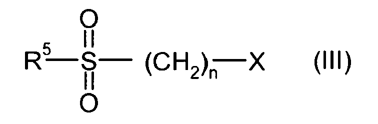 wherein A, R¹, R², a, b, c, y, z and R⁵ are as defined hereinabove for formula (I), n represents 1 to 6 and (CH₂)ₙ may be optionally substituted by one C₁₋₃alkyl group, and X represents a suitable leaving group such as chlorine, bromine, tosylate or mesylate.

The reaction may typically be carried out in a suitable solvent, such as *N*,*N*'-dimethylformamide (DMF), optionally using an appropriate activating agent, e.g. sodium iodide, with a suitable base, such as sodium bicarbonate or potassium carbonate. The reaction is typically heated, for example using a microwave oven at a temperature of about 100 to 150°C for an appropriate time, such as 15 to 30 min. Alternatively, the heating may be conducted using conventional methods for longer periods of time, such as for several hours or overnight as appropriate.

Compounds of formula (II) may be prepared according to Scheme 1 and Scheme 2 below.

Compounds of formula (III) in which X represents Cl or Br may be prepared according to scheme 4 and/or are commercially available. Examples of such compounds which are commercially available, for example from Apollo and/or Aldrich and/or Chemical Blocks and/or TCl Europe, include 1-[(2-chloroethyl)sulfonyl]pentane, 2-chloroethyl phenyl sulfone, *p-*toluenesulfonylmethyl chloride, 1-[(2-chloroethyl)sulfonyl]-4-methylbenzene, 2-chloroethyl 3-[(trifluoromethyl)phenyl] sulphone, 2-chloroethyl 4-fluorophenyl sulphone, 2-chloroethyl 4-chlorophenyl sulfone and 1-{[(2-chloroethyl)sulfonyl]methyl}benzene, bromomethylphenyl sulfone and 3,5-bis(trifluoromethyl)phenyl chloromethyl sulphone.

Compounds of formula (III) in which X represents tosylate or mesylate may be prepared according to scheme 5. 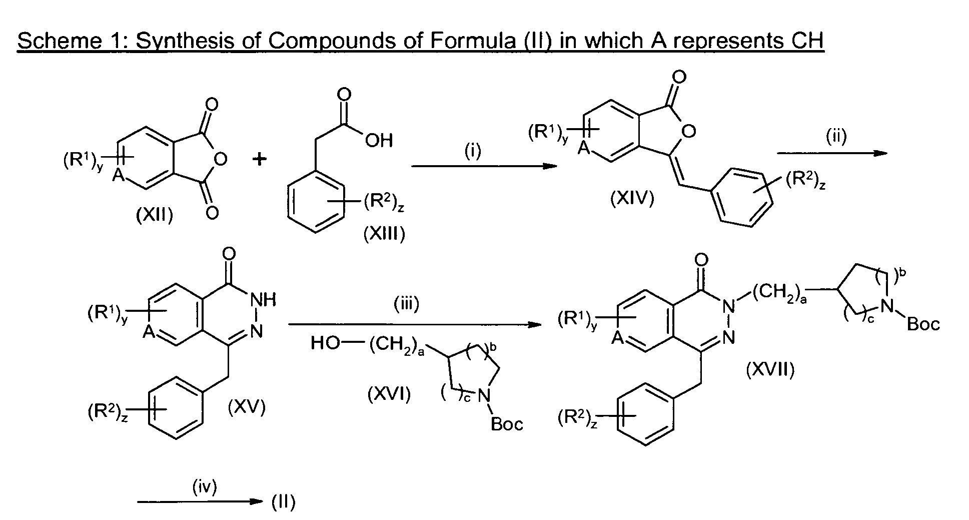 wherein R¹, R², a, b, c, y and z are as defined hereinabove for formula (I), Boc represents t*ert*-butoxycarbonyl and A represents CH.

**Reagents and Conditions**: i) elevated temperature such as about 180 to 250°C e.g. about 240 °C, suitable base e.g. sodium acetate (NaOAc), suitable solvent such as N-methyl-2-pyrrolidone (NMP); ii) NH₂NH₂, or hydrazine sulphate and sodium hydroxide (NaOH), in a suitable solvent such as ethanol; iii) suitable solvent e.g. tetrahydrofuran (THF), appropriate azodicarboxylate e.g. diisopropylazodicarboxylate (DIAD) or other reagent such as di-*tert-*butylazodicarboxylate (TBAD), suitable phosphine e.g. triphenylphosphine (PPh₃), optionally at a lowered temperature; iv) deprotection using an acid e.g. hydrogen chloride (HCl) or trifluoroacetic acid (TFA), in a suitable solvent e.g. 1,4-dioxane or dichloromethane (DCM).

In a modification of the synthesis described above, steps (iii) and (iv) may be performed sequentially, without isolation of the Boc-protected intermediate [compound (XVII)].

Compounds of formula (XII) are commercially available from Sigma-Aldrich, Apollo, Fluorochem, Apin, Davos and/or Merck, such as phthalic anhydride, 3-chlorophthalic anhydride, 4-chlorophthalic anhydride, 4-bromophthalic anhydride, 5-bromo-isobenzofuran-1,3-dione, 3-fluorophthalic anhydride, 4-fluorophthalic anhydride, 3,6-dichlorophthalic anhydride, 4,5-dichlorophthalic anhydride, 4,5-difluorophthalic anhydride, 3,6-difluorophthalic anhydride, 3-hydroxyphthalic anhydride and 4-methylphthalic anhydride and/or may be prepared using methods well known to those skilled in the art, for example 3,6-dihydroxyphthalic anhydride may be prepared from 3,6-diacetoxyphthalic anhydride, which is commercially available from Wako. C₁₋₆alkyl substituted phthalic anhydrides may be prepared using methods well known to those skilled in the art from the commercially available bromide compounds.

Compounds of formula (XIII) are commercially available from Sigma-Aldrich and/or Apollo, such as phenylacetic acid, 2-bromophenylacetic acid, 4-bromophenylacetic acid, 3-chlorophenylacetic acid, 4-chlorophenylacetic acid, 4-methylphenylacetic acid, 4-methoxyphenylacetic acid, 4-hydroxyphenylacetic acid, 3-(trifluoromethyl)phenylacetic acid, 4-(trifluoromethyl)phenylacetic acid, 2-fluoro-3-(trifluoromethyl)phenylacetic acid, 4-hydroxy-3-methoxyphenylacetic acid and 2,4-dimethoxyphenylacetic acid.

Compounds of formula (XIV), such as benzalphthalide, 4-fluorobenzylidene phthalide, 3-(2-bromo-benzylidene)-3H-isobenzofuran-1-one and 4-chlorobenzylidene phthalide are commercially available, for example, from Honeywell and/or Aldrich and/or Aurora Chemicals.

Compounds of formula (XVI) are commercially available from Sigma-Aldrich and/or Fluka, such as (*R*)-1-BOC-2-pyrrolidinemethanol, (S)-1-BOC-2-pyrrolidinemethanol and 1-BOC-4-hydroxypiperidine.

Compounds of formula (XVII) and (XV) are also disclosed in German patent application DE 3634942A1 and US patent 3,813,384, or may be prepared by the methods described herein. 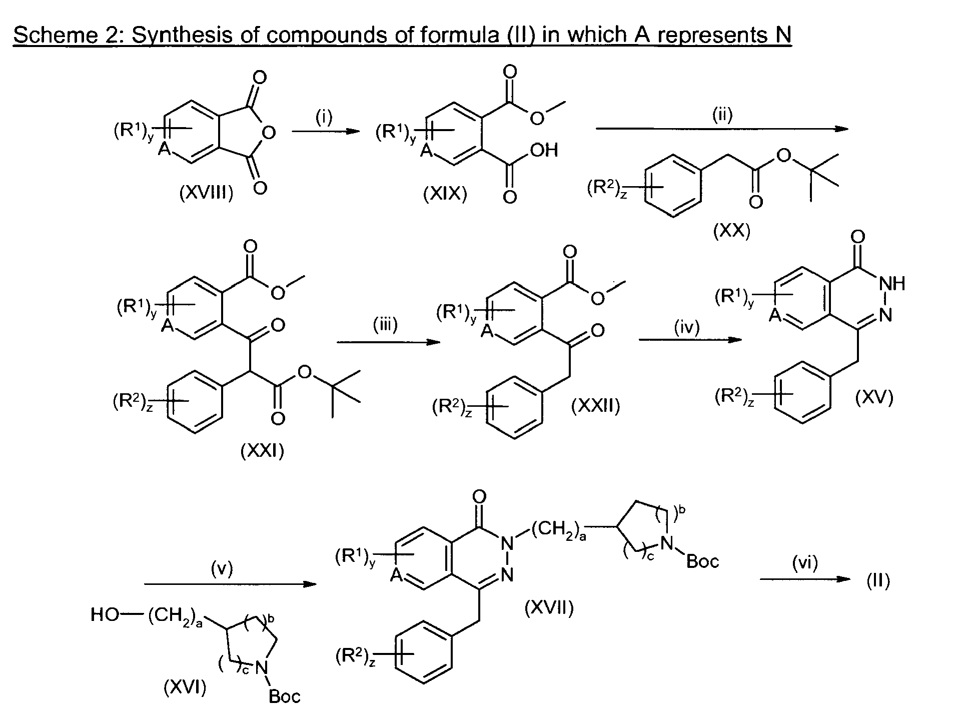 wherein R¹, R², a, b, c, y and z are as described hereinabove for formula (I), Boc represents *tert*-butoxycarbonyl and A represents N

**Reagents and conditions**: i) Sodium methoxide, THF/methanol(MeOH); ii) a) suitable activating agent such as carbonyl diimidazole or oxalyl chloride, suitable solvent such as DMF, appropriate elevated temperature such as at about 50 °C, b) appropriate base for example sodium hydride (NaH), c) compound of formula (XX); iii) suitable acid for example TFA, appropriate solvent such as DCM; iv) hydrazine or hydrazine monohydrate (commercially available, for example, from Aldrich), in an appropriate solvent for example ethanol, catalytic amount of acid such as acetic acid; v) suitable solvent e.g. THF, appropriate azodicarboxylate e.g. DIAD or other reagent such as TBAD, suitable phosphine e.g. PPh₃, optionally at a lowered temperature; vi) deprotection using an acid e.g. HCI or TFA in a suitable solvent e.g. 1,4-dioxane or DCM.

Compounds of formula (XVIII) in which A represents N are commercially available, or may be prepared from known methods. For example, pyridine-3,4-dicarboxylic anhydride is available from Sigma-Aldrich. 2-methyl-pyridine-4,5-dicarboxylic anhydride may be prepared according to the methods described by Werner, W. Graefe, U., Ihn, W., Tresselt, D., Winter, S., Paulus, E., Tetrahedron, 53(1):109-118 (1997), see compound 4. 3-Methoxypyridine-4,5-dicarboxylic anhydride may be prepared according to the methods disclosed by Krapcho, A. P., Maresch, M. J., Gallagher, C. E., Hacker, M. P., J. Het. Chem., 32(6):1693-702, (1995), see compound 10. 2-Methyl-3,4-pyridinedicarboxylic anhydride may be prepared according to the methods described by Moriconi, E. J. and Spano, F. A., J. Amer. Chem. Soc., 86(1):38-46, (1964), see compound 14.

Compounds of formula (XX) may be prepared by the methods described in Scheme 3, below, or by the methods described in WO 2002/079143 (see Preparation 149). 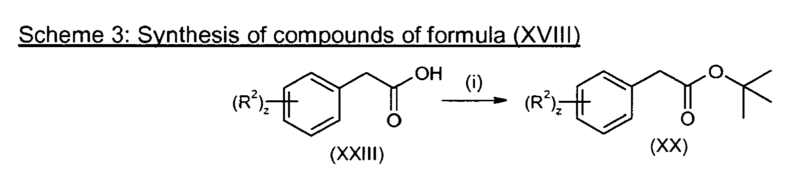 wherein R² and z are as described hereinabove for formula (I).

**Reagents and conditions**: i) dimethylformamide di-*tert*-butyl acetal, suitable solvent such as toluene, elevated temperatue, e.g. about 80°C, for approximately 18 h.

Dimethylformamide di-*tert*-butyl acetal is commercially available, for example, from Sigma-Aldrich.

Compounds of formula (XXIII) are commercially available from Sigma-Aldrich and/or Apollo, such as phenylacetic acid, 2-bromophenylacetic acid, 4-bromophenylacetic acid, 3-chlorophenylacetic acid, 4-chlorophenylacetic acid, 4-methylphenylacetic acid, 4-methoxyphenylacetic acid, 4-hydroxyphenylacetic acid, 3-(trifluoromethyl)phenylacetic acid, 4-(trifluoromethyl)phenylacetic acid, 2-fluoro-3-(trifluoromethyl)phenylacetic acid, 4-hydroxy-3-methoxyphenylacetic acid and 2,4-dimethoxyphenylacetic acid. 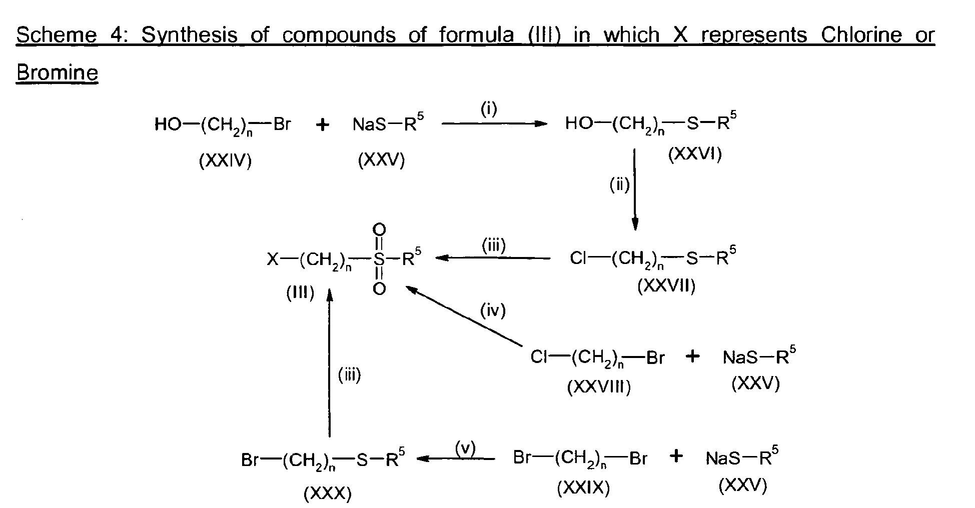 wherein R⁵ is as defined hereinabove for formula (I), n represents 1 to 6, (CH₂)ₙ may be optionally substituted by one C₁₋₃alkyl group and X represents chlorine or bromine.

**Reagents and Conditions:** i) suitable solvent such as DMF, at an elevated temperature such as from about 60 to 90 °C; ii) appropriate base e.g. triethylamine, in a suitable solvent for example DCM, methanesulfonylchloride (commercially available, for example, from Aldrich) and optionally in the presence of additional chloride ions e.g. lithium chloride or tetra-*n-*butylammonium chloride; iii) suitable solvent such as DCM, appropriate oxidising agent e.g. m-chloroperbenzoic acid (commercially available, for example, from Aldrich); iv) appropriate solvent such as ethanol or DMF, optionally at an appropriate elevated temperature e.g. from about 60 to 80°C, followed by treatment with an appropriate oxidising agent e.g. m-chloroperbenzoic acid in a suitable solvent e.g. DCM; v) suitable solvent such as DMF at an appropriate elevated temperature e.g. from about 60 to 80°C.

Compounds of formula (XXV) are commercially available, for example, from Aldrich, and include sodium ethanethiolate, sodium 1-propanethiolate, sodium 2-propanethiolate, sodium 1-butanethiolate, sodium 2-methyl-2-propanethiolate, sodium thiophenoxide and sodium 4-methylbenzenethiolate.

Compounds of formula (XXV) may also be prepared *in situ,* by the addition of a suitable base, such as sodium hydride to a solution of the corresponding thiol in a suitable solvent, such as DMF in an inert atmosphere such as under nitrogen or argon. The suspension may be left for an appropriate amount of time, e.g. about 15 min, before continuing with the reactions described in Scheme 4.

Thiol compounds corresponding to compounds of formula (XXV) are commercially available, for example, from Aldrich and/or TCI-Europe and/or Apollo, and include methanemercaptan, 2-methyl-2-butanethiol, 3-methyl-1-butanethiol, 1-pentanethiol, hexylmercaptan, cyclopentanethiol, cyclohexanethiol, 2-naphthalenethiol, thiophenol, 2-bromothiophenol, 4-fluorothiophenol, 2,5-dichlorothiophenol, 3-methylbenzenethiol, 2-ethylthiophenol, 2-*iso-*propylthiophenol, 2,4-dimethylthiophenol, benzyl mercaptan, phenylethylmercaptan, 2-chlorobenzyl mercaptan, 3-methylbenzyl mercaptan and 3,5-bis(trifluoromethyl)thiophenol.

Compounds of formula (XXIV) are commercially available, for example, from Aldrich and/or Apollo and/or TCI-Europe, and include 2-bromoethanol, 3-bromopropanol, 4-bromobutanol, 5-bromopentanol, 6-bromohexanol, 1-bromo-2-propanol, (*R*)-(-)-3-bromo-2-methyl-1-propanol, (*S*)-(-)-3-bromo-2-methyl-1-propanol and 1-bromo-2-butanol.

Compounds of formula (XXVI) may be prepared as described in Scheme 4, or may also be commercially available, for example, from TCI-Europe and/or Alfa Aesar and/or Aldrich, and include 2-(ethylthio)ethanol, 2-(*iso*-butylthio)ethanol, 4-(methylthio)-1-butanol, 3-(methylthio)-1-hexanol, 2-hydroxyethyl benzyl sulphide, 2-hydroxyethyl *n-*pentyl sulphide, 4-chlorobenzyl 2-hydroxyethyl sulphide and 3-(methylthio)-1-propanol.

Compounds of formula (XXVII) are also commercially available, for example, from Acros and/or Aldrich, and include 2-chloroethyl ethyl sulphide and 1-{[(2-chloroethyl)sulfonyl]methyl}benzene.

Compounds of formula (XXVIII) are commercially available, for example, from TCI-Europe and/or Aldrich and/or Alfa Aesar, and include 1-bromo-2-chloroethane, 2-bromo-1-chloropropane, 1-bromo-3-chloropropane, 1-bromo-4-chlorobutane, 1-bromo-3-chloro-2-methylpropane, 1-bromo-5-chloropentane and 1-bromo-6-chlorohexane.

Compounds of formula (XXIX) are commercially available, for example, from Aldrich, and/or Alfa Aesar and include dibromomethane, 1,2-dibromoethane, 1,2-dibromopropane, 1,2-dibromobutane, 1,3-dibromopropane, 1,3-dibromobutane, 1,4-dibromobutane, 1,4-dibromopentane, 1,5-dibromopentane, 1,5-dibromo-3-methylpentane and 1,6-dibromohexane. 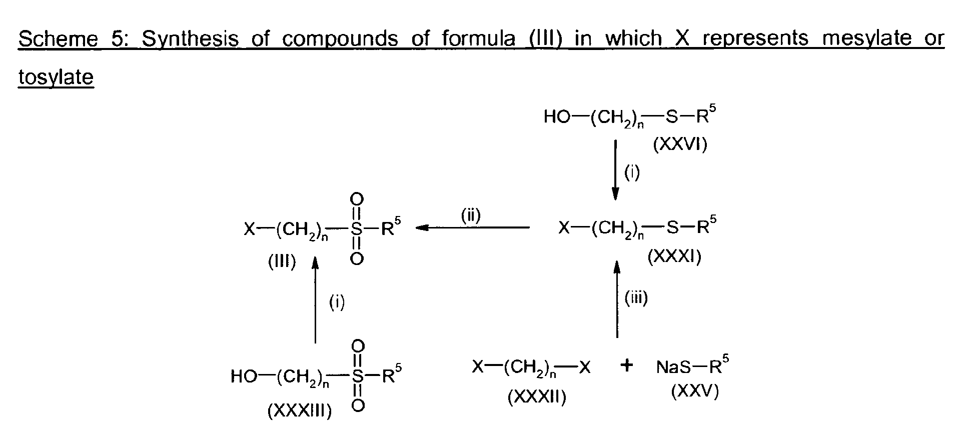 wherein R⁵ is as defined hereinabove for formula (I), n represents 1 to 6, (CH₂)ₙ may be optionally substituted by one C₁₋₃alkyl group, and X represents an activated hydroxyl group such as mesylate or tosylate.

**Reagents and Conditions**: i) suitable activating agent for example methylsulfonyl chloride or *p-*toluenesulfonyl chloride (both commercially available, for example, from Aldrich), suitable solvent such as pyridine or DCM, optionally at a suitable lowered temperature e.g. from about 0 to 5°C; ii) suitable solvent such as DCM, appropriate oxidising agent e.g. m-chloroperbenzoic acid; iii) suitable solvent such as DMF, optionally at an appropriate elevated temperature for example from about 70 to 80°C.

Compounds of formula (XXV) and (XXVI) are commercially available, see above (after Scheme 4).

Compounds of formula (XXV) may also be prepared *in situ,* by the addition of a suitable base, such as sodium hydride to a solution of the corresponding thiol in a suitable solvent, such as DMF. The suspension may be left for an appropriate amount of time, e.g. about 15 min, before continuing with the reactions described in Scheme 5.

Compounds of formula (XXXII) are commercially available, for example, from Aldrich, and include ethylene di(*p-*toluenesulfonate), (*S*)-(-)-1,2-propanediol di-*p-*tosylate, 1,3-propanediol di-*p-*tosylate and 1,4-butanediol dimethanesulfonate. Alternatively, compounds of formula (XXXII) may be prepared by methods well known to those skilled in the art, by activation of the corresponding diol. The reaction may typically be carried out using a suitable activating agent such as methanesulfonyl chloride, or *p-*toluenesulfonyl chloride in a suitable solvent such as DCM or pyridine. Diols corresponding to compounds of formula (XXXII) are commercially available, for example, from Aldrich, and include ethylene glycol, 1,2-butanediol, 1,3-propanediol, 1,3-butanediol, 1,4-butanediol, 1,4-pentanediol, 1,5-pentanediol, 1,5-hexanediol, 3-methyl-1 ,5-pentanediol and 1,6-hexanediol.

Compounds of formula (XXXIII) are commercially available, for example, from Aldrich and/or Alfa Aesar, and include 2-(methylsulfonyl)ethanol and 2-(ethanesulfonyl)ethanol.

In an alternative preparation, the compounds of formula (XXVI) which are HO-(CH₂)₂CH(Y)SR⁵ may be prepared according to Scheme 6 below: 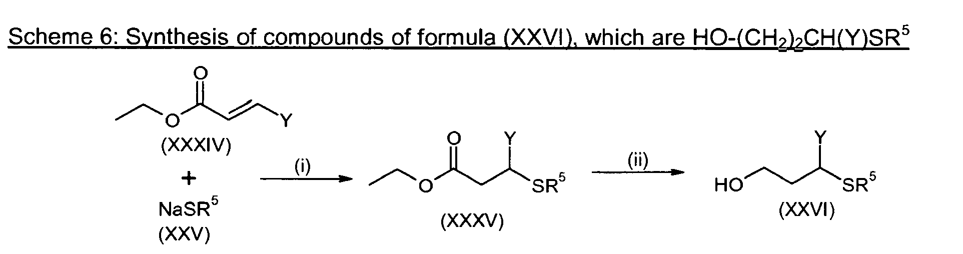 wherein R⁵ is as defined hereinabove for formula (I) and Y represents hydrogen or C₁₋₃alkyl.

**Reagents and Conditions:** i) suitable solvent such as DMF; ii) suitable solvent such as THF, appropriate reducing agent e.g. lithium aluminium hydride solution in ether, suitable lowered temperature such as from about 0 to 5°C.

The compounds of formula (XXXIV) are commercially available, for example from Aldrich and/or Alfa Aesar and/or Rarechem, and include ethyl acrylate, ethyl crotonate, ethyl *trans*-2-pentenoate, ethyl 4-methyl-*trans*-2-pentenoate and ethyl *trans*-2-hexenoate.

Compounds of formula (XXV) are commercially available, see above (after Scheme 4).

According to a second process, B, a compound of formula (I) in which R³ represents a saturated 5 to 7 membered ring containing one SO₂ group, or R³ represents ethylene-SO₂-R⁵, or R³ represents ethylene-SO₂N(R⁷)-R⁵ may be prepared by reacting a compound of formula (II) 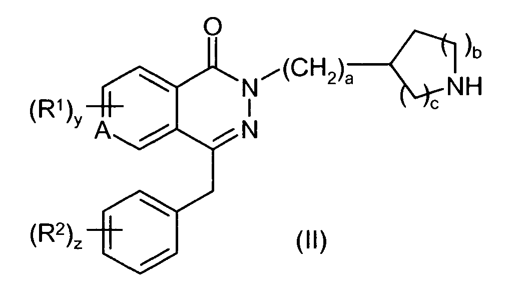 with a compound of formula (IV) or (IVa) or (IVb) 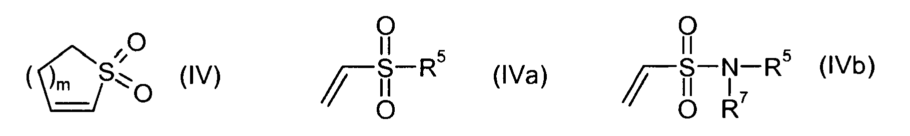 wherein A, R¹, R², a, b, c, y, z and R⁵ are as defined hereinabove for formula (I) and m represents 1 to 3.

The reaction may typically be carried out in a suitable solvent, such asTHF or DMF. Optionally, an appropriate base may be added, for example sodium bicarbonate. The reaction is typically heated for example using a microwave oven at a suitable temperature from about 100 to 150 °C for an appropriate time, such as about 15 to 30 min. Alternatively, the heating may be conducted using conventional methods at a suitable elevated temperature, such as from about 70 to 90°C for longer periods of time, e.g. about 2 to 3 hours or overnight.

Compounds of formula (II) may be prepared according to Scheme 1 and Scheme 2 above.

Compounds of formula (IV) are be commercially available or may be prepared according to methods disclosed herein. 2,3-dihydrothiophene 1,1-dioxide is commercially available, for example, from AKOS. 3,4-dihydro-2*H*-thiopyran 1,1-dioxide may be prepared according to the methods disclosed by X-F. Ren, E. Turos, C.H. Lake and M.R. Churchill, J. Org. Chem., 60:6468-6483, (1995), see page 6483. 2,3,4,5-tetrahydrothiepin 1,1-dioxide may be prepared according to the methods disclosed by B.F. Bonini, M. Comes-Franchini, M. Fochi, G. Mazzanti, A. Ricci, Tetrahedron, 52:4803-4816, (1996), see compound 12.

Compounds of formula (IVa) may be prepared according to methods described herein (see Intermediates 5 and 6) or are commercially available, for example, from Aldrich, and include methyl vinyl sulfone, ethyl vinyl sulfone and phenyl vinyl sulfone.

Compounds of formula (IVb) may be prepared according to methods described herein (see Intermediates 5 and 17).

According to a third process, C, a compound of formula (I) in which R⁴ represents - N(R⁶)SO₂- may be prepared by reacting a compound of formula (V) 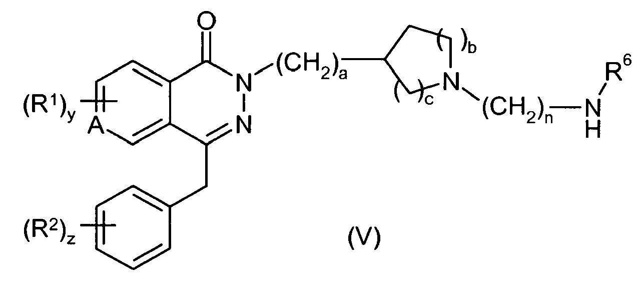 with a compound of formula (VI) 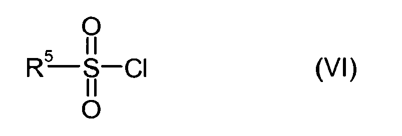 wherein A, R¹, R², a, b, c, y, Z, R⁵ and R⁶ are as defined hereinabove for formula (I), n represents 1 to 6 and (CH₂)ₙ may be optionally substituted by one C₁₋₃alkyl group.

The reaction may typically be carried out using a suitable solvent such as DCM with a suitable base e.g. triethylamine.

Compounds of formula (V) may be prepared according to the following reaction schemes (Schemes 7 and 8).

Compounds of formula (VI) are commercially available, for example, from Aldrich and/or TCI Europe and/or Apollo International and/or Fluorochem, and include methanesulfonyl chloride, ethanesulfonylchloride, 1-propanesulfonyl chloride, *iso*-propylsulfonyl chloride, 2-methyl-1-propylsulfonyl chloride, 1-butanesulfonyl chloride, sec-butylsulfonyl chloride, *n-*pentylsulfonyl chloride, 2-pentylsulfonyl chloride, 1-hexanesulfonyl chloride, cyclopentanesulfonyl chloride, cyclohexanesulfonyl chloride, cyclopentylmethanesulfonyl chloride cyclohexylmethanesutfonyl chloride, benzenesulfonyl chloride, 1-naphthalenesulfonyl chloride, 2-naphthalenesulfonyl chloride, 2-anthracenesulfonyl chloride, 4-ethylbenzenesulfonyl chloride, 4-*n-*propylbenzenesulfonyl chloride, 4-*iso-*propylbenzenesulfonyl chloride, 4-bromobenzenesulfonyl chloride, 4-iodobenzenesulfonyl chloride, 3-(trifluoromethyl)benzenesulfonyl chloride, 4-cyanobenzenesulfonylchloride, 2,5-dichlorobenzenesulfonyl chloride, 2-chloro-4-cyanobenzenesulfonyl chloride, benzylsulfonyl chloride, 2-(1-naphthyl)ethanesulfonyl chloride, 2-phenyl-ethanesulfonyl chloride, 4-chlorobenzylsulfonyl chloride, 4-methylbenzylsulfonyl chloride, 2-trifluoromethylbenzylsulfonyl chloride and 2-(4-chlorophenyl)-ethanesulfonyl chloride. 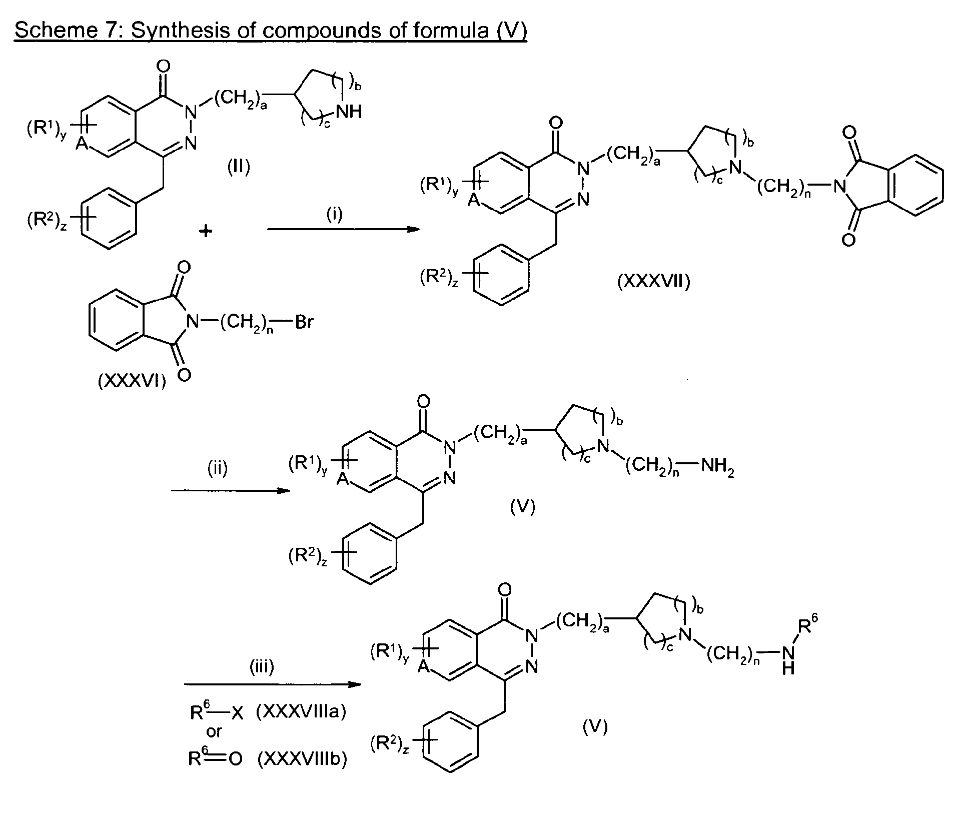 wherein A, R¹, R², a, b, c, y, z and R⁶ are as defined hereinabove for formula (I), n represents 1 to 6, (CH₂)ₙ may be optionally substituted by one C₁₋₃alkyl group and X represents a suitable leaving group such as chlorine, bromine or iodine.

**Reagents and Conditions**: i) suitable solvent such as 2-butanone, appropriate base e.g. potassium carbonate, at an elevated temperature such as from about 70 to 90 °C; ii) suitable solvent such as ethanol, hydrazine or hydrazine monohydrate, at an elevated temperature such as from about 70 to 90 °C; iii) 1 equivalent of R⁶-X (XXXVIIIa), in an appropriate solvent such as DMF, suitable base such as triethylamine or sodium hydride, optionally with an activating agent such as sodium iodide; or reductive amination using R⁶=O (XXXVIIIb), in a suitable solvent e.g. DMF, suitable reducing agent such as sodium triacetoxyborohydride.

The compounds of formula (XXXVI) are commercially available, for example from Acros and/or Aldrich, and include 2-(2-bromoethyl)-1*H*-isoindole-1,3(2*H*)-dione, *N-*(bromomethyl)phthalimide, *N*-(3-bromopropyl)phthalimide, *N*-(4-bromobutyl)phthalimide, *N-*(5-bromopentyl)phthalimide and *N*-(6-bromohexyl)phthalimide.

Compounds of formula (XXXVIIIa) are commercially available, for example from Aldrich, and include methyl iodide, iodoethane, 1-iodopropane, 1-iodobutane, 1-iodopentane and 1-iodohexane.

Compounds of formula (XXXVIIIb) are commercially available, for example, from Aldrich, and include formaldehyde, acetaldehyde, propionaldehyde, methyl ethyl ketone, butyraldehyde, valeraldehyde, 3-pentanone, hexanal, 3-hexanone and 3-methyl-3-pentanone. 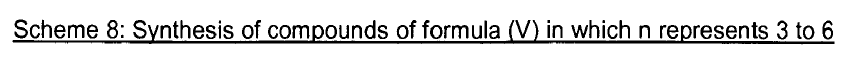 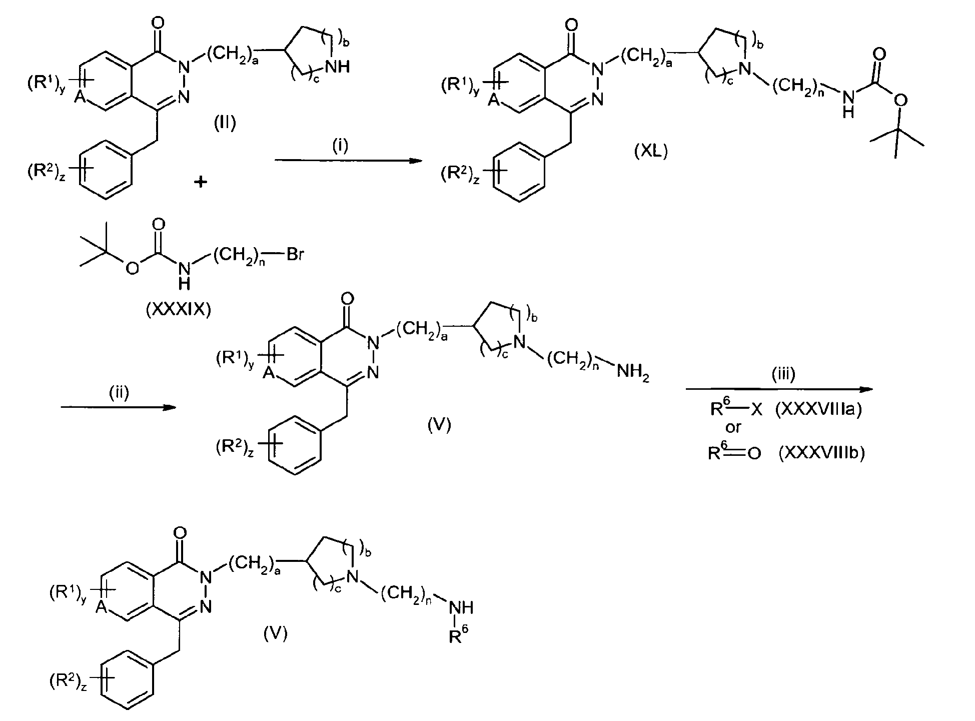 wherein A, R¹, R², a, b, c, y, z, and R⁶ are as defined hereinabove for formula (I), X represents a suitable leaving group such as chlorine, bromine or iodine, n represents 1 to 6 and (CH₂)ₙ may be optionally substituted by one C₁₋₃alkyl group.

**Reagents and Conditions**: i) suitable solvent such as 2-butanone, appropriate base e.g. potassium carbonate, at an elevated temperature such as from about 70 to 90 °C, optionally with an activating agent such as sodium iodide; ii) deprotection using a suitable acid such as hydrogen chloride or TFA in a suitable solvent e.g. dioxane or DCM; iii) 1 equivalent of R⁶-X (XXXVIIIa), in an appropriate solvent such as DMF, suitable base such as triethylamine or sodium hydride, optionally with an activating agent such as sodium iodide; or reductive amination using R⁶=O (XXXVIIIb), in a suitable solvent e.g. DMF, suitable reducing agent such as sodium triacetoxyborohydride.

Compounds of formula (XXXIX) are commercially available, for example, from Aldrich and/or Toronto Chemicals, and include 2-(Boc-amino)ethyl bromide, 3-(Boc-amino)propyl bromide, 4-(Boc-amino)butyl bromide, 5-(Boc-amino)pentyl bromide and 6-(Boc-amino)hexyl bromide.

Compounds of formula (XXXVIIIa) and (XXXVIIIb) are commercially available, see above (after Scheme 7).

According to a fourth process, D, a compound of formula (I) in which R⁴ represents - N(R⁶)SO₂- may be prepared by reacting a compound of formula (II) 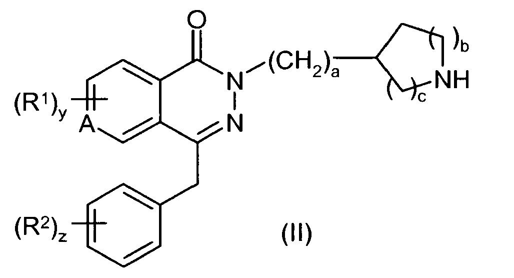 with a compound of formula (VII) 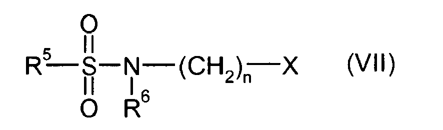 wherein A, R¹, R², a, b, c, y, Z, R⁵ and R⁶ are as defined hereinabove for formula (I), n represents 1 to 6, (CH₂)ₙ may be optionally substituted by one C₁₋₃alkyl group, and X represents a suitable leaving group such as chlorine, bromine, tosylate or mesylate.

The reaction may typically be carried out using a suitable base such as sodium hydrogen carbonate, with an appropriate activating agent e.g. sodium iodide, in a suitable solvent such as DMF. The reaction is typically heated for example, using a microwave oven at an appropriate elevated temperature for example from about 140 to 160°C, for about 10 to 30 minutes, as appropriate. Alternatively, heating may be with conventional apparatus, at elevated temperatures for example from about 50 to 70°C, for a time about 3 hours to overnight, as appropriate.

Compounds of formula (II) may be prepared according to Scheme 1 and Scheme 2 above.

Compounds of formula (VII) in which X represents chlorine or bromine are commercially available, for example, from Apollo, and include *N*-(2-bromoethyl)-4-chlorobenzene-1-sulfonamide, *N*-(2-bromoethyl)-4-fluorobenzene-1-sulphonamide, *N*-(2-bromoethyl)-3-(trifluoromethyl)benzene-1-sulphonamide, *N*-(2-bromoethyl)-2,4-dichlorobenzene sulfonamide and 4-Bromo-*N*-(3-chloropropyl)benzene sulphonamide.

Compounds of formula (VII) in which X represents mesylate or tosylate may be prepared according to Scheme 9 below. 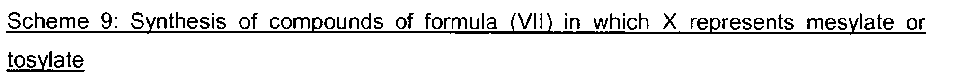 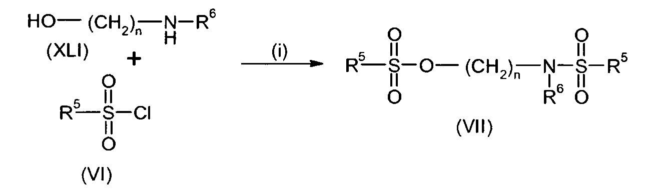 wherein R⁵ and R⁶ are as defined hereinabove for formula (I), n represents 1 to 6 and (CH₂)ₙ may be optionally substituted by one C₁₋₃alkyl group.

**Reagents and Conditions:** i) suitable solvent such as DCM, appropriate base e.g. triethylamine, at a lowered temperature such as from about 0 °C to room temperature.

Compounds of formula (VI) are commercially available, see above (described after Process C).

Compounds of formula (XLI) are commercially available, for example, from Aldrich and/or TCI Europe, and include 2-aminoethanol, 2-(methylamino)ethanol, 2-(ethylamino)ethanol, 2-(propylamino)ethanol, 2-(butylamino)ethanol, 2-(*n-*pentylamino)ethanol, 3-amino-1-propanol, 3-(methylamino)-1-propanol, 4-amino-1-butanol, (R)-4-amino-2-methyl-1-butanol, 4-ethylamino-1-butanol, 4-(*n-*butylamino)-1-butanol, 5-amino-1-pentanol and 6-amino-1-hexanol.

According to a fifth process, E, a compound of formula (I) in which R⁴ represents -SO₂N(R⁷)-may be prepared by reacting a compound of formula (II) 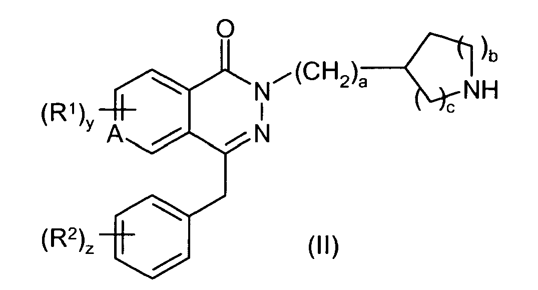 with a compound of formula (VIII) 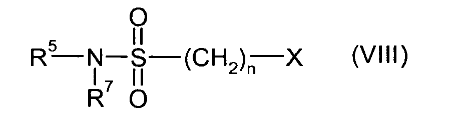 wherein A, R¹, R², a, b, c, y, Z, R⁵ and R⁷ are as defined hereinabove for formula (I), n represents 1 to 6, (CH₂)ₙ may be optionally substituted by one C₁₋₃alkyl group, and X represents a suitable leaving group such as chlorine or bromine.

The reaction may typically be carried out using a suitable solvent such as DMF with an appropriate activating agent for example, sodium iodide, with a suitable base, e.g. potassium carbonate. The reaction is usually heated using conventional apparatus, at an appropriate elevated temperature for example from about 50 to 70°C, for about 3 hours to overnight, as appropriate.

Compounds of formula (II) may be prepared according to Scheme 1 and Scheme 2 above.

Compounds of formula (VIII) may be prepared according to Scheme 10 below. 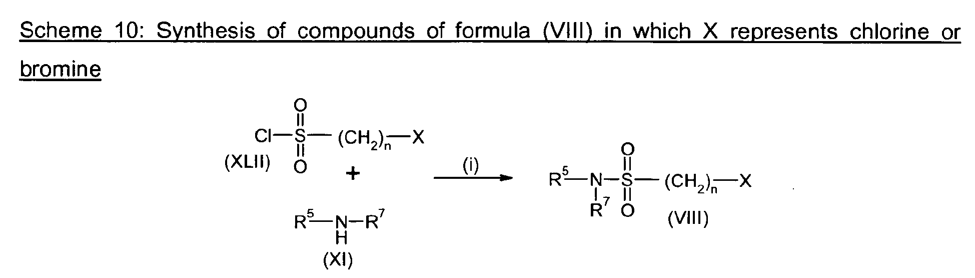 wherein R⁵ and R⁷ are as defined hereinabove for formula (I), n represents 1 to 6 and (CH₂)ₙ may be optionally substituted by one C₁-₃alkyl group.

**Reagents and Conditions**: i) suitable solvent such as DCM, at a lowered temperature e.g. from about 0 °C to room temperature.

Compounds of formula (XLII) are commercially available, for example, from Aldrich and/or TCI Europe, and include 2-chloroethanesulfonyl chloride and 3-chloropropanesulfonyl chloride.

Compounds of formula (XI) are commercially available, for example, from Aldrich and/or ABCR and/or Enamine and/or Chembridge, and include methylamine, ethylamine, propylamine, butylamine, (*R*)-(-)-2-aminobutane, (*S*)-(+)-2-aminobutanepentylamine, *tert-*butylamine, 1,1-dimethylpropylamine, hexylamine, dimethylamine, *N*-ethylmethylamine, *N-*methylpropylamine, diethylamine, dipropylamine, *N*-ethylbutylamine, dibutylamine, dipentylamine, dihexylamine, cyclopentylamine, cyclohexylamine, 2-methylcyclohexylamine, cycloheptylamine, *N*-methylcyclohexylamine, *N*-isopropylcyclohexyamine, *N*-cycloheptyl-*N-*methylamine, *N*-(*sec*-butyl)cycloheptanamine, *N*-(1-ethylpropyl)cycloheptanamine, *N-*isopropylcycloheptanamine, cyclohexanemethylamine, cycloheptanemethylamine, 2-cyclohexylethylamine, aniline, 9-aminophenanthrene, 1-aminoanthracene, 2-aminobenzonitrile, 2-fluoroaniline, 4-chloroaniline, 3-bromoaniline, 3-iodoaniline, 1-amino-2-methylnaphthalene, 2-methylaniline, 3-ethylaniline, 4-propylaniline, 2-isopropylaniline, 2-aminobenzotrifluoride, 3,5-bis(trifluoromethyl)aniline, 3-amino-4-fluorobenzotrifluoride, 5-fluoro-2-methylaniline, *N*-ethyl-1-naphthalene, *N*-methylaniline, *N*-ethylaniline, *N*-butylaniline, *N*-hexylaniline, *N*-ethyl-3-methylaniline, benzylamine, 2-phenylethylamine, 2-(3-chlorophenyl)ethylamine, 3-phenylpropylamine, (3-phenylpropyl)methylamine, *N-*methylphenethylamine, (2-phenylethyl)propylamine, cyclopentylamine, cyclohexylamine, cycloheptylamine, morpholine, thiomorphline, piperazine and *N*-methylpiperazine.

According to a sixth process, F, a compound of formula (I) in which R⁴ represents - N(R⁸)C(O)N(R⁹)-, and R⁹ represents hydrogen, may be prepared by reacting a compound of formula (Va) 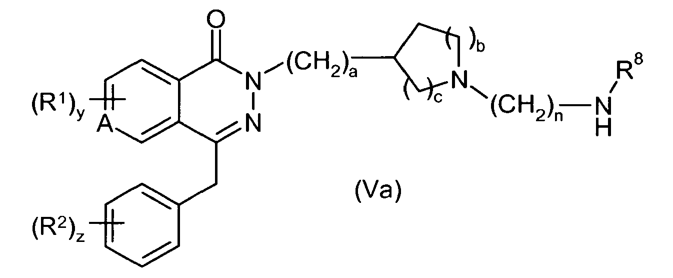 with a compound of formula (IX)

R⁵-N=C=O (IX)

wherein A, R¹, R², a, b, c, y, Z, R⁵ and R⁸ are as defined hereinabove for formula (I), n represents 1 to 6 and (CH₂)ₙ may be optionally substituted by one C₁₋₃alkyl group.

The reaction may typically be carried out using a suitable solvent, such as DCM. The reaction is usually carried out at ambient temperature for an appropriate length of time such as overnight, for example.

Compounds of formula (Va) may be prepared according to Schemes 7 and 8 above, in which R⁶ is R⁸.

Compounds of formula (IX) are commercially available, for example, from Aldrich, and include ethyl isocyanate, isopropyl isocyanate, propyl isocyanate, butyl isocyanate, sec-butyl isocyanate, *tert*-butyl isocyanate, pentyl isocyanate, hexyl isocyanate, cyclopentyl isocyanate, cyclohexyl isocyanate, cycloheptyl isocyanate, cyclohexanemethyl isocyanate, (*R*)-(-)-1-cyclohexylethyl isocyanate, phenyl isocyanate, 3-chlorophenyl isocyanate, 2-fluoro-phenyl isocyanate, 2-bromophenyl isocyanate, 4-iodophenyl isocyanate, 4-methylphenyl isocyanate, 2-ethylphenyl isocyanate, 2-*iso*propylphenyl isocyanate, 2-(trifluoromethyl)phenyl isocyanate, 3-cyanophenyl isocyanate, 2,3-dimethylphenyl isocyanate, 3-chloro-4-methylphenyl isocyanate, 4-bromo-2-(trifluoromethyl)phenyl isocyanate, 2-isopropyl-6-methylphenyl isocyanate, benzyl isocyanate, phenethyl isocyanate, 3-phenylpropyl isocyanate, (*S*)-(-)-1-phenylpropyl isocyanate, 3-methylbenzyl isocyanate, 4-fluorobenzyl isocyanate, 2,4-dichlorobenzyl isocyanate and 4-ethylphenethyl isocyanate.

According to a seventh process, G, a compound of formula (I) in which R⁴ represents - N(R⁸)C(O)N(R⁹)- may be prepared by reacting a compound of formula (X) 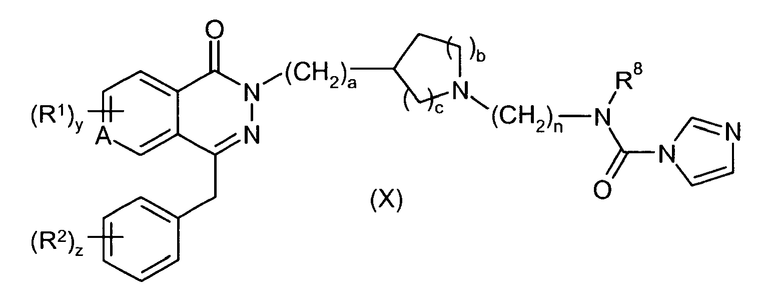 with a compound of formula (Xla) 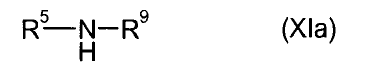 wherein A, R¹, R², a, b, c, y, z, R⁵, R⁸ and R⁹ are as defined hereinabove for formula (I), n represents 1 to 6 and (CH₂)ₙ may be optionally substituted by one C₁₋₃alkyl group.

The reaction may typically be carried out in a suitable solvent such as THF or DCM, usually at an elevated temperature for example at reflux.

Compounds of formula (X) may be prepared according to Scheme 11 below.

Compounds of formula (XIa) are commercially available, for which see compounds of formula (XI) in which R⁷ is R⁹ (see after Scheme 10, above). 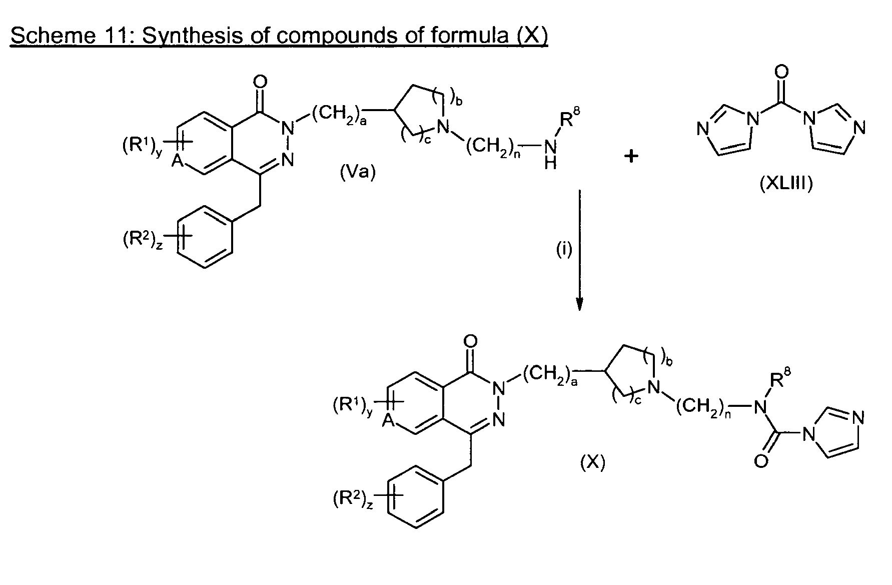 wherein A, R¹, R², a, b, c, y, z and R⁸ are as defined hereinabove for formula (I), n represents 1 to 6 and (CH₂)ₙ may be optionally substituted by one C₁₋₃alkyl group.

**Reagents and Conditions**: i) 1 equivalent 1,1'-carbonyldiimidazole, in an appropriate solvent such as THF or DCM.

Compounds of formula (Va) may be prepared according to Schemes 7 and 8 above, in which R⁶ is R⁸.

The compound of formula (XLIII), 1,1'-carbonyldiimidazole, is commercially available, for example, from Aldrich.

According to an eighth process, H, a compound of formula (I), may be prepared by interconversion from other compounds of formula (I).

Interconversions include, but are not limited to alkylation and deprotection, under standard conditions well known to those skilled in the art.

Thus, typically, an alkylation reaction may be carried out between a compound of formula (I) and a C₁₋₆alkyl, activated to substitution by means of a leaving group such as halogen or an activated hydroxyl group, such as mesylate or tosylate. The reaction usually takes place in the presence of a suitable base such as triethylamine, *N*.*N*-diisopropylethylamine or sodium hydride, in an appropriate solvent such as 2-butanone or DMF, optionally at an appropriate elevated temperature such as at about 80°C.

According to a ninth process, I, a salt of a compound of formula (I) may be prepared by exchange of counterions, or precipitation of said salt from the free base.

Examples of protecting groups that may be employed in the synthetic routes described and the means for their removal can be found in T. W. Greene *et al.* 'Protective Groups in Organic Synthesis' (3rd edition, J. Wiley and Sons, 1999). Suitable amine protecting groups include sulfonyl (e.g. tosyl), acyl (e.g. acetyl, 2',2',2'-trichloroethoxycarbonyl, benzyloxycarbonyl or t-butoxycarbonyl) and arylalkyl (e.g. benzyl), which may be removed by hydrolysis (e.g. using an acid such as hydrogen chloride in dioxane or trifluoroacetic acid in dichloromethane) or reductively (e.g. hydrogenolysis of a benzyl group or reductive removal of a 2',2',2'-trichloroethoxycarbonyl group using zinc in acetic acid) as appropriate. Other suitable amine protecting groups include trifluoroacetyl (-COCF₃), which may be removed by base catalysed hydrolysis or a solid phase resin bound benzyl group, such as a Merrifield resin bound 2,6-dimethoxybenzyl group (Ellman linker), which may be removed by acid cleavage, for example with trifluoroacetic acid.

Compounds of formula (I) or a pharmaceutical acceptable salt thereof may be useful for the treatment of various inflammatory and/or allergic diseases.

Examples of disease states in which a compound of formula (I), or a pharmaceutically acceptable salt thereof may have potentially beneficial anti-inflammatory and/or anti-allergic effects include inflammatory and/or allergic diseases of the respiratory tract, such as allergic rhinitis (seasonal and perennial) or other diseases such as bronchitis (including chronic bronchitis), asthma (including allergen-induced asthmatic reactions), chronic obstructive pulmonary disease (COPD) and sinusitis.

Furthermore, the compounds of formula (I) may be of use in the treatment of nephritis, skin diseases such as psoriasis, eczema, allergic dermatitis and hypersensitivity reactions. Also, the compounds of formula (I) may be useful in the treatment of insect bites and stings.

The compounds of formula (I) may also be of use in the treatment of nasal polyposis, conjunctivitis (e.g. allergic conjunctivitis) or pruritis.

A disease of particular interest is allergic rhinitis.

Other diseases in which histamine may have a pathophysiological role include non-allegic rhinitis, and also diseases of the gastrointestinal tract such as intestinal inflammatory diseases including inflammatory bowel disease (e.g. Crohn's disease or ulcerative colitis) and intestinal inflammatory diseases secondary to radiation exposure or allergen exposure.

It will be appreciated by those skilled in the art that references herein to treatment or therapy may extend to prophylaxis as well as the treatment of established conditions.

As mentioned above, compounds of formula (I) may be useful as therapeutic agents. There is thus provided a compound of formula (I) or a pharmaceutically acceptable salt thereof for use in therapy.

In another embodiment, there is provided a compound of formula (I) or a pharmaceutically acceptable salt thereof for use in the treatment of inflammatory and/or allergic diseases (such as any of the above diseases, in particular allergic rhinitis).

In another embodiment, there is provided the use of a compound of formula (I) or a pharmaceutically acceptable salt thereof for the manufacture of a medicament for the treatment of inflammatory and/or allergic diseases (such as any of the above diseases, in particular allergic rhinitis).

In another embodiment, there is provided a compound for the treatment (or prophylaxis) of inflammatory and/or allergic diseases (such as any of the above diseases, in particular allergic rhinitis), in a patient in need thereof, which comprises administering an effective amount of a compound of formula (I) or a pharmaceutically acceptable salt thereof.

When used in therapy, the compounds of formula (I) or pharmaceutically acceptable salts thereof may typically be formulated in a suitable pharmaceutical composition. Such pharmaceutical compositions may be prepared using standard procedures.

Thus, there is provided a composition which comprises a compound of formula (I) or a pharmaceutically acceptable salt thereof and one or more (e.g. 10 or fewer) pharmaceutically acceptable carriers and/or excipients.

A composition comprising a compound of formula (I) or a pharmaceutically acceptable salt thereof, which may be prepared by admixture, suitably at ambient temperature and atmospheric pressure, may be suitable for topical administration (which includes epicutaneous, inhaled, intranasal or ocular administration), enteral administration (which includes oral or rectal administration) or parenteral administration (such as by injection or infusion). Of interest are compositions comprising a compound of formula (I) or a pharmaceutically acceptable salt thereof, suitable for topical administration, particularly suitable for intranasal administration.

Generally, compositions may be in the form of solutions or suspensions (aqueous or non-aqueous), tablets, capsules, oral liquid preparations, powders, granules, lozenges, lotions, creams, ointments, gels, foams, reconstitutable powders or suppositories as required by the route of administration.

Generally, the compositions comprising a compound of formula (I) or a pharmaceutically acceptable salt thereof may contain from about 0.1% to 99% (w/w), such as from about 10 to 60% (w/w) (based on the total weight of the composition), of the compound of formula (I) or the pharmaceutically acceptable salt thereof, depending on the route of administration. The dose of the compound used in the treatment of the aforementioned diseases will vary in the usual way with the seriousness of the diseases, the weight of the sufferer, and other similar factors. However, as a general guide, suitable unit doses may be about 0.05 to 1000 mg, for example about 0.05 to 200 mg, and such unit doses may be administered more than once a day, for example two or three times a day or as desired. Such therapy may extend for a number of weeks or months.

The proportion of the compound of formula (I) or a pharmaceutically acceptable salt thereof in a topical composition will depend on the precise type of composition to be prepared and the particular route of administration, but will generally be within the range of from about 0.001 to 10% (w/w), based on the total weight of the composition. Generally, however for most types of preparations the proportion used will be within the range of from about 0.005 to 1% (w/w), such as about 0.01 to 1% (w/w), for example about 0.01 to 0.5% (w/w), based on the total weight of the composition. However, in powders for inhalation the proportion used will generally be within the range of from about 0.1 to 5% (w/w), based on the total weight of the composition.

Generally, compositions suitable for intranasal or inhaled administration may conveniently be formulated as aerosols, solutions, suspensions, drops, gels or dry powders, optionally with one or more pharmaceutically acceptable carriers and/or excipients such as aqueous or non-aqueous vehicles, thickening agents, isotonicity adjusting agents, antioxidants, preservatives and/or co-solvents.

For compositions suitable for intranasal or inhaled administration, the compound of formula (I) or a pharmaceutically acceptable salt thereof may typically be in a particle-size-reduced form, which may be prepared by conventional techniques, for example, micronisation, milling and/or microfluidisation. Generally, the size-reduced (e.g. micronised) compound of formula (I) or a pharmaceutically acceptable salt thereof can be defined by a D₅₀ value of about 0.5 to 10 microns, for example of about 1 to 10 microns, such as of about 2 to 4 microns (for example as measured using laser diffraction).

In one embodiment, compositions comprising a compound of formula (I) or a pharmaceutically acceptable salt thereof are suitable for intranasal administration. Intranasal compositions comprising a compound of formula (I) or a pharmaceutically acceptable salt thereof may permit the compound(s) to be delivered to all areas of the nasal cavities (the target tissue) and further, may permit the compound(s) to remain in contact with the target tissue for longer periods of time. A suitable dosing regime for intranasal compositions would be for the patient to inhale slowly through the nose subsequent to the nasal cavity being cleared. During inhalation the composition would be administered to one nostril while the other is manually compressed. This procedure would then be repeated for the other nostril. Typically, one or two administrations per nostril would be administered by the above procedure up to two or three times each day, ideally once daily. Of particular interest are intranasal compositions suitable for once daily administration.

The intranasal compositions containing a compound of formula (I) or a pharmaceutically acceptable salt thereof may be in the form of an aqueous suspension and/or an aqueous solution. Partial suspensions and/or partial solutions are encompassed within the scope of the present invention. Compositions comprising one compound which is in solution and another compound which is in suspension are also included within the scope of the present invention.

Intranasal compositions may optionally contain one or more suspending/thickening agents, one or more preservatives, one or more wetting agents and/or one or more isotonicity adjusting agents as desired. Compositions suitable for intranasal administration may optionally further contain other excipients, such as antioxidants (for example sodium metabisulphite), taste-masking agents (such as menthol) and sweetening agents (for example dextrose, glycerol, saccharin and/or sorbitol).

The skilled person would readily appreciate that some excipients may perform more than one function, depending on the nature and number of excipients used in the composition and the particular properties of the therapeutic compound(s) and other carriers and/or excipients contained therein.

The suspending/thickening agent, if included, will typically be present in the intranasal composition in an amount of between about 0.1 and 5% (w/w), such as between about 1.5% and 2.4% (w/w), based on the total weight of the composition. Examples of suspending/thickening agents include, but are not limited to Avicel® (microcrystalline cellulose and carboxymethylcellulose sodium), carboxymethylcellulose sodium, veegum, tragacanth, bentonite, methylcellulose xanthan gum, carbopol and polyethylene glycols. Suspending/thickening agents may also be included in compositions suitable for inhaled, ocular and oral administration as appropriate.

For stability purposes, intranasal compositions comprising a compound of formula (I) or a pharmaceutically acceptable salt thereof may be protected from microbial or fungal contamination and growth by inclusion of a preservative. Examples of pharmaceutically acceptable anti-microbial agents or preservatives may include quaternary ammonium compounds (e.g. benzalkonium chloride, benzethonium chloride, cetrimide, myristal picolinium chloride, lauralkonium chloride and cetylpyridinium chloride), mercurial agents (e.g. phenylmercuric nitrate, phenylmercuric acetate and thimerosal), alcoholic agents (e.g. chlorobutanol, phenylethyl alcohol and benzyl alcohol), antibacterial esters (e.g. esters of para-hydroxybenzoic acid), chelating agents such as disodium ethylenediaminetetraacetate (EDTA) and other anti-microbial agents such as chlorhexidine, chlorocresol, sorbic acid and its salts (such as potassium sorbate) and polymyxin. Examples of pharmaceutically acceptable anti-fungal agents or preservatives include, but are not limited to sodium benzoate, sorbic acid, sodium propionate, methyl paraben, ethyl paraben, propyl paraben and butyl paraben. The preservative, if included, may be present in an amount of between about 0.001 and 1% (w/w), such as about 0.015% (w/w), based on the total weight of the composition. Preservatives may be included in compositions suitable for other routes of administration as appropriate.

Compositions which contain a suspended medicament may include a pharmaceutically acceptable wetting agent which functions to wet the particles of medicament to facilitate dispersion thereof in the aqueous phase of the composition. Typically, the amount of wetting agent used will not cause foaming of the dispersion during mixing. Examples of wetting agents include, but are not limited to fatty alcohols, esters and ethers, such as polyoxyethylene (20) sorbitan monooleate (Polysorbate 80) macrogol ethers and poloxamers. The wetting agent may be present in intranasal compositions in an amount of between about 0.001 and 0.05% (w/w), for example about 0.025% (w/w), based on the total weight of the composition. Wetting agents may be included in compositions suitable for other routes of administration, e.g. for inhaled and/or ocular administration, as appropriate.

An isotonicity adjusting agent may be included to achieve isotonicity with body fluids e.g. fluids of the nasal cavity, resulting in reduced levels of irritancy. Examples of isotonicity adjusting agents include, but are not limited to sodium chloride, dextrose, xylitol and calcium chloride. An isotonicity adjusting agent may be included in intranasal compositions in an amount of between about 0.1 and 10% (w/w), for example between about 4.5 to 5.5% (w/w), such as about 5.0% (w/w), based on the total weight of the composition. Isotonicity adjusting agents may also be included in compositions suitable for other routes of administration, for example in compositions suitable for inhaled, ocular, oral liquid and parenteral administration, as appropriate.

One or more co-solvent(s) may be included to aid solubility of the active compound(s) and/or other excipients. Examples of pharmaceutically acceptable co-solvents include, but are not limited to, propylene glycol, dipropylene glycol, ethylene glycol, glycerol, ethanol, polyethylene glycols (for example PEG300 or PEG400) and methanol. The co-solvent(s), if present, may be included in an amount of from about 0.05 to 20% (w/w), such as from about 1.5 to 17.5% (w/w), or from about 1.5 to 7.5% (w/w), or from about 0.05% to 0.5% (w/w) based on the total weight of the composition. Co-solvents may also be included in compositions suitable for other routes of administration, as appropriate.

Further, the intranasal compositions comprising a compound of formula (I) or a pharmaceutically acceptable salt thereof may be buffered by the addition of suitable buffering agents such as sodium citrate, citric acid, trometarol, phosphates such as disodium phosphate (for example the dodecahydrate, heptahydrate, dihydrate and anhydrous forms) or sodium phosphate and mixtures thereof. Buffering agents may also be included in compositions suitable for other routes of administration as appropriate.

Compositions for administration topically to the nose (for example, for the treatment of rhinitis) or lung include pressurised aerosol compositions and aqueous compositions delivered to the nasal cavities by pressurised pump. Compositions which are non-pressurised and adapted to be administered topically to the nasal cavity are of particular interest. Suitable compositions contain water as the diluent or carrier for this purpose. Aqueous compositions for administration to the lung or nose may be provided with conventional excipients such as buffering agents, tonicity modifying agents and the like. Aqueous compositions may also be administered to the nose by nebulisation.

A fluid dispenser may typically be used to deliver a fluid composition to the nasal cavities. The fluid composition may be aqueous or non-aqueous, but typically aqueous. Such a fluid dispenser may have a dispensing nozzle or dispensing orifice through which a metered dose of the fluid composition is dispensed upon the application of a user-applied force to a pump mechanism of the fluid dispenser. Such fluid dispensers are generally provided with a reservoir of multiple metered doses of the fluid composition, the doses being dispensable upon sequential pump actuations. The dispensing nozzle or orifice may be configured for insertion into the nostrils of the user for spray dispensing of the fluid composition into the nasal cavity. A fluid dispenser of the aforementioned type is described and illustrated in WO05/044354 the entire content of which is hereby incorporated herein by reference. The dispenser has a housing which houses a fluid discharge device having a compression pump mounted on a container for containing a fluid composition. The housing has at least one finger-operable side lever which is movable inwardly with respect to the housing to cam the container upwardly in the housing to cause the pump to compress and pump a metered dose of the composition out of a pump stem through a nasal nozzle of the housing. In one embodiment, the fluid dispenser is of the general type illustrated in Figures 30-40 of WO05/044354.

Aqueous compositions containing a compound of formula (I) or a pharmaceutically acceptable salt thereof may also be delivered by a pump as disclosed in WO2007/138084, for example as disclosed with reference to Figures 22-46 thereof, or as disclosed in GB0723418.0, for example as disclosed with reference to Figures 7-32 thereof, both of which prior patent applications are incorporated herein by reference in their entirety. The pump may be actuated by an actuator as disclosed in Figures 1-6 of said GB0723418.0.

In one embodiment, there is provided an intranasal composition comprising a compound of formula (I) or a pharmaceutically acceptable salt thereof. In another embodiment, such an intranasal composition is benzalkonium chloride-free.

Inhaled administration involves topical administration to the lung, such as by aerosol or dry powder composition.

Aerosol compositions suitable for inhaled administration may comprise a solution or fine suspension of the compound in a pharmaceutically acceptable aqueous or non-aqueous solvent. Aerosol compositions suitable for inhalation can be either a suspension or a solution and generally contain a compound of formula (I) or a pharmaceutically acceptable salt thereof and a suitable propellant such as a fluorocarbon or hydrogen-containing chlorofluorocarbon or mixtures thereof, such as hydrofluoroalkanes, e.g. 1,1,1,2-tetrafluoroethane, 1,1,1,2,3,3,3-heptafluoro-*n*-propane or a mixture thereof. The aerosol composition may optionally contain additional excipients well known in the art such as surfactants or cosolvents. Examples of surfactants include, but are not limited to oleic acid, lecithin, an oligolactic acid or derivative e.g. as described in WO94/21229 and WO98/34596. An example of a cosolvent includes, but is not limited to ethanol. Aerosol compositions may be presented in single or multidose quantities in sterile form in a sealed container, which may take the form of a cartridge or refill for use with an atomising device or inhaler. Alternatively, the sealed container may be a unitary dispensing device such as a single dose nasal inhaler or an aerosol dispenser fitted with a metering valve (metered dose inhaler), which is intended for disposal once the contents of the container have been exhausted.

Dry powder inhalable compositions may take the form of capsules and cartridges of, for example, gelatine, or blisters of, for example, laminated aluminium foil, for use in an inhaler or insufflator. Such compositions may be formulated comprising a powder mix of a compound of formula (I) or a pharmaceutically acceptable salt thereof and a suitable powder base such as lactose or starch.

Optionally, for dry powder inhalable compositions, a composition suitable for inhaled administration may be incorporated into a plurality of sealed dose containers (e.g. comprising the dry powder composition) mounted longitudinally in a strip or ribbon inside a suitable inhalation device. The container is rupturable or peel-openable on demand and the dose of e.g. the dry powder composition may be administered by inhalation via the device such as the DISKUS^{®} device, marketed by GlaxoSmith Kline. The DISKUS^{®} inhalation device is for example described in GB 2242134 A, and in such a device, at least one container for the composition in powder form (the container or containers may, for example, be a plurality of sealed dose containers mounted longitudinally in a strip or ribbon) is defined between two members peelably secured to one another; the device comprises: a means of defining an opening station for the said container or containers; a means for peeling the members apart at the opening station to open the container; and an outlet, communicating with the opened container, through which a user can inhale the composition in powder form from the opened container.

Aerosol compositions are typically arranged so that each metered dose or "puff' of aerosol contains about 20 µg - 2000 µg, particularly about 20 µg - 500 µg of a compound of formula (I) or a pharmaceutically acceptable salt thereof. Administration may be once daily or several times daily, for example 2, 3, 4 or 8 times, giving for example 1, 2 or 3 doses each time. The overall daily dose with an aerosol will be within the range of about 100 µg - 10 mg, such as between about 200 µg - 2000 µg. The overall daily dose and the metered dose delivered by capsules and cartridges in an inhaler or insufflator will generally be double those with aerosol compositions.

In another embodiment, there is provided a composition comprising a compound of formula (I) or a pharmaceutically acceptable salt thereof which is suitable for epicutaneous administration. An epicutaneous composition to be applied to the affected area e.g. the skin, by one or more application per day, may be in the form of, for example, an ointment, a cream, an emulsion, a lotion, a foam, a spray, an aqueous gel, or a microemulsion. Such compositions may optionally contain one or more solubilising agents, skin-penetration-enhancing agents, surfactants, fragrances, preservatives or emulsifying agents.

Ointments, creams and gels, may, for example, be formulated with an aqueous or oily base with the addition of suitable thickening and/or gelling agent and/or solvents. Such bases may thus, for example, include water and/or an oil such as liquid paraffin or a vegetable oil such as arachis oil or castor oil, or a solvent such as polyethylene glycol. Thickening agents and gelling agents which may be used according to the nature of the base include soft paraffin, aluminium stearate, cetostearyl alcohol, polyethylene glycols, woolfat, beeswax, carboxypolymethylene and cellulose derivatives, and/or glyceryl monostearate and/or nonionic emulsifying agents. Lotions may be formulated with an aqueous or oily base and will in general also contain one or more emulsifying agents, stabilising agents, dispersing agents, suspending agents or thickening agents.

In another embodiment, there is provided a composition comprising a compound of formula (I) or a pharmaceutically acceptable salt thereof which is suitable for ocular administration. Such compositions may optionally contain one or more suspending agents, one or more preservatives, one or more wetting/lubricating agents and/or one or more isotonicity adjusting agents. Examples of ophthalmic wetting/lubricating agents may include cellulose derivatives, dextran 70, gelatin, liquid polyols, polyvinyl alcohol and povidone such as cellulose derivatives and polyols.

In another embodiment, there is provided a composition comprising a compound of formula (I) or a pharmaceutically acceptable salt thereof which is suitable for oral administration.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colorants.

In another embodiment, there is provided a composition comprising a compound of formula (I) or a pharmaceutically acceptable salt thereof which is suitable for parenteral administration. Fluid unit dosage forms suitable for parenteral administration may be prepared utilising a compound of formula (I) or pharmaceutically acceptable salt thereof and a sterile vehicle which may be aqueous or oil based. The compound, depending on the vehicle and concentration used, may be either suspended or dissolved in the vehicle. In preparing solutions, the compound may be dissolved for injection and filter sterilised before filling into a suitable vial or ampoule and sealing. Optionally, adjuvants such as a local anaesthetic, preservatives and buffering agents may be dissolved in the vehicle. To enhance the stability, the composition may be frozen after filling into the vial and the water removed under vacuum. The lyophilised parenteral composition may be reconstituted with a suitable solvent just prior to administration. Parenteral suspensions may be prepared in substantially the same manner, except that the compound is suspended in the vehicle instead of being dissolved, and sterilisation cannot be accomplished by filtration. The compound may be sterilised by exposure to ethylene oxide before suspension in a sterile vehicle. A surfactant or wetting agent may be included in the composition to facilitate uniform distribution of the compound.

The compounds and pharmaceutical compositions according to the invention may also be used in combination with or include one or more (e.g. one or two) other therapeutic agents, for example other antihistaminic agents for example H4 or H3 receptor antagonists, anticholinergic agents, anti-inflammatory agents such as corticosteroids (e.g. fluticasone propionate, fluticasone furoate, beclomethasone dipropionate, mometasone furoate, triamcinolone acetonide, budesonide and the steroid disclosed in WO02/12265), non-steroidal anti-inflammatory drugs (NSAIDs) (e.g. sodium cromoglycate, nedocromil sodium), PDE-4 inhibitors, leukotriene antagonists, lipoxygenase inhibitors, chemokine antagonists (e.g. CCR3, CCR1, CCR2, CCR4, CCR8, CXCR1, CXCR2), IKK antagonists, iNOS inhibitors, tryptase and elastase inhibitors, beta-2 integrin antagonists and adenosine 2a agonists; or beta adrenergic agents (e.g. salmeterol, salbutamol, formoterol, fenoterol, terbutaline, and the beta agonists described in WO 02/66422, WO 02/270490, WO02/076933 WO03/024439 and WO03/072539 and salts thereof); or antiinfective agents e.g. antibiotic agents and antiviral agents.

It will be clear to a person skilled in the art that, where appropriate, the other therapeutic agent(s) may be used in the form of salts, (e.g. as alkali metal or amine salts or as acid addition salts), or prodrugs, or as esters (e.g. lower alkyl esters), or as solvates (e.g. hydrates) to optimise the activity and/or stability and/or physical characteristics (e.g. solubility) of the therapeutic agent. It will be clear also that where appropriate, the therapeutic agents may be used in optically pure form.

There is provided, in another embodiment, a combination comprising a compound of formula (I) or a pharmaceutically acceptable salt thereof together with one or more (such as one or two, e.g. one) other therapeutically active agents, and one or more pharmaceutically acceptable carriers and/or excipients.

In another embodiment, there is provided a combination comprising a compound of formula (I) or a pharmaceutically acceptable salt thereof and an H3 and/or H4 antagonist.

Other histamine receptor antagonists which may be used alone, or in combination with an H1 receptor antagonist include antagonists (and/or inverse agonists) of the H4 receptor, for example, the compounds disclosed in Jablonowski et al., J. Med. Chem. 46:3957-3960 (2003), and antagonists (and/or inverse agonists) of the H3 receptor, for example the compounds described in WO2004/035556, the compounds described in WO2006/125665 and the compounds described in WO2006/090142.

In another embodiment, there is provided a combination comprising a compound of formula (I) or a pharmaceutically acceptable salt thereof and a β₂-adrenoreceptor agonist.

Examples of β₂-adrenoreceptor agonists include salmeterol (which may be a racemate or a single enantiomer, such as the *R*-enantiomer), salbutamol (which may be a racemate or a single enantiomer such as the *R*-enantiomer), formoterol (which may be a racemate or a single diastereomer such as the *R*,*R*-diastereomer), salmefamol, fenoterol, carmoterol, etanterol, naminterol, clenbuterol, pirbuterol, flerbuterol, reproterol, bambuterol, indacaterol, terbutaline and salts thereof, for example the xinafoate (1-hydroxy-2-naphthalenecarboxylate) salt of salmeterol, the sulfate salt or free base of salbutamol or the fumarate salt of formoterol. In one embodiment, combinations containing a compound of formula (I) may include longer-acting β₂-adrenoreceptor agonists, for example, compounds which provide effective bronchodilation for about 12 h or longer.

Other β₂-adrenoreceptor agonists include those described in WO 02/066422, WO 02/070490, WO 02/076933, WO 03/024439, WO 03/072539, WO 03/091204, WO 04/016578, WO 2004/022547, WO 2004/037807, WO 2004/037773, WO 2004/037768, WO 2004/039762, WO 2004/039766, WO01/42193 and WO03/042160.

Examples of β₂-adrenoreceptor agonists include:
3-(4-{[6-({(2*R*)-2-hydroxy-2-[4-hydroxy-3-(hydroxymethyl)phenyl]ethyl}amino)hexyl]oxy} butyl)benzenesulfonamide;
3-(3-{[7-({(2*R*)-2-hydroxy-2-[4-hydroxy-3-hydroxymethyl)phenyl]ethyl}-amino)heptyl]oxy} propyl)benzenesulfonamide;
4-{(1*R*)-2-[(6-{2-[(2,6-dichlorobenzyl)oxy]ethoxy}hexyl)amino]-1-hydroxyethyl}-2-(hydroxyl methyl) phenol;
4-{(1*R*)-2-[(6-{4-[3-(cyclopentylsulfonyl)phenyl]butoxy}hexyl)amino]-1-hydroxyethyl}-2-(hydroxylmethyl)phenol;
N-[2-hydroxyl-5-[(1*R*)-1-hydroxy-2-[[2-4-[[(2*R*)-2-hydroxy-2-phenylethyl]amino]phenyl] ethyl]amino]ethyl]phenyl]formamide;
N-2{2-[4-(3-phenyl-4-methoxyphenyl)aminophenyl]ethyl}-2-hydroxy-2-(8-hydroxy-2(1*H*)-quinolinon-5-yl)ethylamine; and
5-[(*R*)-2-(2-{4-[4-(2-amino-2-methyl-propoxy)-phenylamino]-phenyl}-ethylamino)-1-hydroxy - ethyl]-8-hydroxy-1H-quinolin-2-one.

The β₂-adrenoreceptor agonist may be in the form of a salt formed with a pharmaceutically acceptable acid selected from sulfuric, hydrochloric, fumaric, hydroxynaphthoic (for example 1- or 3-hydroxy-2-naphthoic), cinnamic, substituted cinnamic, triphenylacetic, sulfamic, sulfanilic, naphthaleneacrylic, benzoic, 4-methoxybenzoic, 2- or 4-hydroxybenzoic, 4-chlorobenzoic and 4-phenylbenzoic acid.

In another embodiment, there is provided a combination comprising a compound of formula (I) or a pharmaceutically acceptable salt thereof and an anti-inflammatory agent.

Anti-inflammatory agents include corticosteroids. Suitable corticosteroids which may be used in combination with the compounds of formula (I) are those oral and inhaled corticosteroids and their pro-drugs which have anti-inflammatory activity. Examples include methyl prednisolone, prednisolone, dexamethasone, fluticasone propionate, 6α,9α-difluoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester, 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester (fluticasone furoate), 6α,9α-difluoro-11β-hydroxy-16α-methyl-3-oxo-17α-propionyloxy-androsta-1,4-diene-17β-carbothioic acid *S*-(2-oxo-tetrahydro-furan-3S-yl) ester, 6α,9α-difluoro-11β-hydroxy-16α-methyl-3-oxo-17α-(2,2,3,3-tetramethycyclo propylcarbonyl)oxy-androsta-1,4-diene-17β-carbothioic acid *S*-cyanomethyl ester and 6α,9α-difluoro-11β-hydroxy-16α-methyl-17α-(1-methycyclopropylcarbonyl)oxy-3-oxo-androsta-1,4-diene-17β-carbothioic acid *S*-fluoromethyl ester, beclomethasone esters (for example the 17-propionate ester or the 17,21-dipropionate ester), budesonide, flunisolide, mometasone esters (for example mometasone furoate), triamcinolone acetonide, rofleponide, ciclesonide (16α,17-[[(*R*)-cyclohexylmethylene]bis(oxy)]-11β,21-dihydroxy-pregna-1,4-diene-3,20-dione), butixocort propionate, RPR-106541, and ST-126. Corticosteroids of particular interest may include fluticasone propionate, 6α,9α-difluoro-11β-hydroxy-16α-methyl-17α-[(4-methyl-1,3-thiazole-5-carbonyl)oxy]-3-oxo-androsta-1,4-diene-17β-carbothioic acid S-fluoromethyl ester, 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester, 6α,9α-difluoro-11β-hydroxy-16α-methyl-3-oxo-17α-(2,2,3,3-tetramethycyclopropylcarbonyl)oxy-androsta-1,4-diene-17β-carbothioic acid *S*-cyano methylester, 6α,9α-difluoro-11β-hydroxy-16α-methyl-17α-(1-methycyclopropylcarbonyl) oxy-3-oxo-androsta-1,4-diene-17β-carbothioic acid *S-*fluoromethyl ester and mometasone furoate. In one embodiment the corticosteroid is 6α,9α-difluoro-17α-[(2-furanylcarbonyl)oxy]-11β-hydroxy-16α-methyl-3-oxo-androsta-1,4-diene-17β-carbothioic acid S-fluoromethyl ester (fluticasone furoate) or mometasone furoate.

There is provided, in a further embodiment, a combination comprising a compound of formula (I) or a pharmaceutically acceptable salt thereof, together with a corticosteroid, such as fluticasone propionate or 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester (fluticasone furoate) or mometasone furoate. In a further embodiment there is provided a combination comprising a compound of formula (I) or a pharmaceutically acceptable salt thereof, together with 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester (fluticasone furoate). Such combinations may be of particular interest for intranasal administration.

In another embodiment, there is provided a combination comprising a compound of formula (I) or a pharmaceutically acceptable salt thereof and a glucocorticoid agonist.

Non-steroidal compounds having glucocorticoid agonism that may possess selectivity for transrepression over transactivation and that may be useful in combination therapy include those covered in the following patent application and patents: WO03/082827, WO98/54159, WO04/005229, WO04/009017, WO04/018429, WO03/104195, WO03/082787, WO03/082280, WO03/059899, WO03/101932, WO02/02565, WO01/16128, WO00/66590, WO03/086294, WO04/026248, WO03/061651, WO03/08277, WO06/000401, WO06/000398 and WO06/015870.

Anti-inflammatory agents include non-steroidal anti-inflammatory drugs (NSAID's).

NSAID's include sodium cromoglycate, nedocromil sodium, phosphodiesterase (PDE) inhibitors (e.g. theophylline, PDE4 inhibitors or mixed PDE3/PDE4 inhibitors), leukotriene antagonists, inhibitors of leukotriene synthesis (eg. montelukast), iNOS (inducible nitric oxide synthase) inhibitors (e.g. oral iNOS inhibitors), IKK antagonists, tryptase and elastase inhibitors, beta-2 integrin antagonists and adenosine receptor agonists or antagonists (e.g. adenosine 2a agonists), cytokine antagonists (e.g. chemokine antagonists, such as a CCR1, CCR2, CCR3, CCR4, or CCR8 antagonists) or inhibitors of cytokine synthesis, or 5-lipoxygenase inhibitors. iNOS inhibitors include those disclosed in WO93/13055, WO98/30537, WO02/50021, WO95/34534 and WO99/62875.

In another embodiment there is provided the use of the compounds of formula (I) or a pharmaceutically acceptable salt thereof in combination with a phosphodiesterase 4 (PDE4) inhibitor. The PDE4-specific inhibitor useful in this embodiment may be any compound that is known to inhibit the PDE4 enzyme or which is discovered to act as a PDE4 inhibitor, and which are only PDE4 inhibitors, not compounds which inhibit other members of the PDE family, such as PDE3 and PDE5, as well as PDE4.

Compounds which may be of interest include *cis*-4-cyano-4-(3-cyclopentyloxy-4-methoxyphenyl)cyclohexan-1-carboxylic acid, 2-carbomethoxy-4-cyano-4-(3-cyclopropylmethoxy-4-difluoromethoxyphenyl)cyclohexan-1-one and *cis*-[4-cyano-4-(3-cyclopropylmethoxy-4-difluoromethoxyphenyl)cyclohexan-1-ol]. Also, cis-4-cyano-4-[3-(cyclopentyloxy)-4-methoxyphenyl]cyclohexane-1-carboxylic acid (also known as cilomilast) and its salts, esters, pro-drugs or physical forms, which is described in U.S. patent 5,552,438 issued 03 September, 1996.

Other PDE4 inhibitors include AWD-12-281 from Elbion (Hofgen, N. et al., 15th EFMC Int. Symp. Med. Chem., (Sept 6-10, Edinburgh) 1998, Abst. P. 98; CAS reference No. 247584020-9); a 9-benzyladenine derivative nominated NCS-613 (INSERM); D-4418 from Chiroscience and Schering-Plough; a benzodiazepine PDE4 inhibitor identified as CI-1018 (PD-168787) and attributed to Pfizer; a benzodioxole derivative disclosed by Kyowa Hakko in WO99/16766; K-34 from Kyowa Hakko; V-11294A from Napp (Landells, L.J. et al., Eur. Resp. J. [Ann. Cong. Eur. Resp. Soc. (Sept 19-23, Geneva) 1998] 1998, 12 (Suppl. 28): Abst P2393); roflumilast (CAS reference No 162401-32-3) and a pthalazinone (WO99/47505) from Byk-Gulden; Pumafentrine, (-)-p*-*[(4*aR**,10*bS**)-9-ethoxy-1,2,3,4,4a,10b-hexahydro-8-methoxy-2-methylbenzo[c][1,6]naphthyridin-6-yl]-*N*,*N*-diisopropylbenzamide which is a mixed PDE3/PDE4 inhibitor which has been prepared and published on by Byk-Gulden, now Altana; arofylline under development by Almirall-Prodesfarma; VM554/UM565 from Vernalis; or T-440 (Tanabe Seiyaku; Fuji, K. et al., J. Pharmacol. Exp. Ther., 284(1):162, (1998)), and T2585.

Further PDE4 inhibitors which may be of interest are disclosed in the published international patent applications WO04/024728 (Glaxo Group Ltd), WO04/056823 (Glaxo Group Ltd) and WO04/103998 (Glaxo Group Ltd). A particular compound of interest is 6-({3-[(di methylamino)carbonyl]phenyl}sulfonyl)-8-methyl-4-{[3-(methyloxy)phenyl]amino}-3-quinolinecarboxamide or a pharmaceutically acceptable salt thereof, which is described in International Patent Application WO04/103998.

In another embodiment, there is provided a combination comprising a compound of formula (I) or a pharmaceutically acceptable salt thereof and an anticholinergic agent.

Anticholinergic agents are those compounds that act as antagonists at the muscarinic receptors, in particular those compounds which are antagonists of the M₁ or M₃ receptors, dual antagonists of the M₁/M₃ or M₂/M₃, receptors or pan-antagonists of the M₁/M₂/M₃ receptors. Exemplary compounds for administration *via* inhalation include ipratropium (for example, as the bromide, CAS 22254-24-6, sold under the name Atrovent), oxitropium (for example, as the bromide, CAS 30286-75-0) and tiotropium (for example, as the bromide, CAS 136310-93-5, sold under the name Spiriva). Also of interest are revatropate (for example, as the hydrobromide, CAS 262586-79-8) and LAS-34273 which is disclosed in WO01/04118. Exemplary compounds for oral administration include pirenzepine (for example, CAS 28797-61-7), darifenacin (for example, CAS 133099-04-4, or CAS 133099-07-7 for the hydrobromide sold under the name Enablex), oxybutynin (for example, CAS 5633-20-5, sold under the name Ditropan), terodiline (for example, CAS 15793-40-5), tolterodine (for example, CAS 124937-51-5, or CAS 124937-52-6 for the tartrate, sold under the name Detrol), otilonium (for example, as the bromide, CAS 26095-59-0, sold under the name Spasmomen), trospium chloride (for example, CAS 10405-02-4) and solifenacin (for example, CAS 242478-37-1, or CAS 242478-38-2, or the succinate also known as YM-905 and sold under the name Vesicare).

Other anticholinergics may be found in WO 2004/012684; WO2004/091482; WO2005/009439; WO2005/009362; WO2005/009440; WO2005/037280; WO2005/037224; WO2005/046586; WO2005/055940; WO2005/05594; WO2005/067537; WO2005/087236; WO2005/086873; WO2005/094835; WO2005/094834; WO2005/094251; WO2005/095407; WO2005/099706; WO2005/104745; WO2005/112644; WO2005/118594; WO2006/005057; WO2006/017768; WO2006/017767; WO2006/050239; WO2006/055553; WO2006/055503; WO2006/065755; WO2006/065788; WO2007/018514; WO2007/018508; WO2007/016650; WO2007/016639; and WO2007/022351.

Other anticholinergic agents include compounds which are disclosed in US patent application 60/487981, published as WO2005/009439 and those compounds disclosed in US patent application 60/511009, published as WO2005/037280.

The individual compounds of such combinations may be administered either sequentially in separate pharmaceutical compositions as well as simultaneously in combined pharmaceutical compositions. Additional therapeutically active ingredients may be suspended in the composition together with a compound of formula (I). Appropriate doses of known therapeutic agents will be readily appreciated by those skilled in the art.

Compounds of formula (I) may be prepared by the methods described below or by similar methods. Thus the following Intermediates and Examples illustrate the preparation of the compounds of formula (I), and are not to be considered as limiting the scope of the disclosure in any way.

### GENERAL EXPERIMENTAL

### Abbreviations

- h:: Hours
- HPLC:: High performance liquid chromatography
- LCMS:: Liquid-chromatography mass-spectroscopy
- MDAP:: Mass-directed auto-preparative HPLC
- min: Minutes

### General Experimental Procedures

Flash silica gel refers to Merck Art No. 9385; silica gel refers to Merck Art No. 7734.
SCX cartridges are Ion Exchange SPE columns where the stationary phase is polymeric benzene sulfonic acid. These are used to isolate amines.
SCX2 cartridges are Ion Exchange SPE columns where the stationary phase is polymeric propylsulfonic acid. These are used to isolate amines.

LCMS was conducted on a Supelcosil LCABZ+PLUS column (3.3 cm × 4.6 mm ID) eluting with 0.1 % formic acid and 0.01 M ammonium acetate in water (solvent A) and 0.05% formic acid 5% water in MeCN (solvent B), using the following elution gradient 0.0 - 7 min 0% B, 0.7 - 4.2 min 100% B, 4.2 - 5.3 min 0% B, 5.3 - 5.5min 0% B at a flow rate of 3 mlmin⁻¹. The mass spectra were recorded on a Fisons VG Platform spectrometer using electrospray positive and negative mode (ES+ve and ES-ve).

The Flashmaster II is an automated multi-user flash chromatography system, available from Argonaut Technologies Ltd, which utilises disposable, normal phase, SPE cartridges (2 g to 100 g). It provides quaternary on-line solvent mixing to enable gradient methods to be run. Samples are queued using the multi-functional open access software, which manages solvents, flow-rates, gradient profile and collection conditions. The system is equipped with a Knauer variable wavelength UV-detector and two Gilson FC204 fraction-collectors enabling automated peak cutting, collection and tracking.

Mass directed autopreparative (MDAP) HPLC was conducted on a Waters FractionLynx system comprising of a Waters 600 pump with extended pump heads, Waters 2700 autosampler, Waters 996 diode array and Gilson 202 fraction collector on a 10 cm × 2.54 cm internal diameter ABZ+ column, eluting with 0.1 % formic acid in water (solvent A) and 0.1 % formic acid in MeCN (solvent B), using an appropriate elution gradient over 15 min (or 25 min) at a flow rate of 20 mlmin⁻¹ and detecting at 200 - 320 nm at room temperature. Mass spectra were recorded on Micromass ZMD mass spectrometer using electro spray positive and negative mode, alternate scans. The software used was MassLynx 3.5 with OpenLynx and FractionLynx options. The elution gradient used was 15 min, unless otherwise specified.

The ¹H NMR spectra were recorded on a Bruker AV400 operating at 400 MHz. Standard deuterated solvents were used. Tetramethylsilane may have been used as internal standard. Reactions are routinely monitored by methods well known to those skilled in the art, such as TLC, LCMS and/or HPLC. Such methods are used to assess whether a reaction has gone to completion, and reaction times may be varied accordingly.

Compounds were named using ACD/Name PRO 6.02 chemical naming software Advanced Chemistry Developments Inc.; Toronto, Ontario, M5H2L3, Canada.

### Intermediates

### Intermediate 1

### 1,1-Dimethylethyl (2R)-2-{[4-[(4-chlorophenyl)methyl]-1-oxo-2(1H)-phthalazinyl] methyl}-1-pyrrolidinecarboxylate

To a solution of triphenylphosphine (1.86 g, 7.09 mmol) in dry tetrahydrofuran (6 ml) was added diisopropyl azodicarboxylate (1.12 ml, 5.69 mmol) at -15°C. The resulting pale yellow thick suspension was stirred at -15 °C for 2 min. To aid stirring more dry tetrahydrofuran (2 ml) was added. The reaction mixture was then treated with a suspension of 4-[(4-chlorophenyl)methyl]-1(2*H*)-phthalazinone, (for example, as disclosed in US patent 1,377,231, Example 10, Step 1) (0.571 g, 2.11 mmol) and *N*-*tert*-butoxycarbonyl-*D-*prolinol (commercially available, for example, from Fluka), (0.650 g, 3.23 mmol) in dry tetrahydrofuran (10 ml) at -15°C. The reaction mixture was allowed to warm to room temperature and stirred at 20°C for 23 h. Methanol (20 ml) was then added and the solvents were removed *in vacuo.* The resultant residue was purified by Flashmaster II chromatography (70 g silica cartridge) eluted with 0 - 50% ethyl acetate-cyclohexane gradient over 40 min. The solvents were removed *in vacuo* to afford the *title compound* as a dark brown oil (1.05 g). LCMS RT = 3.71 min.

### Intermediate 2

### 4-[(4-Chlorophenyl)methyl]-2-[(2R)-2-pyrrolidinylmethyl]-1(2H)-phthalazinone

To a solution of 1,1-dimethylethyl (2*R*)-2-{[4-[(4-chlorophenyl)methyl]-1-oxo-2(1H)-phthalazinyl]methyl}-1-pyrrolidinecarboxylate (for example, as prepared for Intermediate 1) (1.05 g, 2.31 mmol) in dry dioxane (12 ml) was added a solution of hydrogen chloride in 1,4-dioxane (4.0 M, 6 ml). The solution was stirred at 20°C for 2 h. Trifluoroacetic acid (1 ml) was added to the mixture and stirred for 30 min, then more trifluoroacetic acid (3 × approximately 1 ml) was added at 10 minute intervals until deprotection was completed. The solvent was removed *in vacuo* and the residue applied onto an SCX cartridge (20 g), washed with methanol (x 2) and then eluted with 10% aqueous ammonia in methanol (2 × 50 ml). The solvents were removed *in vacuo* and the resultant residue purified by Flashmaster II chromatography (50 g silica cartridge) eluted with 0 - 30% methanol + 1% triethylamine - dichloromethane gradient over 40 min to afford the *title compound* as a dark brown foam (0.351 g). LCMS RT = 2.45 min.

### Intermediate 3

### 1,1-Dimethylethyl (2S)-2-{[4-[(4-chlorophenyl)methyl]-1-oxo-2(1H)-phthalazinyl] methyl}-1-pyrrolidinecarboxylate

A solution of di-*tert*-butyl azodicarboxylate (1.15 g, 5 mmol) in tetrahydrofuran (5 ml) was added to a stirred solution of triphenylphosphine (1.8 g, 7 mmol) in tetrahydrofuran (5 ml), cooled to -15°C. The resulting thick suspension was treated with a suspension of 4-[(4-chlorophenyl)methyl]-1(2*H*)-phthalazinone (for example, as disclosed in US patent 1,377,231, see Example 9, Step 1) (0.558 g, 2 mmol) and *N*-*tert*-butoxycarbonyl-*D-*prolinol (commercially available from, for example, Fluka) (0.64 g, 3.2 mmol) in tetrahydrofuran (5 ml), and then further tetrahydrofuran (15 ml) was added to the stirred mixture. Stirring was continued under a nitrogen atmosphere overnight, allowing the temperature to rise to room temperature. The reaction mixture was concentrated *in vacuo,* and the residue was purified by chromatography on silica (Flashmaster II, 100 g, gradient of 0 - 50% ethyl acetate-cyclohexane over 60 min). The appropriate fractions were combined and concentrated *in vacuo* to afford *the title compound* (0.738 g). LCMS RT = 3.71 min, ES+ve *m*/*z* 454/456 [M+H]⁺.

### Intermediate 4

### 4-[(4-Chlorophenyl)methyl]-2-[(2S)-2-pyrrolidinylmethyl]-1(2H)-phthalazinone

A solution of 1,1-dimethylethyl (2*S*)-2-{[4-[(4-chlorophenyl)methyl]-1-oxo-2(1*H*)-phthalazinyl]methyl}-1-pyrrolidinecarboxylate, (for example, as prepared for Intermediate 3) (738 mg, 1.6 mmol) in dioxane (5 ml) was treated with a solution of hydrogen chloride in dioxane (4 M, 5 ml), and stirred at room temperature under a nitrogen atmosphere for 1 h. Further hydrogen chloride in dioxane (4 M, 2 ml) was added, and stirring continued for 40 min more. The reaction mixture was concentrated *in vacuo.* The residue was applied to an SCX catridge (50 g), eluting with methanol, and then a solution of 10% aqueous ammonia in methanol. The appropriate fractions were combined and concentrated *in vacuo* to afford *the title compound* (0.555 g). LCMS RT = 2.45 min, ES+ve *m*/*z* 354/356 [M+H]⁺.

### Intermediate 5

### 2-Bromoethanesulfonyl chloride

Phosphorus pentachloride (commercially available, for example, from Aldrich) (11.8 g, 57 mmol) was added portionwise to stirred sodium bromoethanesulfonate (commercially available, for example, from Aldrich) (4.0 g, 19 mmol) over five minutes. When the addition was complete, the suspension was heated to 110 °C for 2 h before cooling to 21 °C and then pouring onto ice. The product was extracted with dichloromethane and the organic layer was washed successively with water, sodium bicarbonate and water. The organic solution was dried over magnesium sulfate and evaporated to give *the title compound* (3.24 g), ¹H NMR δ (CDCl₃) 4.12 (2H, t, J = 8 Hz), 3.79 (2H, t, J = 8 Hz).

### Intermediate 6

### 1-(Ethenylsulfonyl)piperidine

2-Bromoethanesulfonyl chloride (for example, as prepared for Intermediate 5) (415 mg, 2 mmol) was stirred in dichloromethane (4 ml) with ice-water cooling under nitrogen and triethylamine (0.42 ml, 3 mmol) was added. When the solution had cooled back down to 5 °C, piperidine (237 µl, 2.4 mmol) in dichloromethane (2 ml) was added dropwise over 10 min. The mixture was left to warm to room temperature over 2 h and then stirred for 3 h. The solution was diluted with more dichloromethane and it was washed successively with water and 2N hydrochloric acid, then water (× 2), each time back-extracting with dichloromethane. The combined organic solutions were dried over magnesium sulfate and evaporated to give *the title compound* (99 mg), LCMS RT = 0.89 min, ES+ve *mlz* 176 (M+H)⁺.

### Intermediate 7

### 3-Chloropropyl ethyl sulfone

Sodium ethanethiolate (commercially available, for example, from Aldrich) (2.0 g, 24 mmol) in ethanol (24 ml) was treated with 1-bromo-3-chloropropane (commercially available, for example, from Aldrich) (2.35 ml, 24 mmol) and the mixture was stirred at room temperature for 3 days. The mixture was then diluted with diethyl ether and filtered through fluted filter paper to remove the white precipitate. The filtrate was then concentrated by distillation of the solvents at atmospheric pressure. The solid residue from the filtration was combined with the solid residue from the distillation, and partitioned between water and dichloromethane. The aqueous phase was extracted once more with dichloromethane and the combined organic solutions were dried (magnesium sulfate), filtered, and the filtrate was treated with m-chloroperbenzoic acid (commercially available, for example, from Aldrich) (1 g, 60% pure, 3 mmol) and the mixture was stirred at room temperature overnight. The reaction mixture was diluted with dichloromethane and washed with sodium bicarbonate solution. The organic solution was washed with aqueous sodium metabisulfite solution (x 2), aqueous sodium bicarbonate solution, dried (magnesium sulfate) and evaporated under reduced pressure. The residue (790 mg) was dissolved in dichloromethane and applied to a silica cartridge (20 g) eluting with a gradient of diethyl ether-petroleum ether (40-60 °C) (20% to 60%) to give *the title compound* (260 mg, 6%): ¹H NMR δ (CDCl₃) 3.71 (2H, t, J = 7 Hz), 3.15 (2H, t, J = 7 Hz), 3.04 (2H, q, J = 7 Hz), 2.40-2.30 (2H, m), 1.44 (3H, t, J = 7 Hz).

### Intermediate 8

### Ethyl 3-(ethylthio)butanoate

Ethyl crotonate (commercially available, for example, from Aldrich) (2.37 g, 20.8 mmol) was dissolved in *N*,*N*'-dimethylformamide (60 ml) and stirred at room temperature. Sodium ethanethiolate (commercially available, for example, from Aldrich) (1.66 g, 19.7 mmol) was added portionwise. On completion of the addition the mixture was stirred at room temperature overnight. The mixture was diluted with water and extracted with ethyl acetate (× 3). The combined organic solutions were dried (magnesium sulfate), and concentrated *in vacuo,* for an extensive period of time to remove excess *N*,*N'*-dimethylformamide. The residue was applied to a silica cartridge (50 g), eluting with a gradient of ethyl acetate-cyclohexane (2% to 6%) to give *the title compound* as a colourless oil (527 mg, 14%): ¹H NMR δ (CDCl₃) 4.16 (2H, q, J = 9 Hz), 3.28-3.18 (1H, m), 2.66-2.55 (3H, m), 2.44 (1H, dd, J = 15, 8 Hz), 1.33 (3H, d, J = 7 Hz), 1.31-1.23 (6H, m).

### Intermediate 9

### 3-(Ethylthio)-1-butanol

Ethyl 3-(ethylthio)butanoate (for example, as prepared for Intermediate 8) (526 mg, 2.98 mmol) was dissolved in tetrahydrofuran (9 ml) and added dropwise to a stirred solution of lithium aluminium hydride in ether (1.0 M, 6 ml), cooled in an external ice-water bath, and under a nitrogen atmosphere. The mixture was stirred under nitrogen for 2.5 h, and then quenched by the addition of saturated aqueous sodium sulfate solution. The mixture was filtered, and the filtrate was concentrated *in vacuo* to give *the title compound* as a colourless oil (493 mg, approximately 100%, contained some residual tetrahydrofuran): ¹H NMR δ (CDCl₃) 3.89-3.72 (2H, m), 3.00-2.91 (1H, m), 2.59 (2H, q, J = 7.5 Hz), 1.93-1.77 (2H, m), 1.33 (3H, d, J = 7 Hz), 1.27 (3H, t, J = 7.5 Hz).

### Intermediate 10

### 3-(Ethylthio)butyl methanesulfonate

3-(Ethylthio)-1-butanol (for example, as prepared for Intermediate 9) (247 mg, 1.84 mmol) was dissolved in dichloromethane (10 ml), and the stirred solution was cooled in an external ice-water bath. Methanesulfonyl chloride (commercially available, for example, from Aldrich) (154 µl, 1.99 mmol) was added and stirring was continued under a nitrogen atmosphere for 2.5 h. The reaction mixture was diluted with further dichloromethane (5 ml) and quenched with saturated aqueous sodium hydrogen carbonate. The layers were separated and the aqueous phase was extracted with further dichloromethane (x 2) (hydrophobic frit). The combined organic solutions were concentrated *in vacuo* to give *the title compound* as a colourless oil, which later partially solidified (360 mg, 92%): ¹H NMR δ (CDCl₃) 4.46-4.32 (2H, m), 3.03 (3H, s), 2.98-2.88 (1H, m), 2.57 (2H, q, J = 7.5 Hz), 2.03-1.87 (2H, m), 1.35 (3H, d, J = 7 Hz), 1.26 (3H, t, J = 7 Hz).

### Intermediate 11

### 3-(Ethylsulfonyl)butyl methanesulfonate

3-(Ethylthio)butyl methanesulfonate (for example, as prepared for Intermediate 10) (360 mg, 1.70 mmol) was dissolved in dichloromethane (10 ml) with stirring. The solution was treated with *m*-chloroperbenzoic acid (commercially available, for example, from Aldrich) (57-86%, 0.90 g, at least 3 mmol), and the mixture was stirred at room temperature for 2.5 h. Excess *m*-chloroperbenzoic acid was quenched by the addition of aqueous sodium metabisulfite. Saturated aqueous sodium hydrogen carbonate and further dichloromethane were added. The mixture was shaken, the layers were separated, and the aqueous solution was extracted with further dichloromethane. The combined dichloromethane extracts were washed with further aqueous sodium hydrogen carbonate (× 3), dried (magnesium sulfate), and concentrated *in vacuo* to give *the title compound* as a colourless gum (393 mg, 95%): ¹H NMR δ (CDCl₃) 4.54-4.47 (1H, m), 4.38 (1H, ddd, J = 10, 8, 5 Hz), 3.30-3.20 (1H, m), 3.06 (3H, s), 3.02 (2H, q, J = 7.5 Hz), 2.57-2.47 (1H, m), 2.03-1.92 (1H, m), 1.47-1.40 (6H, m); LCMS RT = 1.64 min, ES+ve *m*/*z* 262 (M+NH₄)⁺

### Intermediate 12

**2-[2-((2*R*)-2-{[4-[(4-Chlorophenyl)methyl]-1-oxo-2(1*H*)-phthalazinyl]methyl}-1-pyrrolidinyl)ethyl]-2*H*-isoindole-1,3(2*H*)-dione** 4-[(4-Chlorophenyl)methyl)-2-[(2*R*)-2-pyrrolidinylmethyl]-1(2*H*)-phthalazinone (for example, as prepared for Intermediate 2) (3.05 g, 8.59 mmol) was stirred with 2-(2-bromoethyl)-1*H-*isoindole-1,3(2*H*)-dione (4.82 g, 19 mmol) (commercially available, for example, from Acros) and potassium carbonate (5.9 g, 43 mmol) in 2-butanone (75 ml) under nitrogen at 80 °C for 18 h. Further 2-(2-bromoethyl)-1*H*-isoindole-1,3(2*H*)-dione (2.4 g, 9.4 mmol) and potassium carbonate (3.0 g, 22 mmol) were added and the heating and stirring were continued for a further day. After three further days at room temperature, the mixture was partitioned between water and dichloromethane. The aqueous layer was extracted with more dichloromethane and the combined organic layers were washed with water, dried (magnesium sulfate), and evaporated. The residual oil was re-dissolved in dichloromethane and loaded onto a column of silica gel (250 g) that had been set up in 40% ethyl acetate in 40-60 °C petroleum ether. The column was eluted with this mixture, then 50%, 70%, 80% and neat ethyl acetate to give *the title compound* (3.96 g, 87%): LCMS RT = 3.19 min, ES+ve *m*/*z* 527/529 [M+H]⁺.

### Intermediate 13

### 2-{[(2R)-1-(2-Aminoethyl)-2-pyrrolidinyl]methyl}-4-[(4-chlorophenyl)methyl]-1(2H)-phthalazinone

2-[2-((2*R*)-2-{[4-[(4-Chlorophenyl)methyl]-1-oxo-2(1*H*)-phthalazinyl]methyl}-1-pyrrolidinyl) ethyl]-1*H*-isoindole-1,3(2*H*)-dione (for example, as prepared for Intermediate 12) (3.96 g, 7.51 mmol) was dissolved in ethanol (50 ml) at 80 °C with stirring and hydrazine monohydrate (commercially available, for example, from Aldrich) (0.91 ml, 19 mmol) was added. The mixture was heated with stirring for 1.25 h. The reaction was cooled with ice-water and the white solid was removed by filtration. The filter-cake was leached with ethanol and the combined filtrates were evaporated to a white solid. This solid was mixed with 2M hydrochloric acid (10 ml) and water (approximately 100 ml). The opaque solution was washed successively with ethyl acetate and dichloromethane. The aqueous layer was then made alkaline with 2M sodium hydroxide solution and the product was extracted with dichloromethane (× 4). The combined organic layers were washed with water and dried over magnesium sulfate and evaporated to give *the title compound* as a white solid, (2.29 g): LCMS RT = 2.74 min, ES+ve *m*/*z* 397/399 [M+H]⁺.

### Intermediate 14

### 1,1-Dimethylethyl [3-((2R)-2-{[4-[(4-chlorophenyl)methyl]-1-oxo-2(1H)-phthalazinyl] methyl}-1-pyrrolidinyl)propyl]carbamate

4-[(4-Chlorophenyl)methyl]-2-[(2*R*)-2-pyrrolidinylmethyl]-1(2*H*)-phthalazinone (for example, as prepared for Intermediate 2) (495 mg, 1.40 mmol) was stirred with 1,1-dimethylethyl (3-bromopropyl)carbamate (commercially available, for example, from Fluka) (404 mg, 1.70 mmol), potassium carbonate (293 mg, 2.12 mmol) and sodium iodide (41 mg, 0.27 mmol) in 2-butanone (20 ml) under nitrogen at 80 °C overnight. The solvent was removed *in vacuo,* and the residue was dissolved in a mixture of dichloromethane and water. The layers were separated, and the dichloromethane solution was passed through a hydrophobic frit, and then concentrated *in vacuo* to give *the title compound* (737 mg, 100%): LCMS RT = 2.74 min, ES+ve *m*/*z* 511/513 [M+H]⁺.

### Intermediate 15

### 2-{[(2R)-1-(3-Aminopropyl)-2-pyrrolidinyl]methyl}-4-[(4-chlorophenyl)methyl]-1(2H)-phthalazi none

1,1-Dimethylethyl [3-((2*R*)-2-{[4-[(4-chlorophenyl)methyl]-1-oxo-2(1*H*)-phthalazinyl]methyl}-1-pyrrolidinyl)propyl]carbamate (for example, as prepared for Intermediate 14) (737 mg, 1.4 mmol) was dissolved in a solution of hydrogen chloride in 1,4-dioxane (4M, 25 ml), and the mixture was stirred at room temperature under nitrogen overnight. The reaction mixture was concentrated *in vacuo,* and the residue was dissolved in acetonitrile and applied to a SCX cartridge (20 g, preconditioned with methanol and then acetonitrile). The cartridge was washed with acetonitrile and then eluted with 10% aqueous 0.880 s.g. ammonia in acetonitrile. The appropriate basic fractions were combined and the solvent removed *in vacuo,* to give *the title compound* (508 mg, 88%): LCMS RT = 2.11 min, ES+ve *m*/*z* 411/413 [M+H]⁺.

### Intermediate 16

### 4-[(Ethylsulfonyl)amino]butyl ethanesulfonate

4-Amino-1-butanol (commercially available, for example, from Aldrich) (0.5 ml, 5.4 mmol) was dissolved in dichloromethane (25 ml) together with triethylamine (4.5 ml, 32 mmol), and the stirred solution was cooled in an external ice-water bath under a nitrogen atmosphere. Ethanesulfonyl chloride (commercially available, for example, from Fluka) (1.5 ml, 16 mmol), dissolved in dichloromethane (15 ml), was added dropwise, using further dichloromethane (10 ml) to wash in. The reaction mixture was stirred under nitrogen and allowed to warm gradually to room temperature over three hours. The mixture was diluted with further dichloromethane (50 ml) and washed with saturated aqueous sodium hydrogen carbonate. The aqueous layer was extracted with further dichloromethane (x 2). The combined organic solutions were dried (magnesium sulfate) and concentrated *in vacuo* to give the crude product (1.70 g). A portion of the crude material was purified by chromatography. The brown oil (792 mg) was applied to a silica cartridge (50 g, Flashmaster 2), eluting with 0-100% ethyl acetate-dichloromethane over 40 min to give the pure *title compound* as a colourless gum (621 mg, ca. 90%): LCMS RT = 2.15 min, ES+ve *m*/*z* 291 (M+NH₄)⁺.

### Intermediate 17

### N-[3-(Methyloxy)propyl]ethenesulfonamide

2-Bromoethanesulfonyl chloride (for example, as prepared for Intermediate 5) (415 mg, 2 mmol) was stirred with 3-methoxypropylamine (0.245 ml, 2.4 mmol) in dichloromethane (5 ml) at room temperature and triethylamine (0.42 ml, 3 mmol) was added. The solution was left to stand at room temperature overnight. It was then diluted with more dichloromethane and it was washed successively with water and 2N hydrochloric acid, then water, each time back extracting with dichloromethane. The combined organic solutions were dried over magnesium sulfate and evaporated to give *the title compound* (225 mg), LCMS RT = 1.64 min, ES+ve *m*/*z* 180 (M+H)⁺.

### Intermediate 18

### 4-[(Methyloxy)carbonyl]-3-pyridinecarboxylic acid

To a suspension of pyridine-3,4-dicarboxylic acid anhydride (commercially available, for example, from Aldrich) (26.73 g, 180 mmol) in dry tetrahydrofuran (250 ml) at -70 °C under nitrogen was added a suspension of sodium methoxide (11.2 g, 2.01 mol) in dry methanol (50 ml). The reaction mixture was allowed to warm to room temperature and stirred for 18 h. The solvents were removed *in vacuo* and the residue was dissolved in water (350 ml). This was acidified to approximately pH 2 using concentrated hydrochloric acid. The resultant solid was collected by filtration and washed with water. The solid was dried *in vacuo* at 45 °C to give *the title compound* (14.6 g, 45%) as a white solid. LCMS RT = 0.98 min, ES+ve *m*/*z* 182 (M+H)⁺.

### Intermediate 19

### Methyl 3-{2-(4-chlorophenyl)-3-[(1,1-dimethylethyl)oxy]-3-oxopropanoyl}-4-pyridine carboxylate

To a solution of 4-[(methyloxy)carbonyl]-3-pyridinecarboxylic acid (for example, as prepared for Intermediate 18) (1.81 g, 10 mmol) in dry *N*.*N*'-dimethylformamide (90 ml) under nitrogen was added carbonyl diimidazole (1.7 g, 10.5 mmol). The reaction mixture was heated at 50 °C for 90 min and then cooled to -5 °C in a salt/ice bath. To this was added 1,1-dimethylethyl 4-chlorophenylacetate (for example, as prepared for Intermediate 23) (2.38 g, 10.5 mmol), followed by the portionwise addition of sodium hydride (1.4 g of 60% dispersion in mineral oil, 35 mmol) over 15 min. The reaction mixture was stirred at -5 °C for 10 min and then warmed to room temperature. After 2 h, the reaction mixture was poured into a saturated solution of ammonium chloride (100 ml). This was extracted using ethyl acetate (3 × 100 ml). The combined organics were washed with water (2 × 100 ml) and brine (2 × 100 ml). The organic phase was dried (MgSO₄) and the solvent removed *in vacuo.* The residue was dissolved in dichloromethane (5 ml and applied to a silica cartridge (100 g). This was eluted using a gradient of 0-50% ethyl acetate in cyclohexane over 60 min. The required fractions were evaporated *in vacuo* to give the *title compound* (2.94 g, 75%, mixture of ketone and enol purity 99%) as a pale brown oil. LCMS RT = 3.41 and 3.63 (U-shaped peak) min ES+ve *m*/*z* 390/392 (M+H)⁺.

### Intermediate 20

### Methyl 3-[(4-chlorophenyl)acetyl]-4-pyridinecarboxylate

Methyl 3-{2-(4-chlorophenyl)-3-[(1,1-dimethylethyl)oxy]-3-oxopropanoyl}-4-pyridine carboxylate (for example, as prepared for Intermediate 19) (2.94 g, 7.5 mmol) was dissolved in dry dichloromethane (12 ml) and to this was added trifluoroacetic acid (5 ml). The reaction mixture was stirred at room temperature under nitrogen for 20 h. The solvent was removed *in vacuo* and the residue was dissolved in dichloromethane (5 ml). This was applied to a silica cartridge (100 g) and eluted with a gradient of 0-100% ethyl acetate in cyclohexane over 60 min. The required fractions were combined and evaporated *in vacuo* to give *the title compound* (1.59 g, 73%) as a pale orange oil. LCMS RT = 3.02 min ES+ve *m*/*z* 290/292 (M+H)⁺.

### Intermediate 21

### 4-[(4-Chlorophenyl)methyl]pyrido[3,4-d]pyridazin-1(2H)-one

Methyl 3-[(4-chlorophenyl)acetyl]-4-pyridinecarboxylate (for example, as prepared for Intermediate 20) (1.59 g, 5.5 mmol) was dissolved in ethanol (60 ml) and to this was added hydrazine hydrate (commercially available, for example, from Aldrich) (0.3 ml, 6 mmol) and a few drops of acetic acid. The reaction mixture was heated at reflux for 3 h. The reaction mixture was allowed to cool and the solid was collected by filtration and washed with ethanol (10 ml). The solid was dried *in vacuo* to give *the title compound* (1.17 g, 78%) as a white solid. LCMS RT = 2.73 min, ES+ve *m*/*z* 272/274 (M+H)⁺.

### Intermediate 22

### 4-[(4-Chlorophenyl)methyl]-2-[(2R)-2-pyrrolidinylmethyl]pyrido[3,4-d]pyridazin-1(2H)-one

To a solution of triphenylphosphine (10.42 g, 40 mmol) in anhydrous tetrahydrofuran (80 ml) at -10 °C was added a solution of di-*tert*-butyl azodicarboxylate (8.38 g, 36 mmol) (commercially available, for example, from Aldrich) in anhydrous tetrahydrofuran (60 ml). The solution was allowed to warm to 15 °C and then cooled to 0-5 °C. To the slight suspension was added a suspension of 4-[(4-chlorophenyl)methyl]pyrido[3,4-d]pyridazin-1(2*H*)-one (for example, as prepared for Intermediate 21) and *N*-Boc-D-prolinol (commercially available, for example, from Aldrich) (5.14 g, 25.6 mmol) in anhydrous tetrahydrofuran (100 ml). The suspension was allowed to warm to ambient temperature and stirred for 23 h. The solvent was removed *in vacuo* to leave an oil (30 g). LCMS RT = 3.48 min, ES+ve *m*/*z* 455/457. To a solution of the crude product (30 g) in 1,4-dioxane (80 ml) was added 4.0 M hydrogen chloride in 1,4-dioxane (80 ml, 320 mmol). The solution was stirred at ambient temperature for 5 h. The solvent was removed *in vacuo* and the residue was partitioned between 1 M aqueous hydrochloric acid (400 ml) and ethyl acetate (200 ml). The phases were separated and the aqueous phase washed with ethyl acetate (200 ml). The combined organic extracts were washed with 1 M aqueous hydrochloric acid (200 ml). The combined aqueous extracts were basified to pH 10 using 2 M aqueous sodium hydroxide (300-350 ml) and the resulting suspension extracted with ethyl acetate (2 × 400 ml, 1 × 200 ml). The combined organic extracts were concentrated *in vacuo* to leave the *title compound* (8.0 g). LCMS RT = 2.15 min, ES+ve *m*/*z* 355/357 (M+H)⁺.

### Intermediate 23

### 1,1-Dimethylethyl (4-chlorophenyl)acetate

(4-Chlorophenyl)acetic acid (commercially available, for example, from Aldrich) (13.76 g, 81 mmol) was suspended in toluene (100 ml) under nitrogen. To this was added di-*tert*-butyl dimethylacetal (commercially available, for example, from Aldrich) (50 ml) and the reaction mixture was heated at 80 °C for 18 h. The solvent was removed *in vacuo* and the residue was dissolved in ethyl acetate (200 ml). The solution was washed with saturated sodium bicarbonate solution (2 × 200 ml) and brine (2 × 200 ml). The organic phase was dried (MgSO₄) and evaporated *in vacuo* to give *the title compound* (6.68 g, 36%) as pale brown oil. ¹H NMR (CDCl₃) δ 7.28 (2H, d, J 8.5 Hz), 7.19 (2H, d, J 8.5 Hz), 3.48 (2H, s), 1.43 (9H, s).

### Intermediate 24

### 3-Chloropropyl ethyl sulfone

Sodium ethanethiolate (commercially available, for example, from Aldrich) (2.0 g, 24 mmol) in ethanol (24 ml) was treated with 1-bromo-3-chloropropane (commercially available, for example, from Aldrich) (2.35 ml, 24 mmol) and the mixture was stirred at room temperature for 3 days. The mixture was then diluted with diethyl ether and filtered to remove the white precipitate. The filtrate was then concentrated by distillation of the solvents at atmospheric pressure. The solid residue from the filtration was combined with the solid residue from the distillation, and partitioned between water and dichloromethane. The aqueous phase was extracted with dichloromethane and the combined organic solutions were dried (MgSO₄), filtered, and the filtrate was treated with m-chloroperbenzoic acid (commercially available, for example, from Aldrich) (1 g, 57-86% pure, at least 3 mmol) and the mixture was stirred at room temperature overnight. The reaction mixture was diluted with dichloromethane and washed with sodium bicarbonate solution. The organic solution was washed with aqueous sodium metabisulfite solution (x 2), aqueous sodium bicarbonate solution, dried (MgSO₄) and evaporated under reduced pressure. The residue (790 mg) was dissolved in dichloromethane and applied to a silica cartridge (20 g) eluting with a gradient of diethylether-petroleum ether (40 - 60 °C) (20% - 60%) to give *the title compound* (260 mg, 6%): ¹H NMR δ (CDCl₃) 3.71 (2H, t, J = 7 Hz), 3.15 (2H, t, J = 7 Hz), 3.04 (2H, q, J = 7 Hz), 2.40-2.30 (2H, m), 1.44 (3H, t, J = 7 Hz).

### Intermediate 25

### 2-[2-((2R)-2-{[4-[(4-Chlorophenyl)methyl]-1-oxopyrido[3,4-d]pyridazin-2(1H)-yl]methyl}-1-pyrrolidinyl)ethyl]-1H-isoindole-1,3(2H)-dione

4-[(4-Chlorophenyl)methyl]-2-[(2*R*)-2-pyrrolidinylmethyl]pyrido[3,4*-d*]pyridazin-1(2*H*)-one (for example, as prepared for Intermediate 22) (840 mg, 2.37 mmol) was stirred with 2-(2-bromoethyl)-1*H*-isoindole-1,3(2*H*)-dione (commercially available, for example, from Aldrich) (1.51 g, 5.93 mmol) and potassium carbonate (1.64 g, 11.9 mmol) in 2-butanone (25 ml) under nitrogen at 80 °C overnight. Further potassium carbonate (0.17 g, 1.2 mmol) was added and the heating and stirring were continued for a further 4 h. The butanone was removed *in vacuo.* The residue was partitioned between water and dichloromethane. The aqueous layer was extracted twice with further dichloromethane, and the combined organic layers were washed with water, dried (MgSO₄) and concentrated *in vacuo.* Purification on silica (100 g), eluting with 0-100% ethyl acetate-cyclohexane over 60 min, gave the *tittle compound* as a pale yellow foam (996 mg, 80%). LCMS RT = 2.57 min, ES+ve *mlz* 528/530 [M+H]⁺.

### Intermediate 26

### 2-{[(2R)-1-(2-Aminoethyl)-2-pyrrolidinyl]methyl}-4-[(4-chlorophenyl)methyl] pyrido[3,4-d]pyridazin-1 (2H)-one

2-[2-((2*R*)-2-{[4-[(4-Chlorophenyl)methyl]-1-oxopyrido[3,4*-d*]pyridazin-2(1*H*)-yl]methyl}-1-pyrrolidinyl)ethyl]-1*H*-isoindole-1,3(2*H*)-dione (for example, as prepared for Intermediate 25) (996 mg, 1.89 mmol) was dissolved in ethanol (20 ml) and hydrazine monohydrate (0.23 ml, 4.73 mmol) (commercially available, for example, from Aldrich) was added. The mixture was heated at 80 °C with stirring for 4 h. The reaction was allowed to cool and the solid was removed by filtration, washing with excess ethanol. The combined filtrate and washings were concentrated *in vacuo* to give the *title compound* as a yellow gum (636 mg, 85%) LCMS RT = 2.44 min, ES+ve *m*/*z* 398/400 [M+H]⁺.

### Examples

### Example 1

### 4-[(4-Chlorophenyl)methyl]-2-({(2R)-1-[2-(ethylsulfonyl)ethyl]-2-pyrrolidinyl}methyl)-1(2H)-phthalazinone, formate salt

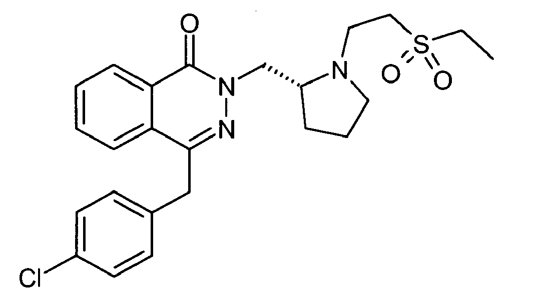

4-[(4-Chlorophenyl)methyl]-2-[(2*R*)-2-pyrrolidinylmethyl]-1(2*H*)-phthalazinone (for example, as prepared for Intermediate 4) (38 mg, 0.10 mmol) in acetonitrile (1 ml) was treated with ethyl vinyl sulfone (commercially available, for example, from Aldrich) (0.5 ml, 4.8 mmol) and the mixture was heated in a SmithCreator^{™} microwave oven at 100 °C for 15 min. The progress of the reaction was followed by LCMS. Only partial reaction occurred and the mixture was heated for a further 10 min, however the reaction did not progress further. More ethyl vinyl sulfone (0.4 ml, 3.8 mmol) was added and the mixture heated for a further 10 min. The reaction did not progress any further and the mixture was poured on a 10 g SCX-2 cartridge that had been preconditioned with methanol. The cartridge was washed through with methanol and then the product was eluted with 10% aqueous 0.880 s.g. ammonia solution in methanol to give the crude product (42 mg). This was dissolved in methanol-DMSO (3:1, 0.8 ml) and purified twice by MDAP HPLC. Evaporation of the appropriate combined fractions gave *the title compound* (4.1 mg), LCMS RT = 2.67 min, ES+ve *m*/*z* 474/476 (M+H)⁺.

### Example 2

**4-[(4-Chlorophenyl)methyl]-2-({(2*R*)-1-[2-(1-piperidinylsulfonyl)ethyl]-2-pyrrolidinyl}methyl)-1(2*H*)-phthalazinone, formate salt** 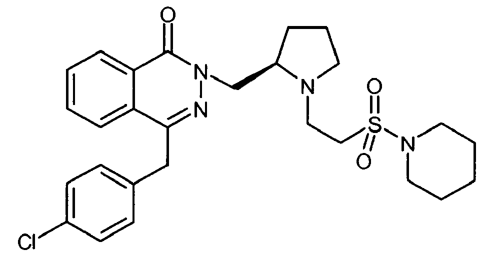 1-(Ethenylsulfonyl)piperidine (for example, as prepared for Intermediate 6) (45 mg, 0.26 mmol) was heated with 4-[(4-chlorophenyl)methyl]-2-[(2*R*)-2-pyrrolidinylmethyl]-1(2*H*)-phthalazinone (for example, as prepared for Intermediate 2) (90 mg, 0.26 mmol) in *N*,*N*'-dimethylformamide(1 ml) at 90 °C under nitrogen for 30 hours. During this time the reaction was followed by LCMS. Only partial reaction occurred, which did not progress further. After standing at room temperature, the solution was diluted with methanol and poured onto a SCX-2 cartridge that had been preconditioned with methanol. The cartridge was washed through with methanol and then the product was eluted with 10% aqueous 0.880 s.g. ammonia solution in methanol to give the crude product (95 mg). This was dissolved in 1:1 methanol-DMSO (2 ml) and each 1 ml portion was loaded onto a MDAP HPLC (2 runs) using a 25 min gradient. Evaporation of the appropriate combined fractions gave *the title compound* (26 mg), LCMS RT = 2.77 min, ES+ve *mlz* 529/531 (M+H)⁺.

### Example 3

### 4-[(4-Chlorophenyl)methyl]-2-({(2R)-1-[3-(ethylsulfonyl)propyl]-2-pyrrolidinyl}methyl)-1(2H)-phthalazinone, trifluoroacetate salt

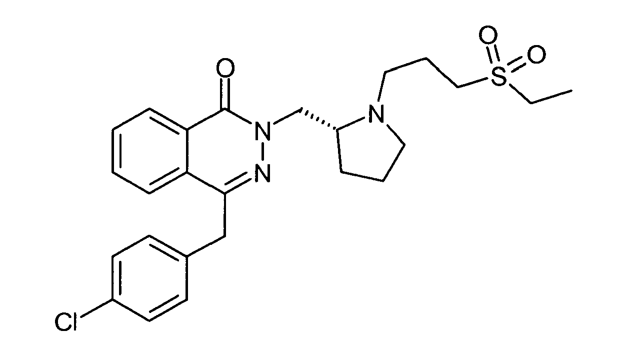

4-[(4-Chlorophenyl)methyl]-2-[(2*R*)-2-pyrrolidinylmethyl]-1(2*H*)-phthalazinone (for example, as prepared for Intermediate 4) (38 mg, 0.10 mmol), 3-chloropropyl ethyl sulfone (for example, as prepared for Intermediate 7) (58 mg, 0.3 mmol), sodium bicarbonate (64 mg, 0.76 mmol) and sodium iodide (15 mg, 0.10 mmol) in *N*,*N*'-dimethylformamide (1.4 ml) was heated in a SmithCreator^{™} microwave oven at 150 °C for 15 min. The mixture was partitioned between ethyl acetate and water, and the organic phase was washed with aqueous sodium bicarbonate, brine and dried (magnesium sulfate). The solution was filtered and the filtrate evaporated under reduced pressure. The residue (41 mg) was dissolved in methanol-DMSO (1:1, 0.8 ml) and purified by MDAP HPLC. Appropriate fractions were combined, acidified with excess trifluoroacetic acid and evaporated under reduced pressure to give *the title compound* (31 mg), LCMS RT = 2.52 min, ES+ve *mlz* 488/490 (M+H)⁺.

### Example 4

### 4-[(4-Chlorophenyl)methyl]-2-({(2R)-1-[3-(ethylsulfonyl)butyl]-2-pyrrolidinyl}methyl)-1(2H)-phthalazinone, hydrochloride salt

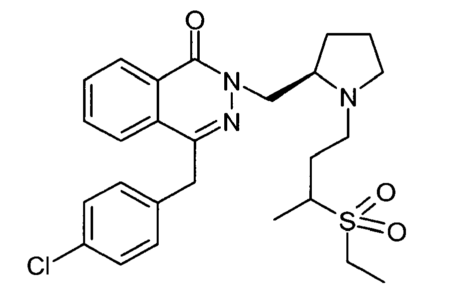

4-[(4-Chlorophenyl)methyl]-2-[(2*R*)-2-pyrrolidinylmethyl]-1(2*H*)-phthalazinone (for example, as prepared for Intermediate 2) (118 mg, 0.33 mmol) was mixed with 3-(ethylsulfonyl)butyl methanesulfonate (for example, as prepared for Intermediate 11) (156 mg, 0.64 mmol), sodium hydrogen carbonate (177 mg, 2.1 mmol) and sodium iodide (94 mg, 0.63 mmol) in *N*,*N*'-dimethylformamide (3 ml). The resulting suspension was heated to 150 °C for 30 min in a Smith Creator^{™} microwave oven. The mixture was diluted with methanol and applied to an SCX-2 cartridge (50 g) (preconditioned with methanol), eluting with methanol, followed by 10% aqueous 0.88 s.g. ammonia in methanol. The relevant basic fractions were concentrated, and the residue was purified by MDAP HPLC. The appropriate pure fractions were combined and concentrated to give the free base of the title compound as a mixture of diastereomers (60 mg); LCMS RT = 2.82 min, ES+ve *m*/*z* 502/504 [M+H]⁺. This sample was divided, and approximately one-third of the material, dissolved in methanol, was treated with a solution of hydrogen chloride in methanol (1.25M, 0.5 ml, excess). This mixture was concentrated under a flow of nitrogen to give *the title compound* as the hydrochloride salt and as a mixture of diastereomers (21 mg, 12%); LCMS RT = 2.88 min, ES+ve *m*/*z* 502/504 [M+H]⁺.

Some less pure fractions resulting from the MDAP HPLC purification were concentrated, and the residue was repurified by further MDAP HPLC. Further material was thus obtained as the formate salt of the title compound, as a mixture of diastereomers. This was combined with the residual free base material, and the total was applied to an SCX-2 cartridge (2 g) (preconditioned with methanol), eluting with methanol, followed by 10% aqueous 0.88 s.g. ammonia in methanol. The relevant basic fractions were concentrated to give the free base of *the title compound* as a mixture of diastereomers (50 mg, 30%); LCMS RT = 2.92 min, ES+ve *m*/*z* 502/504 [M+H]⁺.

### Examples 5A and 5B

**4-[(4-Chlorophenyl)methyl]-2-({(2*R*)-1-[(3*S*)-3-(ethylsulfonyl)butyl]-2-pyrrolidinyl} methyl)-(2*H*)-phthalazinone, hydrochloride salt**
**and**
**4-[(4-Chlorophenyl)methyl]-2-({(2*R*)-1-[(3*R*)-3-(ethylsulfonyl)butyl]-2-pyrrolidinyl} methyl)-(2*H*)-phthalazinone, hydrochloride salt** 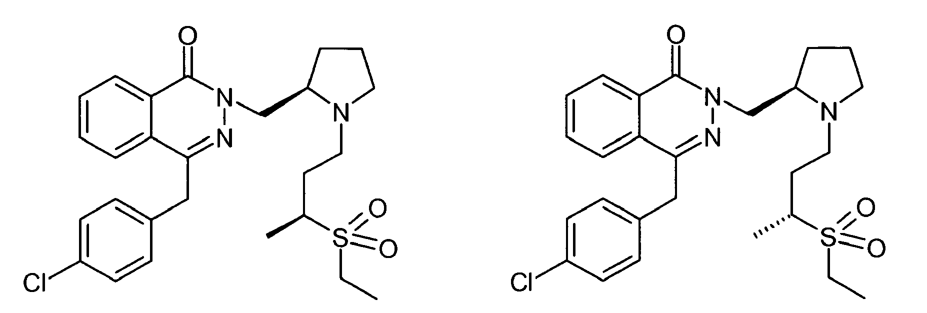 4-[(4-Chlorophenyl)methyl]-2-({(2*R*)-1-[3-(ethylsulfonyl)butyl]-2-pyrrolidinyl}methyl)-1(2*H*)-phthalazinone as the free base and a mixture of diastereomers (for example, as prepared for Example 4) was further purified using a 25 cm × 2 cm Chiralcel OJ column using 50% ethanol/heptane at 15ml/min detecting at 215 nm. The two diastereomers were obtained as separate pure compounds.

Analytical chiral HPLC was conducted on a 25 cm × 0.46 cm Chiralcel OJ column using 50% ethanol/heptane at 1ml/min, detecting at 215 nm.
Isomer 1: chiral HPLC RT 9.17 min; LCMS RT = 2.56 min, ES+ve *m*/*z* 502/504 [M+H]⁺.
Isomer 2: chiral HPLC RT 14.73 min; LCMS RT = 2.58 min, ES+ve *m*/*z* 502/504 [M+H]⁺.

### Example 6

### N-[2-((2R)-2{[4-[(4-Chlorophenyl)methyl]-1-oxo-2(1H)-phthalazinyl]methyl}-1-pyrrolidinyl)ethyl]ethanesulfonamide, triflouroacetate salt

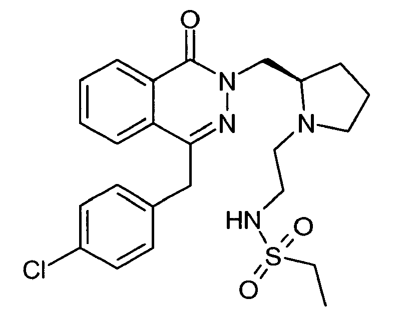

2-{[(2*R*)-1-(2-Aminoethyl)-2-pyrrolidinyl]methyl}-4-[(4-chlorophenyl)methyl]-1(2*H*)-phthalazinone (for example, as prepared for Intermediate 13) (40 mg, 0.10 mmol) was stirred with ethanesulfonyl chloride (commercially available, for example, from Aldrich) (11.5 µl, 0.12 mmol) and triethylamine (21 µl, 0.16 mmol) in dichloromethane (3 ml) at room temperature for 30 min and then left to stand overnight. Saturated aqueous sodium hydrogen carbonate solution was added to the mixture and the layers were separated. The aqueous layer was extracted with further dichloromethane (× 2). The combined organic extracts were concentrated. The residue was purified by MDAP HPLC and the appropriate fractions combined. The solvent was removed *in vacuo* and the residue re-dissolved in methanol and treated with excess trifluroacetic acid to convert the product to the trifluoroacetate salt. The solvent and excess acid were removed using a stream of nitrogen to give *the title compound* (50 mg, 83%). LCMS RT = 2.49 min, ES+ve *m*/*z* 489/491 (M+H)⁺.

### Example 7

### N-[2-((2R)-2-{[4-[(4-Chlorophenyl)methyl]-1-oxo-2(1H)-phthalazinyl]methyl}-1-pyrrolidinyl)ethyl]-1-propanesulfonamide, hydrochloride salt

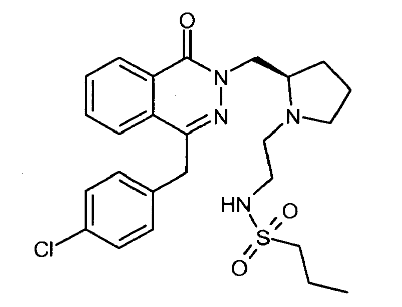

2-{[(2*R*)-1-(2-Aminoethyl)-2-pyrrolidinyl]methyl}-4-[(4-chlorophenyl)methyl]-1(2*H*)-phthalazinone (for example, as prepared for Intermediate 13) (30 mg, 0.076 mmol) was stirred with 1-propane sulfonyl chloride (commercially available, for example, from Aldrich) (11 µl, 0.091 mmol) in dichloromethane (3 ml) containing triethylamine (20 µl, 0.15 mmol) at room temperature for 30 min, when LCMS showed reaction was complete. The solution was washed with aqueous sodium bicarbonate solution. The phases were separated in a cartridge and the aqueous layer was extracted with more dichloromethane. The combined organic layers were evaporated to dryness to give the crude product (52 mg). This was purified by MDAP HPLC to give the formate salt of *the title compound* (34 mg). This was dissolved in methanol (5 ml) and 1.25M hydrogen chloride in methanol was added. The mixture was evaporated to dryness to give *title compound* (37.8 mg), LCMS RT = 2.62 min, ES+ve *m*/*z* 503/505 (M+H)⁺.

### Example 8

### N-[2-((2R)-2-{[4-[(4-Chlorophenyl)methyl]-1-oxo-2(1H)-phthalazinyl]methyl}-1-pyrrolidinyl)ethyl]-2-propanesulfonamide, trifluoroacetate salt

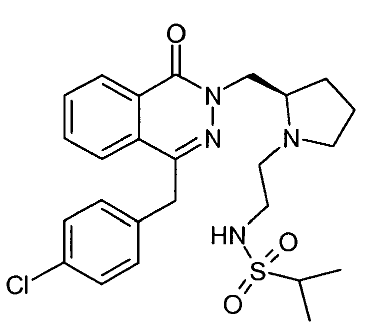

2-{[(2*R*)-1-(2-Aminoethyl)-2-pyrrolidinyl]methyl}-4-[(4-chlorophenyl)methyl]-1(2*H*)-phthalazinone (for example, as prepared for Intermediate 13) (40 mg, 0.10 mmol) was stirred with 2-propanesulfonyl chloride (commercially available, for example, from Aldrich) (13.5 µl, 0.12 mmol) and triethylamine (21 µl, 0.16 mmol) in dichloromethane (3 ml) at room temperature for 2 h. LCMS analysis indicated incomplete reaction. Further 2-propanesulfonyl chloride (13.5 µl, 0.12 mmol) and triethylamine (21 µl, 0.16 mmol) were added and stirring was continued at room temperature for 1.5 h. LCMS analysis still indicated incomplete reaction. Further 2-propanesulfonyl chloride (68 µl, 0.6 mmol) and triethylamine (105 µl, 0.8 mmol) were added and stirring was continued at room temperature overnight. Saturated aqueous sodium hydrogen carbonate solution was added to the mixture and the layers were separated. The aqueous layer was extracted with further dichloromethane (x 2). The combined organic extracts were concentrated. The residue was purified by MDAP HPLC and the appropriate fractions combined. The solvent was removed *in vacuo* and the residue re-dissolved in methanol and treated with excess trifluoracetic acid to convert the product to the trifluoroacetate salt. The solvent and excess acid were removed using a stream of nitrogen to give *the title compound* (5 mg, 8%). LCMS RT = 2.58 min, ES+ve *m*/*z* 503/505 (M+H)⁺.

### Example 9

### N-[4-((2R)-2-{[4-[(4-Chlorophenyl)methyl]-1-oxo-2(1H)-phthalazinyl]methyl}-1-pyrrolidinyl)butyl]ethanesulfonamide, hydrochloride salt

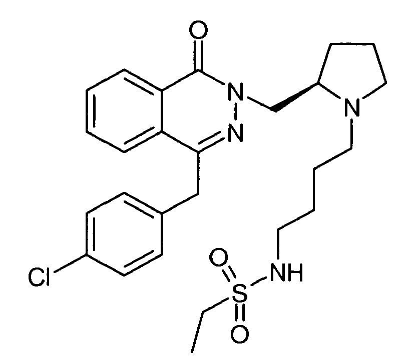

4-[(4-Chlorophenyl)methyl]-2-[(2*R*)-2-pyrrolidinylmethyl]-1(2*H*)-phthalazinone (for example, as prepared for Intermediate 2) (56 mg, 0.16 mmol) was stirred with 4-[(ethylsulfonyl)amino]butyl ethanesulfonate (for example, as prepared for Intermediate 16) (65 mg, 0.24 mmol), sodium hydrogen carbonate (96 mg, 1.1 mmol) and sodium iodide (24 mg, 0.16 mmol) in *N*,*N*'-dimethylformamide (6 ml) under nitrogen at 60 °C for 6 h. Further 4-[(ethylsulfonyl)amino]butyl ethanesulfonate (68 mg, 0.25 mmol) was added in N,N'-dimethylformamide(1 ml), and stirring at 60 °C was continued overnight. LCMS analysis indicated that reaction was still incomplete, so further 4-[(ethylsulfonyl)amino]butyl ethanesulfonate (63 mg, 0.23 mmol) was added in *N*,*N*'-dimethylformamide (1 ml), and stirring at 60 °C was continued for a second night. The reaction mixture was applied to a SCX-2 cartridge (20 g, preconditioned with methanol). The cartridge was washed with methanol and then eluted with 10% aqueous 0.880 s.g. ammonia in methanol. The appropriate basic fractions were combined and the solvent removed *in vacuo.* The residue was purified by chromatography using a silica cartridge (5 g), eluting with dichloromethane-ethanol-aqueous triethylamine (200:8:1). Appropriate fractions were combined and concentrated *in vacuo* to give the free base of the *title compound.* This material was dissolved in methanol and treated with a solution of hydrogen chloride in methanol (1.25M, 0.5 ml). The mixture was concentrated under a flow of nitrogen to give *the title compound* as a colourless gum (6 mg, 7%); LCMS RT = 2.90 min, ES+ve *m*/*z* 517/519 [M+H]⁺.

### Example 10

### 2-((2R)-2-{[4-[(4-Chlorophenyl)methyl]-1-oxo-2(1H)-phthalazinyl]methyl}-1-pyrrolidinyl)-N-[3-(methyloxy)propyl]ethanesulfonamide

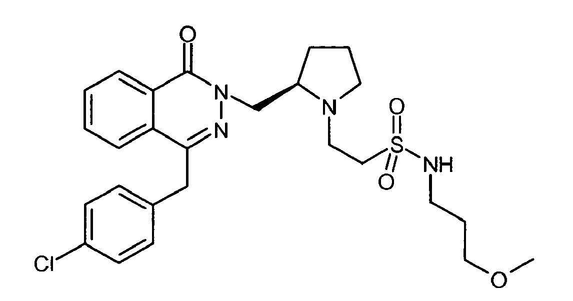

4-[(4-Chlorophenyl)methyl]-2-[(2*R*)-2-pyrrolidinylmethyl]-1(2*H*)-phthalazinone (for example, as prepared for Intermediate 2) (50 mg, 0.14 mmol) was heated at 80 °C to 90 °C under nitrogen with *N*-[3-methyloxy)propyl]ethenesulfonamide (for example, as prepared for Intermediate 17) (38 mg, 0.21 mmol) in isopropanol (2 ml) for four days. LCMS indicated reaction had only gone to a small extent. The isopropanol was evaporated and the residue was dissolved in *N,N*'-dimethylformamide and heating was continued at 100 °C for two more days. Reaction was incomplete so acetic acid (8.6 µl, 0.15 mmol) was added to try to suppress sulphonamide deprotonation. However, after two further days at 100 °C little further reaction had occurred so the solution was mixed with a little methanol and poured onto a 10 g SCX-2 cartridge that had been preconditioned with methanol. The cartridge was washed through with methanol and then the product was eluted with 10% aqueous 0.880 s.g. ammonia solution in methanol to give the crude product mixture (46 mg). This was dissolved in methanol and loaded onto a 20 × 20 cm Whatman 1 mm preparative plate which was run in 100:8:1, dichloromethane-ethanol-0.880 s.g. aqueous ammonia solution. Elution of the top strong UV band with 1:1 methanol-dichloromethane and evaporation gave a mixture containing the *title compound* (15 mg). This was purified further using a MDAP HPLC to give *the title compound* (6.8 mg), LCMS RT = 2.65 min, ES+ve *m*/*z* 533/535 (M+H)⁺.

### Example 11

### N-[2-((2R)-2-{[4-[(4-Chlorophenyl)methyl]-1-oxo-2(1H)-phthalazinyl]methyl}-1-pyrrolidinyl)ethyl]-N'-propylurea, trifluoroacetate salt

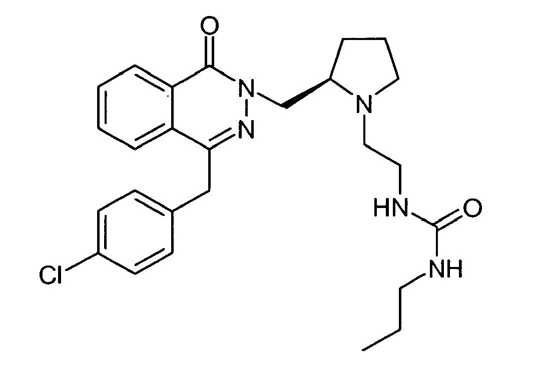

2-{[(2*R*)-1-(2-Aminoethyl)-2-pyrrolidinyl]methyl}-4-[(4-chlorophenyl)methyl]-1(2*H*)-phthalazinone (for example, as prepared for Intermediate 13) (34 mg, 0.09 mmol) in dichloromethane (2 ml) was treated with n-propylisocyanate (commercially available, for example, from Aldrich) (20 µl, 0.21 mmol), and the mixture was left to stand overnight. The mixture was applied to an SCX cartridge (10 g) (preconditioned with methanol), eluting with methanol, followed by 10% aqueous 0.88 s.g. ammonia in methanol. The relevant basic fraction was concentrated, and the residue was purified by MDAP HPLC. The appropriate fractions were combined, treated with trifluoroacetic acid (0.5 ml) and concentrated *in vacuo* to give *the title compound* (48 mg, 94%). LCMS RT = 2.59 min, ES+ve *m*/*z* 482/484 (M+H)⁺.

### Example 12

### 4-[(4-Chlorophenyl)methyl]-2-({(2R)-1-[3-(ethylsulfonyl)propyl]-2-pyrrolidinyl} methyl)pyrido[3,4-d]pyridazin-1(2H)-one, trifluoroacetate salt

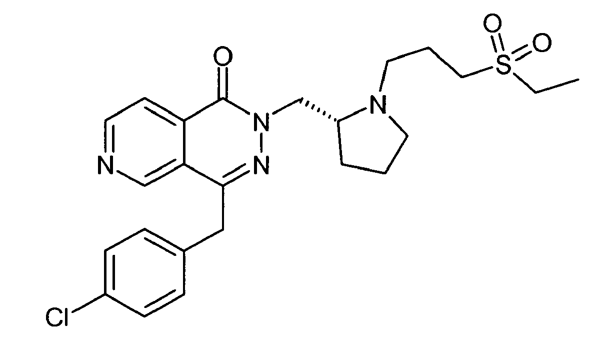

A mixture of 3-chloropropyl ethyl sulfone (for example, as prepared for Intermediate 24) (84 mg, 0.49 mmol), 4-[(4-chlorophenyl)methyl]-2-[(2*R*)-2-pyrrolidinylmethyl]pyrido[3,4-*d*]pyridazin-1(2*H*)-one (for example, as prepared for Intermediate 22) (35 mg, 0.1 mmol) sodium bicarbonate (64 mg, 0.76 mmol) sodium iodide (17 mg, 0.1 mmol) in *N*,*N*'-dimethylformamide (1.4 ml) was sealed in a microwave vial and heated to 150 °C for 15 min in a SmithCreator^{™} microwave oven. The mixture was diluted with methanol and applied to an SCX-2 cartridge (10 g, pre-conditioned with methanol) eluting with methanol, followed by 10% aqueous 0.88 s.g. ammonia in methanol. The ammoniacal fractions were evaporated *in vacuo* and the residue (52 mg) was dissolved in methanol-dimethylsulfoxide (3:1, 0.8 ml) and purified by MDAP HPLC. Appropriate fractions were combined and evaporated *in vacuo.* The residue was dissolved in methanol and treated with trifluoroacetic acid (50 µl) and re-evaporated *in vacuo* to give the product contaminated with dimethylsulfoxide (64 mg). This was dissolved in methanol and applied to another SCX-2 cartridge (5 g) eluting with with methanol, followed by 10% aqueous 0.88 s.g. ammonia in methanol. The ammoniacal fractions were evaporated *in vacuo* and the residue was dissolved in methanol, treated with trifluoroacetic acid (50 µl) and re-evaporated *in vacuo* to give *the title compound* (36 mg) LCMS (Method B, shorter run time) RT = 0.86 min, ES+ve *m*/*z* 489/491 (M+H)⁺.

### Example 13

### 4-[(4-Chlorophenyl)methyl]-2-({(2R)-1-[2-(ethylsulfonyl)ethyl]-2-pyrrolidinyl}methyl) pyrido[3,4-d]pyridazin-1(2H)-one, trifluoroacetate salt

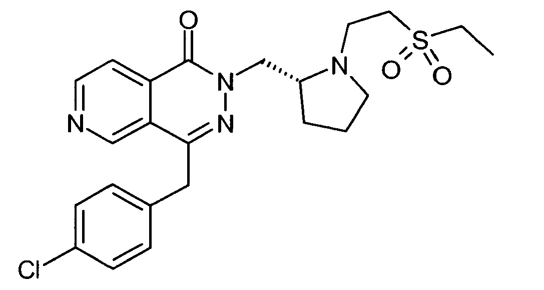

A mixture of ethyl vinyl sulfone (commercially available, for example, from Aldrich) (0.6 ml, 5.7 mmol), 4-[(4-chlorophenyl)methyl]-2-[(2*R*)-2-pyrrolidinylmethyl]pyrido[3,4*-d*]pyridazin-1(2*H*)-one (for example, as prepared for Intermediate 22) (40 mg, 0.11 mmol), sodium bicarbonate (60 mg, 0.7 mmol) in acetonitrile (1 ml) was sealed in a microwave vial and heated to 100 °C for 15 min in a SmithCreator^{™} microwave oven. The mixture was diluted with methanol and applied to a pre-conditioned SCX-2 cartridge (10 g) eluting with methanol, followed by 10% aqueous 0.88 s.g. ammonia in methanol. The ammoniacal fractions were evaporated under reduced pressure and the residue (43 mg) was dissolved in methanol-dimethylsulfoxide (1:3, 0.8 ml) and purified by MDAP HPLC. Appropriate fractions were combined and evaporated under reduced pressure. The residue was dissolved in methanol and treated with trifluoroacetic acid (50 µl) and re-evaporated under reduced pressure to give *the title compound* (50 mg); LCMS (Method B, shorter run time) RT = 0.92 min, ES+ve *m*/*z* 475/477 (M+H)⁺.

### Example 14

### N-[2-((2R)-2-{[4-[(4-chlorophenyl)methyl]-1-oxopyrido[3,4-d]pyridazin-2(1H)-yl]methyl}-1-pyrrolidinyl)ethyl]-N'-propylurea

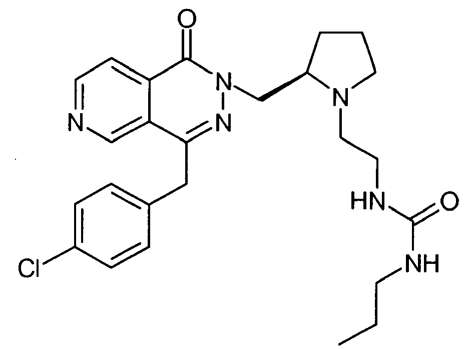

A suspension of 2-{[(2*R*)-1-(2-aminoethyl)-2-pyrrolidinyl]methyl}-4-[(4-chlorophenyl)methyl]pyrido[3,4*-d*]pyridazin-1(2*H*)-one (for example, as prepared for Inermediate 26) (38 mg, 0.096 mmol) in dichloromethane (2 ml) was treated with n-propyl isocyanate (commercially available, for example, from Aldrich) (0.02 ml, 0.21 mmol) and stirred overnight. The mixture was diluted with methanol and applied to an SCX-2 cartridge (10 g, pre-conditioned with methanol) eluting with methanol, followed by 10% aqueous 0.88 s.g. ammonia in methanol. The basic fraction was concentrated in vacuo and the residue was treated with dimethylsulfoxide-methanol to disslove for MDAP HPLC purification.The product crystallised so the solid was dissolved in warm methanol and applied to another SCX-2 and process repeated as above. Evaporation of the basic fraction gave the product as a yellow solid (38 mg) which was recrystallised from isopropanol to give *the title compound* LCMS RT = 2.39 min, ES+ve m/z 483/485 (M+H)⁺.

### Biological Assays

The compounds of the invention may be tested for *in vitro* and/or *in vivo* biological activity in accordance with the following or similar assays.

### H1 receptor cell line generation and FLIPR assay protocol

### 1. Generation of histamine H1 cell line

The human H1 receptor is cloned using known procedures described in the literature [Biochem. Biophys. Res. Commun., 201(2):894 (1994)]. Chinese hamster ovary (CHO) cells stably expressing the human H1 receptor are generated according to known procedures described in the literature [Br. J. Pharmacol., 117(6):1071 (1996)].

### Histamine H1 functional antagonist assay: Determination of functional pKi values

The histamine H1 cell line is seeded into non-coated black-walled clear bottom 384-well tissue culture plates in alpha minimum essential medium (Gibco/Invitrogen, cat no. 22561-021), supplemented with 10% dialysed foetal calf serum (Gibco/Invitrogen cat no. 12480-021) and 2 mM L-glutamine (Gibco/Invitrogen cat no 25030-024) and is maintained overnight at 5% CO₂, 37 °C.

Excess medium is removed from each well to leave 10 µl. 30 µl loading dye (250 µM Brilliant Black, 2 µM Fluo-4 diluted in Tyrodes buffer + probenecid (145 mM NaCl, 2.5 mM KCI, 10 mM HEPES, 10 mM D-glucose, 1.2 mM MgCl₂, 1.5 mM CaCl₂, 2.5 mM probenecid, pH adjusted to 7.40 with NaOH 1.0 M)) is added to each well and the plates are incubated for 60 min at 5% CO₂, 37 °C.

10 µl of test compound, diluted to the required concentration in Tyrodes buffer + probenecid (or 10 µl Tyrodes buffer + probenecid as a control) is added to each well and the plate is incubated for 30 min at 37 °C, 5% CO₂. The plates are then placed into a FLIPR™ (Molecular Devices, UK) to monitor cell fluorescence (λₑₓ = 488 nm, λ_{EM} = 540 nm) in the manner described in Sullivan et al., (In: Lambert DG (ed.), Calcium Signaling Protocols, New Jersey: Humana Press, 1999, 125-136) before and after the addition of 10 µl histamine at a concentration that results in the final assay concentration of histamine being EC₈₀.

Functional antagonism is indicated by a suppression of histamine induced increase in fluorescence, as measured by the FLIPR™ system (Molecular Devices). By means of concentration effect curves, functional affinities are determined using standard pharmacological mathematical analysis.

### Histamine H1 functional antagonist assay: Determination of antagonist pA2 and duration

The histamine H1 receptor expressing CHO cells are seeded into non-coated black-walled clear bottom 96-well tissue culture plates as described above.

Following overnight culture, growth medium is removed from each well, washed with 200 µl PBS and is replaced with 50 µl loading dye (250 µM Brilliant Black, 1 µM Fluo-4 diluted in Tyrodes buffer + probenecid (145 mM NaCl, 2.5 mM KCl, 10mM HEPES, 10mM D-glucose, 1.2 mM MgCl₂, 1.5 mM CaCl₂, 2.5 mM probenecid, pH adjusted to 7.40 with NaOH 1.0 M)). Cells are incubated for 45 min at 37 °C. The loading buffer is removed and the cells are washed as above, and 90 µl of Tyrodes buffer + probenecid is added to each well. 10 µl of test compound, diluted to the required concentration in Tyrodes buffer + probenecid (or 10 µl Tyrodes buffer + probenecid as a control) is added to each well and the plate is incubated for 30 min at 37 °C, 5% CO₂.

The plates are then placed into a FLIPR™ (Molecular Devices, UK) to monitor cell fluorescence (λₑₓ = 488 nm, λ_{EM} = 540 nm) in the manner described in Sullivan et al., (In: Lambert DG (ed.), Calcium Signaling Protocols, New Jersey: Humana Press, 1999, 125-136) before and after the addition of 50 µl histamine over a concentration range of 1 mM - 0.1 nM. The resultant concentration response curves are analysed by non-linear regression using a standard four parameter logistic equation to determine the histamine EC₅₀, the concentration of histamine required to produce a response of 50% of the maximum response to histamine. The antagonist pA2 is calculated using the following standard equation: pA2 = log(DR-1)-log[B] where DR = dose ratio, defined as EC₅₀antagonist-treated/EC₅₀control and [B] = concentration of antagonist.

To determine the antagonist duration, cells are cultured overnight in non-coated black-walled clear bottom 96-well tissue culture plates, are washed with PBS and are incubated with a concentration of antagonist chosen to give an approximate DR in the range 30 - 300. Following the 30 min antagonist incubation period, the cells are washed two or three times with 200 µl of PBS and then 100 µl Tyrodes buffer is added to each well to initiate antagonist dissociation. Following incubation for predetermined times, typically 30 - 270 min at 37 °C, the cells are then washed again with 200 µl PBS and are incubated with 100 µl Tyrodes buffer containing Brilliant Black, probenecid and Fluo-4 for 45 min at 37 °C, as described above. After this period, the cells are challenged with histamine in the FLIPR™ as described above. The dose ratio at each time point is used to determine the fractional H1 receptor occupancy by the following equation: fractional receptor occupancy = (DR-1)/DR. The decrease in receptor occupancy over time approximates to a straight line and is analysed by linear regression. The slope of this straight line fit is used as an index of the dissociation rate of the antagonist. The dose ratios for antagonist treated cells and for antagonist treated and washed cells at each time point are used to calculate a relative dose ratio (rel DR) which is also used as an index of antagonist duration. Antagonists with long duration of action produce rel DR values close to 1, and antagonists with short duration of action produce rel DR values that approaches the dose ratio value obtained for antagonist treatment alone.

### 2. H3 receptor cell line generation, membrane preparation and functional GTPγS assay protocols

### Generation of histamine H3 cell line

The histamine H3 cDNA is isolated from its holding vector, pCDNA3.1 TOPO (InVitrogen), by restriction digestion of plasmid DNA with the enzymes BamH1 and Not-1 and is ligated into the inducible expression vector pGene (InVitrogen) digested with the same enzymes. The GeneSwitch™ system (a system where in transgene expression is switched off in the absence of an inducer and switched on in the presence of an inducer) is performed as described in US Patents: 5,364,791; 5,874,534; and 5,935,934. Ligated DNA is transformed into competent DH5α *E. coli* host bacterial cells and is plated onto Luria Broth (LB) agar containing Zeocin™ (an antibiotic which allows the selection of cells expressing the *sh ble* gene which is present on pGene and pSwitch) at 50 µgml⁻¹. Colonies containing the re-ligated plasmid are identified by restriction analysis. DNA for transfection into mammalian cells is prepared from 250 ml cultures of the host bacterium containing the pGeneH3 plasmid and is isolated using a DNA preparation kit (Qiagen Midi-Prep) as per manufacturers guidelines (Qiagen).

CHO K1 cells previously transfected with the pSwitch regulatory plasmid (InVitrogen) are seeded at 2×10⁶ cells per T75 flask in Complete Medium, containing Hams F12 (GIBCOBRL, Life Technologies) medium supplemented with 10% v/v dialysed foetal bovine serum, L-glutamine, and hygromycin (100 µgml⁻¹), 24 h prior to use. Plasmid DNA is transfected into the cells using Lipofectamine plus according to the manufacturer's guidelines (InVitrogen). 48 h post transfection, cells are placed into complete medium supplemented with 500 µgml⁻¹ Zeocin™.

10-14 days post selection, 10 nM Mifepristone (InVitrogen) is added to the culture medium to induce the expression of the receptor. 18 h post induction, cells are detached from the flask using ethylenediamine tetra-acetic acid (EDTA; 1:5000; InVitrogen), following several washes with PBS, pH 7.4 and are resuspended in Sorting Medium containing Minimum Essential Medium (MEM), without phenol red, and are supplemented with Earles salts and 3% Foetal Clone II (Hyclone). Approximately 1×10⁷ cells are examined for receptor expression by staining with a rabbit polyclonal antibody, 4a, raised against the *N*-terminal domain of the histamine H3 receptor, are incubated on ice for 60 min, followed by two washes in sorting medium. Receptor bound antibody is detected by incubation of the cells for 60 min on ice with a goat anti rabbit antibody, conjugated with Alexa 488 fluorescence marker (Molecular Probes). Following two further washes with Sorting Medium, cells are filtered through a 50 µm Filco™ (BD Biosciences) and then are analysed on a FACS Vantage SE Flow Cytometer fitted with an Automatic Cell Deposition Unit. Control cells are non-induced cells treated in an analogous manner. Positively stained cells are sorted as single cells into 96-well plates, containing Complete Medium containing 500 µgml⁻¹ Zeocin™ and are allowed to expand before reanalysis for receptor expression via antibody and ligand binding studies. One clone, 3H3, is selected for membrane preparation.

### Membrane preparation from cultured cells

All steps of the protocol are carried out at 4 °C and with pre-cooled reagents. The cell pellet is resuspended in 10 volumes of homogenisation buffer (50 mM *N*-2-hydroxyethylpiperazine-*N*'-2-ethanesulfonic acid (HEPES), 1 mM ethylenediamine tetra-acetic acid (EDTA), pH 7.4 with KOH, supplemented with 10⁻⁶ M leupeptin (acetyl-leucyl-leucyl-arginal; Sigma L2884), 25 µgml⁻¹ bacitracin (Sigma B0125), 1 mM phenylmethylsulfonyl fluoride (PMSF) and 2×10⁻⁶ M pepstain A (Sigma)). The cells are then homogenised by 2 × 15 second bursts in a 1 litre glass Waring blender, followed by centrifugation at 500 g for 20 min. The supernatant is then spun at 48,000 g for 30 min. The pellet is resuspended in homogenisation buffer (4x the volume of the original cell pellet) by vortexing for 5 sec, followed by homogenisation in a Dounce homogeniser (10-15 strokes). At this point the preparation is aliquoted into polypropylene tubes and stored at -80 °C.

### Histamine H3 functional antagonist assay

For each compound being assayed, in a solid white 384 well plate, is added:
(a) 0.5 µl of test compound diluted to the required concentration in DMSO (or 0.5 µl DMSO as a control);
(b) 30 µl bead/membrane/GDP mix which is prepared by mixing Wheat Germ Agglutinin Polystyrene LeadSeeker® (WGA PS LS) scintillation proximity assay (SPA) beads with membrane (prepared in accordance with the methodology described above) and diluting in assay buffer (20 mM *N*-2-hydroxyethylpiperazine-*N*'-2-ethanesulfonic acid (HEPES) + 100 mM NaCl + 10 mM MgCl₂, pH 7.4 NaOH) to give a final volume of 30 µl which contains 5 µg protein, 0.25 mg bead per well and 10 µM final assay concentration of guanosine 5' diphosphate (GDP) (Sigma, diluted in assay buffer) incubating at room temperature for 60 min on a roller;
(c) 15 µl 0.38 nM [³⁵S]-GTPγS (Amersham; Radioactivity concentration = 37 MBqml⁻¹; Specific activity = 1160 Cimmol⁻¹), histamine (at a concentration that results in the final assay concentration of histamine being EC₈₀).

After 2-6 h, the plate is centrifuged for 5 min at 1500 rpm and counted on a Viewlux counter using a 613/55 filter for 5 minplate⁻¹. Data is analysed using a 4-parameter logistic equation. Basal activity is used as minimum, i.e. histamine not added to well.

### Results

In these or similar biological assays, the following data were obtained:
(i) The compounds of Examples 2, 7, 8 and 10 had an average pKᵢ (pK_{b}) at H1 of greater than 7. The remaining compounds of the Examples had an average pKᵢ (pK_{b}) at H1 greater than 8.
   The compounds of Examples 5A, 5B, 10 and 13 had an average pA2 value of greater than approximately 8. The compounds of Examples 1, 3, 4, 6, 7, 8, 11, 12 and 14 had average pA2 values of greater than approximately 9.
(ii) The compounds of the Examples had an average pKᵢ (pK_{b}) at H3 of less than 6.5.
(iii) The compounds of Examples 3, 6, 7, 8, 10, 11 and 12 exhibited at one or more time points a longer duration of action than azelastine in the histamine H1 functional antagonist assay. Other compounds were either not tested or were tested and did not exhibit a longer duration of action.

## Claims

1. A compound of formula (I) 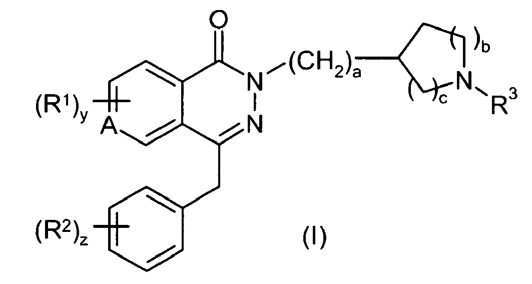 wherein
A represents CH or N;
R¹ and R² each independently represent halogen, C₁₋₆alkyl, C₁₋₆alkoxy, hydroxyl or trifluoromethyl;
y and z each independently represent 0, 1 or 2;
a represents 0 or 1;
b represents 0, 1 or 2 and c represents 0, 1, 2 or 3, such that b and c cannot both be 0;
R³ represents -C₁₋₆alkylene-R⁴-R⁵, in which the alkylene is straight chain and is optionally substituted by one C₁₋₃alkyl, or R³ represents a saturated 5 to 7 membered ring containing one SO₂;
R⁴ represents -SO₂-, -N(R⁶)SO₂-, -SO₂N(R⁷)- or -N(R⁸)C(O)N(R⁹)-;
R⁵ represents -C₁₋₆alkyl (optionally substituted by one, two or three halogen or by one or two C₁₋₆alkoxy, in which the C₁₋₆alkoxy may be optionally substituted by one, two or three halogen), -C₅₋₇cycloalkyl (optionally substituted by one or two C₁₋₃alkyl), -C₁₋₃alkyleneC₅-₇cycloalkyl (in which the C₅₋₇cycloalkyl is optionally substituted by one or two C₁₋₃alkyl), -aryl (optionally substituted by one or two substituents independently selected from halogen, C₁₋₃alkyl, trifluoromethyl, or cyano), or -C₁₋₃alkylenearyl (optionally substituted on aryl by one or two substituents independently selected from halogen, C₁₋₃alkyl, trifluoromethyl, or cyano); R⁶, R⁷, R⁸ and R⁹ each independently represent hydrogen or C₁₋₆alkyl;
or together R⁷ and R⁵ together with the N atom to which they are attached represent a 5 to 7 membered saturated heterocyclic ring optionally containing one further heteroatom independently selected from O and S;
or a salt thereof.

2. A compound according to claim 1 in which A represents CH.

3. A compound according to claim 1 or claim 2 in which R² represents halogen, C₁₋₃alkyl, C₁₋₃alkoxy, hydroxyl or trifluoromethyl.

4. A compound according to any of claims 1 to 3 in which R² represents halogen.

5. A compound according to any of claims 1 to 4 in which a represents 0, b represents 2 and c represents 1.

6. A compound according to any of claims 1 to 5 in which a represents 1, b represents 0 and c represents 2.

7. A compound according to any of claims 1 to in which R⁵ represents -C₁₋₆alkyl (optionally substituted by one or two C₁₋₆alkoxy), -C₅₋₇cycloalkyl (optionally substituted by one or two C₁₋₃alkyl), -C₁₋₃alkyleneC₅₋₇cycloalkyl (in which the C₅₋₇cycloalkyl is optionally substitued by one or two C₁₋₃alkyl).

8. A compound according to any of claims 1 to 7 in which R⁶, R⁷, R⁸ and R⁹ each independently represent hydrogen or C₁₋₃alkyl or together R⁷ and R⁵ together with the N atom to which they are attached represent a 5 to 7 membered saturated heterocyclic ring.

9. A compound according to claim 1 which is:
4-[(4-Chlorophenyl)methyl]-2-({(2*R*)-1-[2-(ethylsulfonyl)ethyl]-2-pyrrolidinyl}methyl)-1-(2*H*)-phthalazinone;
4-[(4-Chlorophenyl)methyl]-2-({(2*R*)-1-[2-(1-piperidinylsulfonyl)ethyl]-2-pyrrolidinyl}methyl)-1(2*H*)-phthalazinone;
4-[(4-Chlorophenyl)methyl]-2-({(2*R*)-1-[3-(ethylsulfonyl)propyl]-2-pyrrolidinyl}methyl)-1(2*H*)-phthalazinone;
4-[(4-Chlorophenyl)methyl]-2-({(2*R*)-1-[3-(ethylsulfonyl)butyl]-2-pyrrolidinyl}methyl)-1-(2*H*)-phthalazinone;
4-[(4-Chlorophenyl)methyl]-2-({(2*R*)-1-[(3S)-3-(ethylsulfonyl)butyl]-2-pyrrolidinyl}methyl)-(2*H*)-phthalazinone;
4-[(4-Chlorophenyl)methyl]-2-({(2*R*)-1-[(3*R*)-3-(ethylsulfonyl)butyl]-2-pyrrolidinyl}methyl)-(2*H*)-phthalazinone;
*N*-[2-((2*R*)-2-{[4-[(4-Chlorophenyl)methyl]-1-oxo-2(1*H*)-phthalazinyl]methyl}-1-pyrrolidinyl) ethyl]ethanesulfonamide;
*N*-[2-((2*R*)-2-{[4-[(4-Chlorophenyl)methyl]-1-oxo-2(1*H*)-phthalazinyl]methyl}-1-pyrrolidinyl) ethyl]-1-propanesulfonamide;
*N*-[2-((2*R*)-2-{[4-[(4-Chlorophenyl)methyl]-1-oxo-2(1*H*)-phthalazinyl]methyl}-1-pyrrolidinyl) ethyl]-2-propanesulfonamide;
*N*-[4-((2*R*)-2-{[4-[(4-Chlorophenyl)methyl]-1-oxo-2(1*H*)-phthalazinyl]methyl}-1-pyrrolidinyl) butyl]ethanesulfonamide;
2-((2*R*)-2-{[4-[(4-Chlorophenyl)methyl]-1-oxo-2(1*H*)-phthalazinyl]methyl}-1-pyrrolidinyl)-*N*-[3-(methyloxy)propyl]ethanesulfonamide;
*N*-[2-((2*R*)-2-{[4-[(4-Chlorophenyl)methyl]-1-oxo-2(1*H*)-phthalazinyl]methyl}-1-pyrrolidinyl) ethyl]-*N*'-propylurea;
4-[(4-Chlorophenyl)methyl]-2-({(2*R*)-1-[3-(ethylsulfonyl)propyl]-2-pyrrolidinyl}methyl)pyrido[3,
4*-d*]pyridazin-1(2*H*)-one;
4-[(4-Chlorophenyl)methyl]-2-({(2*R*)-1-[2-(ethylsulfonyl)ethyl]-2-pyrrolidinyl}methyl)pyrido[3,4 *-d*]pyridazin-1(2*H*)-one;
*N*-[2-((2*R*)-2-{[4-[(4-chlorophenyl)methyl]-1-oxopyrido[3,4*-d*]pyridazin-2(1*H*)-yl]methyl}-1-pyrrolidinyl)ethyl]-*N*'-propylurea;
or a salt thereof.

10. A compound according to any one of claims 1 to 9, or a pharmaceutically acceptable salt thereof.

11. A compound as defined in any one of claims 1 to 9, or a pharmaceutically acceptable salt thereof for use in therapy.

12. A compound according to claim 11 for use in the treatment of inflammatory and/or allergic diseases.

13. A compound according to claim for use in the treatment of allergic rhinitis.

14. A composition which comprises a compound as defined in any one of claims 1 to 9, or a pharmaceutically acceptable salt thereof and one or more pharmaceutically acceptable carriers and/or excipients.

15. A composition according to claim 14 further comprising one or more other therapeutic agents.

## Patentansprüche

1. Verbindung der Formel (I), 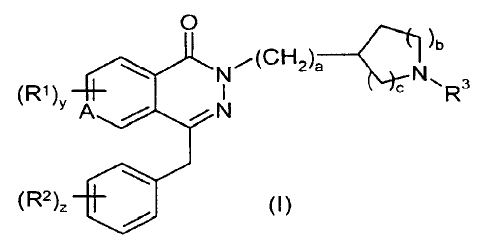 worin
A CH oder n darstellt;
R¹ und R² jeweils unabhängig voneinander Halogen, C₁₋₆-Alkyl, C₁₋₆-Alkoxy, Hydroxyl oder Trifluormethyl darstellen;
y und z jeweils unabhängig voneinander 0, 1 oder 2 darstellen;
a 0 oder 1 darstellt;
b 0, 1 oder 2 darstellt und
c 0, 1, 2 oder 3 darstellt, so dass B und C nicht beide 0 sein können;
R³ -C₁₋₆-Alkylen-R⁴-R⁵ darstellt, worin das Alkylen geradkettig ist und wahlweise durch ein -C₁₋₃-Alkyl substituiert ist, oder R³ einen gesättigten 5- bis 7-gliedrigen Ring darstellt, der ein SO₂ enthält;
R⁴-SO₂-, -N(R⁶)SO₂-, -SP₂(N(R⁷) - oder -N(R⁸)C(O)N(R⁹)-darstellt;
R⁵-C₁₋₆-Alkyl (wahlweise mit einem, zwei oder drei Halogenen oder durch ein oder zwei C₁₋₆-Alkoxy substituiert, worin das C₁₋₆-Alkoxy wahlweise durch ein, zwei oder drei Halogene substituiert sein kann), -C₅₋₇-Cycloalkyl (wahlweise substituiert durch ein oder zwei C₁₋₃-Alkyle), -C₁₋₃-Alkylen-C₅₋₇-cycloalkyl (worin das C₅₋₇-Cycloalkyl wahlweise durch ein oder zwei C₁₋₃-Alkyle substituiert ist), -Aryl (wahlweise substituiert durch ein oder zwei Substituenten, die unabhängig voneinander aus Halogen, C₁₋₃-Alkyl, Trifluormethyl oder Cyano ausgewählt werden) oder -C₁₋₃-Alkylenaryl (wahlweise substituiert am Aryl durch ein oder zwei Substituenten, die unabhängig voneinander aus Halogen, C₁₋₃-Alkyl, Trifluormethyl oder Cyano ausgewählt werden) darstellt;
R⁶, R⁷, R⁸ und R⁹ jeweils unabhängig voneinander Wasserstoff oder C₁₋₆-Alkyl darstellen;
oder R⁷ und R⁵ zusammen, gemeinsam mit dem N-Atom, an das sie angebracht sind, einen 5- bis 7-gliedrigen gesättigten heterocyclischen Ring darstellen, der wahlweise ein weiteres Heteroatom enthält, das unabhängig aus O und S ausgewählt wird;
oder ein Salz davon.

2. Verbindung gemäß Anspruch 1, worin A CH darstellt.

3. Verbindung gemäß Anspruch 1 oder Anspruch 2, worin R² Halogen, C₁₋₃-Alkyl, C₁₋₃-Alkoxy, Hydroxyl oder Trifluormethyl darstellt.

4. Verbindung gemäß irgendeinem der Ansprüche 1 bis 3, worin R² Halogen darstellt.

5. Verbindung gemäß irgendeinem der Ansprüche 1 bis 4, worin a 0 darstellt, b 2 darstellt und c 1 darstellt.

6. Verbindung gemäß irgendeinem der Ansprüche 1 bis 5, worin a 1 darstellt, b 0 darstellt und c 2 darstellt.

7. Verbindung gemäß irgendeinem der Ansprüche 1 bis 6, worin R⁵ C₁₋₆-Alkyl (wahlweise substituiert durch ein oder zwei C₁₋₆-Alkoxy), -C₅₋₇-Cycloalkyl (wahlweise substituiert durch ein oder zwei C₁₋₃-Alkyle), -C₁₋₃-Alkylen-C₅₋₇-cycloalkyl (worin das C₅₋₇-Cycloalkyl wahlweise durch ein oder zwei C₁₋₃-Alkyle substituiert ist) darstellt.

8. Verbindung gemäß irgendeinem der Ansprüche 1 bis 7, worin R⁶, R⁷, R⁸ und R⁹ jeweils unabhängig voneinander Wasserstoff oder C₁₋₃-Alkyl darstellen, oder R⁷ und R⁵ zusammen, gemeinsam mit dem N-Atom, an das sie angebracht sind, einen 5- bis 7-gliedrigen gesättigten heterocyclischen Ring darstellen.

9. Verbindung gemäß Anspruch 1, welche ist:
4-[(4-Chlorphenyl)methyl]-2-({(2R)-1-[2-(ethylsulfonyl)ethyl]-2-pyrrolidinyl}methyl)-1(2H)-phthalazinon;
4-[(4-Chlorphenyl)methyl]-2-({(2R)-1-[2-(1-pidperidinylsulfonyl)ethyl]-2-pyrrolidinyl}methyl)-1(2H)-phthalazinon;
4-[(4-Chlorphenyl)methyl]-2-({(2R)-1-[3-(ethylsulfonyl)propyl]-2-pyrrolidinyl}methyl)-1(2H)-phthalazinon;
4-[(4-Chlorphenyl)methyl]-2-({(2R)-1-[3-(ethylsulfonyl)butyl]-2-pyrrolidinyl}methyl)-1(2H)-phthalazinon;
4-[(4-Chlorphenyl)methyl]-2-({(2R)-1-[(3S)-3-(ethylsulfonyl)butyl]-2-pyrrolidinyl}methyl)-(2H)-phthalazinon;
4-[(4-Chlorphenyl)methyl]-2-({(2R)-1-[(3R)-3-(ethylsulfonyl)butyl]-2-pyrrolidinyl}methyl)-(2H)-phthalazinon;
N-[2-((2R)-2-{(4[(4-Chlorphenyl)methyl]-1-oxo-2(1H)-phthalazinyl]methyl}-1-pyrrolidinyl)ethyl]ethansulfonamid;
N-[2-((2R)-2-{(4[(4-Chlorphenyl)methyl]-1-oxo-2(1H)-phthalazinyl]methyl}-1-pyrrolidinyl)ethyl]-1-propansulfonamid;
N-[2-((2R)-2-{(4[(4-Chlorphenyl)methyl]-1-oxo-2(1H)-phthalazinyl]methyl}-1-pyrrolidinyl)ethyl]-2-propansulfonamid;
N-[4-((2R)-2-{(4[(4-Chlorphenyl)methyl]-1-oxo-2(1H)-phthalazinyl]methyl}-1-pyrrolidinyl)butyl]ethansulfonamid;
2-((2R)-2-{(4[(4-Chlorphenyl)methyl]-1-oxo-2(1H)-phthalazinyl]methyl}-1-pyrrolidinyl)-N-[3-(methyloxy)propyl]ethansulfonamid;
N-[2-((2R)-2-{(4[(4-Chlorphenyl)methyl]-1-oxo-2(1H)-phthalazinyl]methyl}-1-pyrrolidinyl)ethyl]-N'-propylharnstoff;
4-[(4-Chlorphenyl)methyl]-2-({(2R)-1-[3-(ethylsulfonyl)propyl]-2-pyrrolidinyl}methyl)pyrido[3,4-d]pyridazin-1(2H)-on;
4-[(4-Chlorphenyl)methyl]-2-({(2R)-1-[2-(ethylsulfonyl)ethyl]-2-pyrrolidinyl}methyl)pyrido[3,4-d]pyridazin-1(2H)-on;
N-[2-((2R)-2-{[4-[(4-Chlorphenyl)methyl]-1-oxopyrido[3,4-d]pyridazin-2-(1H-yl]methyl}-1-pyrrolidinyl)ethyl]N'-propylharnstoff;
oder ein Salz davon.

10. Verbindung gemäß irgendeinem der Ansprüche 1 bis 9 oder ein pharmazeutisch akzeptables Salz davon.

11. Verbindung, wie sie in irgendeinem der Ansprüche 1 bis 9 definiert wurde, oder ein pharmazeutisch akzeptables Salz davon für die Verwendung bei der Therapie.

12. Verbindung gemäß Anspruch 11 für die Verwendung bei der Behandlung von entzündlichen und/oder allergischen Erkrankungen.

13. Verbindung gemäß Anspruch 12 für die Verwendung bei der Behandlung von allergischer Rhinitis.

14. Zusammensetzung, die eine Verbindung, wie sie in irgendeinem der Ansprüche 1 bis 9 definiert wurde, oder ein pharmazeutisch akzeptables Salz davon und einen oder mehrere pharmazeutisch akzeptable Träger und/oder Hilfsstoffe umfasst.

15. Zusammensetzung gemäß Anspruch 14, die weiterhin ein oder mehrere andere therapeutische Wirkstoffe umfasst.

## Revendications

1. Composé de formule (I) 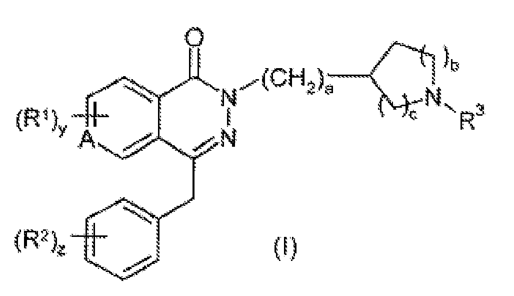 dans laquelle
A représente CH ou N ;
R¹ et R² représentent chacun indépendamment un atome d'halogène, un groupe alkyle en C₁ à C₆, alcoxy en C₁ à C₆, hydroxyle ou trifluorométhyle ;
y et z représentent chacun indépendamment 0, 1 ou 2 ;
a représente 0 ou 1 ;
b représente 0, 1 ou 2 et c représente 0, 1, 2 ou 3, de telle manière que b et c ne puissent pas valoir tous les deux 0 ;
R³ représente un groupe -alkylène en C₁ à C₆-R⁴-R⁵, où le radical alkylène est une chaîne linéaire et est éventuellement substitué par un groupe alkyle en C₁ à C₃, ou R³ représente un cycle saturé de 5 à 7 chaînons contenant un groupe SO₂ ;
R⁴ représente un groupe -SO₂-, -N(R⁶)SO₂-, -SO₂N(R⁷)- ou -N(R⁸)C(O)N(R⁹)- ;
R⁵ représente un groupe alkyle en C₁ à C₆ (éventuellement substitué par un, deux ou trois atomes d'halogène ou par un ou deux groupes alcoxy en C₁ à C₆, où le groupe alcoxy en C₁ à C₆ peut être éventuellement substitué par un, deux ou trois atomes d'halogène), -cycloalkyle en C₅ à C₇ (éventuellement substitué par un ou deux groupes alkyle en C₁ à C₃), -alkylène en C₁ à C₃-cycloalkyle en C₅ à C₇ (où le radical cycloalkyle en C₅ à C₇ est éventuellement substitué par un ou deux groupes alkyle en C₁ à C₃), -aryle (éventuellement substitués par un ou deux substituants choisis indépendamment parmi un atome d'halogène, un groupe alkyle en C₁ à C₃, trifluorométhyle ou cyano) ou -alkylène en C₁ à C₃-aryle (éventuellement substitué sur le radical aryle par un ou deux substituants choisis indépendamment parmi un atome d'halogène, un groupe alkyle en C₁ à C₃, trifluorométhyle ou cyano) ;
R⁶, R⁷, R⁸ et R⁹ représentent chacun indépendamment un atome d'hydrogène ou un groupe alkyle en C₁ à C₆;
ou ensemble R⁷ et R⁵ conjointement avec l'atome N auquel ils sont fixés, représentent un cycle hétérocyclique saturé de 5 à 7 chaînons contenant éventuellement un autre hétéroatome choisi indépendamment parmi 0 et S ;
ou un sel de celui-ci ;

2. Composé selon la revendication 1, dans lequel A représente CH.

3. Composé selon la revendication 1 ou la revendication 2, dans lequel R² représente un atome d'halogène, un groupe alkyle en C₁ à C₃, alcoxy en C₁ à C₃, hydroxyle ou trifluorométhyle.

4. Composé selon l'une quelconque des revendications 1 à 3, dans lequel R² représente un atome d'halogène.

5. Composé selon l'une quelconque des revendications 1 à 4, dans lequel a représente 0, b représente 2 et c représente 1.

6. Composé selon l'une quelconque des revendications 1 à 5, dans lequel a représente 1, b représente 0 et c représente 2.

7. Composé selon l'une quelconque des revendications 1 à 6, dans lequel R⁵ représente un groupe -alkyle en C₁ à C₆ (éventuellement substitué par un ou deux groupes alcoxy en C₁ à C₆), -cycloalkyle en C₅ à C₇ (éventuellement substitué par un ou deux groupes alkyle en C₁ à C₃), -alkylène en C₁ à C₃-cycloalkyle en C₅ à C₇ (où le radical cycloalkyle en C₅ à C₇ est éventuellement substitué par un ou deux groupes alkyle en C₁ à C₃).

8. Composé selon l'une quelconque des revendications 1 à 7, dans lequel R⁶, R⁷, R⁸ et R⁹ représentent chacun indépendamment un atome d'hydrogène ou un groupe alkyle en C₁ à C₃ ou ensemble R⁷ et R⁵ conjointement avec l'atome N auquel ils sont fixés représentent un cycle hétérocyclique saturé de 5 à 7 chaînons.

9. Composé selon la revendication 1, qui est :
la 4-[(4-chlorophényl)méthyl]-2-({(2*R*)-1-[2-(éthylsulfonyl)éthyl]-2-pyrrolidinyl}méthyl)-1(2*H*)-phtalazinone ;
la 4-[(4-chlorophényl)méthyl]-2-({(2*R*)-1-[2-(1-pipéridinylsulfonyl)éthyl]-2-pyrrolidinyl}méthyl)-1(2*H*)-phtalazinone ;
la 4-[(4-chlorophényl)méthyl]-2-({(2*R*)-1-[3-(éthylsulfonyl)propyl]-2-pyrrolidinyl}méthyl)-1(2*H*)-phtalazinone ;
la 4-[(4-chlorophényl)méthyl]-2-({(2*R*)-1-[3-(éthylsulfonyl)butyl]-2-pyrrolidinyl}méthyl)-1(2*H*)-phtalazinone ;
la 4-[(4-chlorophényl)méthyl]-2-({(2*R*)-1-[(3*S*)-3-(éthylsulfonyl)butyl]-2-pyrrolidinyl}méthyl)-(2*H*)-phtalazinone ;
la 4-[(4-chlorophényl)méthyl]-2-({(2*R*)-1-[(3*R*)-3-(éthylsulfonyl)butyl]-2-pyrrolidinyl}méthyl)-(2*H*)-phtalazinone ;
le *N*-[2-((2*R*)-2-{[4-[(4-chlorophényl)méthyl]-1-oxo-2(1*H*)-phtalazinyl]méthyl}-1-pyrrolidinyl)éthyl]-éthanesulfonamide ;
le *N*-[2-((2*R*)-2-{[4-[(4-chlorophényl)méthyl]-1-oxo-2(1*H*)-phtalazinyl]méthyl}-1-pyrrolidinyl)éthyl]-1-propanesulfonamide ;
le *N*-[2-((2*R*)-2-{[4-[(4-chlorophényl)méthyl]-1-oxo-2(1*H*)-phtalazinyl]méthyl}-1-pyrrolidinyl)éthyl]-2-propanesulfonamide ;
le *N*-[4-((2*R*)-2-{[4-[(4-chlorophényl)méthyl]-1-oxo-2(1*H*)-phtalazinyl]méthyl}-1-pyrrolidinyl)butyl]-éthanesulfonamide ;
le 2-((2*R*)-2-{[4-[(4-chlorophényl)méthyl]-1-oxo-2(1*H*)-phtalazinyl]méthyl}-1-pyrrolidinyl)-*N*-[3-(méthyloxy)propyl]éthanesulfonamide ;
la *N*-[2-((2*R*)-2-{[4-[(4-chlorophényl)méthyl]-1-oxo-2(1*H*)-phtalazinyl]méthyl}-1-pyrrolidinyl)éthyl]-*N*'-propylurée ;
la 4-[(4-chlorophényl)méthyl]-2-({(2*R*)-1-[3-(éthylsulfonyl)propyl]-2-pyrrolidinyl}méthyl)pyrido-[3,4-d]pyridazin-1(2*H*)-one ;
la 4-[(4-chlorophényl)méthyl]-2-({(2*R*)-1-[2-(éthylsulfonyl)éthyl]-2-pyrrolidinyl}méthyl)pyrido-[3,4-d]pyridazin-1(2*H*)-one ;
la *N*-[2-((2*R*)-2-{[4-[(4-chlorophényl)méthyl]-1-oxopyrido[3,4-*d*]pyridazin-2(1*H*)-yl]méthyl}-1-pyrrolidinyl)éthyl]-*N*'-propylurée ;
ou un sel de celui-ci.

10. Composé selon l'une quelconque des revendications 1 à 9, ou un sel pharmaceutiquement acceptable de celui-ci.

11. Composé tel que défini dans l'une quelconque des revendications 1 à 9, ou un sel pharmaceutiquement acceptable de celui-ci pour une utilisation en thérapie.

12. Composé selon la revendication 11, pour une utilisation dans le traitement de maladies inflammatoires et/ou allergiques.

13. Composé selon la revendication 12, pour une utilisation dans le traitement de la rhinite allergique.

14. Composition qui comprend un composé tel que défini dans l'une quelconque des revendications 1 à 9, ou un sel pharmaceutiquement acceptable de celui-ci et un ou plusieurs supports et/ou excipients pharmaceutiquement acceptables.

15. Composition selon la revendication 14, comprenant en outre un ou plusieurs autres agents thérapeutiques.
